# EUROPEAN PATENT SPECIFICATION

(11) **EP 1 012 293 B1**
(45) Date of publication and mention of the grant of the patent: **07.01.2009**
(21) Application number: 98924827.3
(22) Date of filing: 20.05.1998
(51) Int. Cl.: C12N 15/31, C07K 14/35, A61K 39/04, A61K 48/00, A61K 49/00, C12N 15/62, C07K 19/00, G01N 33/50, G01N 33/60, G01N 33/569, C12N 1/19, C12N 1/21, C12R 1/19

(54) **COMPOUNDS FOR IMMUNOTHERAPY AND DIAGNOSIS OF TUBERCULOSIS AND METHODS OF THEIR USE**
VERBINDUNGEN ZUR IMMUNTHERAPIE UND DIAGNOSE VON TUBERKULOSE UND METHODEN ZU DEREN VERWENDUNG
COMPOSES POUR L'IMMUNOTHERAPIE ET POUR LE DIAGNOSTIC DE LA TUBERCULOSE ET LEUR UTILISATION

(30) Priority: 20.05.1997 US 859381
(43) Date of publication of application: 28.06.2000
(73) Proprietor: CORIXA CORPORATION, Wilmington, DE 19808 (US)
(72) Inventor: ALDERSON, Mark, R., Bainbridge Island, WA 98110 (US); DILLON, Davin, C., Redmond, WA 98053 (US); SKEIKY, Yasir, A., W., Seattle, WA 98117 (US); CAMPOS-NETO, Antonio, Bainbridge Island, WA 98110 (US)
(74) Representative: Teuten, Andrew John
(86) International application number: PCT/US1998/010407
(87) International publication number: WO 1998/053075

## Description

### Technical Field

The present invention relates generally to detecting, treating and preventing *Mycobacterium tuberculosis* infection. The invention is more particularly related to polypeptides comprising a *Mycobacterium tuberculosis* antigen, or a portion or other variant thereof, and the use of such polypeptides for diagnosing and vaccinating against *Mycobacterium tuberculosis* infection.

### Background of the Invention

Tuberculosis is a chronic, infectious disease, that is generally caused by infection with *Mycobacterium tuberculosis.* It is a major disease in developing countries, as well as an increasing problem in developed areas of the world, with about 8 million new cases and 3 million deaths each year. Although the infection may be asymptomatic for a considerable period of time, the disease is most commonly manifested as an acute inflammation of the lungs, resulting in fever and a nonproductive cough. If left untreated, serious complications and death typically result.

Although tuberculosis can generally be controlled using extended antibiotic therapy, such treatment is not sufficient to prevent the spread of the disease. Infected individuals may be asymptomatic, but contagious, for some time. In addition, although compliance with the treatment regimen is critical, patient behavior is difficult to monitor. Some patients do not complete the course of treatment, which can lead to ineffective treatment and the development of drug resistance.

Inhibiting the spread of tuberculosis requires effective vaccination and accurate, early diagnosis of the disease. Currently, vaccination with live bacteria is the most efficient method for inducing protective immunity. The most common Mycobacterium employed for this purpose is *Bacillus* Calmette-Guerin (BCG), an avirulent strain of *Mycobacterium bovis.* However, the safety and efficacy of BCG is a source of controversy and some countries, such as the United States, do not vaccinate the general public. Diagnosis is commonly achieved using a skin test, which involves intradermal exposure to tuberculin PPD (protein-purified derivative). Antigen-specific T cell responses result in measurable induration at the injection site by 48-72 hours after injection, which indicates exposure to Mycobacterial antigens. Sensitivity and specificity have, however, been a problem with this test, and individuals vaccinated with BCG cannot be distinguished from infected individuals.

While macrophages have been shown to act as the principal effectors of *M. tuberculosis* immunity, T cells are the predominant inducers of such immunity. The essential role of T cells in protection against *M. tuberculosis* infection is illustrated by the frequent occurrence of *M. tuberculosis* in AIDS patients, due to the depletion of CD4 T cells associated with human immunodeficiency virus (HIV) infection. Mycobacterium-reactive CD4 T cells have been shown to be potent producers of gamma-interferon (IFN-γ), which, in turn, has been shown to trigger the anti-mycobacterial effects of macrophages in mice. While the role of IFN-γ in humans is less clear, studies have shown that 1,25-dihydroxy-vitamin D3, either alone or in combination with IFN-γ or tumor necrosis factor-alpha, activates human macrophages to inhibit *M. tuberculosis* infection. Furthermore, it is known that IFN-γ stimulates human macrophages to make 1,25-dihydroxy-vitamin D3. Similarly, IL-12 has been shown to play a role in stimulating resistance to *M. tuberculosis* infection. For a review of the immunology of *M. tuberculosis* infection see Chan and Kaufmann in Tuberculosis: Pathogenesis, Protection and Control, Bloom (ed.), ASM Press, Washington, DC, 1994. Document WO/97/09428 discloses compounds and methods for immunotherapy and diagnosis of tuberculosis.

Accordingly, there is a need in the art for improved vaccines and methods for preventing, treating and detecting tuberculosis. The present invention fulfills these needs and further provides other related advantages.

### Summary of the Invention

Briefly stated, this invention provides compounds for preventing and diagnosing tuberculosis.

The present invention provides an isolated polypeptide comprising:
(i) an immunogenic portion of the *M. tuberculosis* antigen recited in SEQ ID NO: 109; or
(ii) a variant of the *M. tuberculosis* antigen recited in SEQ ID NO: 109 having at least 95% identity to SEQ ID NO: 109;
with the proviso that the isolated polypeptide does not consist of the *M. tuberculosis* antigen recited in SEQ ID NO: 109.

Also provided are fusion proteins comprising a polypeptide comprising:
(i) an immunogenic portion of the *M. tuberculosis* antigen recited in SEQ ID NO: 109; or
(ii) a variant of the *M. tuberculosis* antigen recited in SEQ ID NO: 109 having at least 90% identity to SEQ ID NO: 109.

Within other aspects, the present invention provides a DNA molecule comprising a nucleotide sequence encoding a polypeptide as described above, with the proviso that the DNA molecule does not comprise a nucleotide sequence encoding the polypeptide consisting of the *M. tuberculosis* antigen recited in SEQ ID NO: 109. Additionally provided is a DNA molecule comprising a nucleotide sequence encoding a fusion protein as described above or a DNA molecule consisting of a nucleotide sequence encoding a polypeptide or fusion protein as described above.

In other aspects, expression vectors are provided comprising a DNA molecule comprising a nucleotide sequence encoding a polypeptide comprising:
(i) an immunogenic portion of the *M. tuberculosis* antigen recited in SEQ ID NO: 109; or
(ii) a variant of the *M. tuberculosis* antigen recited in SEQ ID NO: 109 having at least 95% identity to SEQ ID NO: 109, with the proviso that the expression vector does not comprise a nucleotide sequence encoding the polypeptide consisting of the *M. tuberculosis* antigen recited in SEQ ID NO: 109, together with host cells transformed or transfected with said expression vectors.

A further aspect of the invention is a pharmaceutical composition comprising at least one polypeptide comprising:
(i) an immunogenic portion of the *M. tuberculosis* antigen recited in SEQ ID NO: 109; or
(ii) a variant of the *M. tuberculosis* antigen recited in SEQ ID NO: 109 having at least 90% identity to SEQ ID NO: 109;
or comprising a DNA molecule comprising a nucleotide sequence encoding said polypeptide,
and a physiologically acceptable carrier.

Methods are disclosed for inducing protective immunity in a patient, comprising administering to a patient an effective amount of one or more of the above polypeptides.

In further aspects of this invention, diagnostic kits are provided for detecting tuberculosis in a patient. The diagnostic kits comprise one or more of the above polypeptides in combination with an apparatus sufficient to contact the polypeptide with the dermal cells of a patient.

Additionally provided is a vaccine comprising at least one polypeptide comprising:
(i) an immunogenic portion of the *M. tuberculosis* antigen recited in SEQ ID NO: 109; or
(ii) a variant of the *M. tuberculosis* antigen recited in SEQ ID NO: 109 having at least 90% identity to SEQ ID NO: 109,
or a DNA molecule comprising a nucleotide sequence encoding said polypeptide, and a non-specific immune response enhancer.

A polypeptide comprising:
(i) an immunogenic portion of the *M. tuberculosis* antigen recited in SEQ ID NO: 109; or
(ii) a variant of the *M. tuberculosis* antigen recited in SEQ ID NO: 109 having at least 90% identity to SEQ ID NO: 109,
or a DNA molecule comprising a nucleotide sequence encoding said polypeptide, are provided for use in inducing protective immunity in a patient.

There is also provided the use of a polypeptide comprising:
(i) an immunogenic portion of the *M. tuberculosis* antigen recited in SEQ ID NO: 109; or
(ii) a variant of the *M. tuberculosis* antigen recited in SEQ ID NO: 109 having at least 90% identity to SEQ ID NO: 109,
or the use of a DNA molecule comprising a nucleotide sequence encoding said polypeptide, in the manufacture of a pharmaceutical composition for inducing protective immunity in a patient.

Further provided is the use of a polypeptide comprising:
(i) an immunogenic portion of the *M. tuberculosis* antigen recited in SEQ ID NO: 109; or
(ii) a variant of the *M. tuberculosis* antigen recited in SEQ ID NO: 109 having at least 90% identity to SEQ ID NO: 109,
or the use of a DNA molecule comprising a nucleotide sequence encoding said polypeptide, in the manufacture of a vaccine for inducing protective immunity in a patient.

### Brief Description of the Drawings

Figures 1A and 1B illustrate the stimulation of proliferation and interferon-γ production, respectively, in T cells derived from a first PPD-positive donor (referred to as D7) by recombinant ORF-2 and synthetic peptides to ORF-2.
Figures 2A and 2B illustrate the stimulation of proliferation and interferon-γ production, respectively, in T cells derived from a second PPD-positive donor (referred to as D160) by recombinant ORF-2 and synthetic peptides to ORF-2.

### Detailed Description of the Invention

As noted above, the present invention is generally directed to compositions for preventing, treating and diagnosing tuberculosis. The compositions of the subject invention include polypeptides that comprise at least one immunogenic portion of a *M. tuberculosis* antigen, or a variant of such an antigen that differs only in conservative substitutions and/or modifications. As used herein, the term "polypeptide" encompasses amino acid chains of any length, including full length proteins (*i.e*., antigens), wherein the amino acid residues are linked by covalent peptide bonds. Thus, a polypeptide comprising an immunogenic portion of one of the above antigens may consist entirely of the immunogenic portion, or may contain additional sequences. The additional sequences may be derived from the native *M. tuberculosis* antigen or may be heterologous, and such sequences may (but need not) be immunogenic.

"Immunogenic," as used herein, refers to the ability to elicit an immune response (*e.g.*, cellular) in a patient, such as a human, and/or in a biological sample. In particular, antigens that are immunogenic (and immunogenic portions or other variants of such antigens) are capable of stimulating cell proliferation, interleukin-12 production and/or interferon-γ production in biological samples comprising one or more cells selected from the group of T cells, NK cells, B cells and macrophages, where the cells are derived from an *M. tuberculosis*-immune individual. Polypeptides comprising at least an immunogenic portion of one or more *M. tuberculosis* antigens may generally be used to detect tuberculosis or to induce protective immunity against tuberculosis in a patient.

The compositions and methods of this invention also encompass variants of the above polypeptides. A polypeptide "variant," as used herein, is a polypeptide that differs from the recited polypeptide only in conservative substitutions and/or modifications, such that the therapeutic, antigenic and/or immunogenic properties of the polypeptide are retained, and having at least

90% and most preferably at least about 95% identity to the identified polypeptides. For polypeptides with immunoreactive properties, variants may, alternatively, be identified by modifying the amino acid sequence of one of the above polypeptides, and evaluating the immunoreactivity of the modified polypeptide. For polypeptides useful for the generation of diagnostic binding agents, a variant may be identified by evaluating a modified polypeptide for the ability to generate antibodies that detect the presence or absence of tuberculosis. Alternatively, variants of the claimed antigens that may be usefully employed in the inventive diagnostic methods may be identified by evaluating modified polypeptides for their ability to detect antibodies present in the sera of tuberculosis-infected patients. Such modified sequences may be prepared and tested using, for example, the representative procedures described herein.

A "conservative substitution" is one in which an amino acid is substituted for another amino acid that has similar properties, such that one skilled in the art of peptide chemistry would expect the secondary structure and hydropathic nature of the polypeptide to be substantially unchanged. In general, the following groups of amino acids represent conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

Variants may also (or alternatively) be modified by, for example, the deletion or addition of amino acids that have minimal influence on the immunogenic properties, secondary structure and hydropathic nature of the polypeptide. For example, a polypeptide may be conjugated to a signal (or leader) sequence at the N-terminal end of the protein which co-translationally or post-translationally directs transfer of the protein. The polypeptide may also be conjugated to a linker or other sequence for ease of synthesis, purification or identification of the polypeptide (*e.g.*, poly-His), or to enhance binding of the polypeptide to a solid support. For example, a polypeptide may be conjugated to an immunoglobulin Fc region.

In general, *M. tuberculosis* antigens, and DNA sequences encoding such antigens, may be prepared using any of a variety of procedures. For example, genomic or cDNA libraries derived from *M. tuberculosis* may be screened directly using peripheral blood mononuclear cells (PBMCs) or T cell lines or clones derived from one or more *M. tuberculosis*-immune individuals. Direct library screens may generally be performed by assaying pools of expressed recombinant proteins for the ability of induce proliferation and/or interferon-γ production in T cells derived from an *M. tuberculosis-*immune individual. Potential T cell antigens may be first selected based on antibody reactivity, as described above.

Alternatively, DNA sequences encoding antigens may be identified by screening an appropriate *M. tuberculosis* genomic or cDNA expression library with sera obtained from patients infected with *M. tuberculosis.* Such screens may generally be performed using techniques well known to those of ordinary skill in the art, such as those described in Sambrook et al., Molecular Cloning: A Laboratory Manual, Cold Spring Harbor Laboratories, Cold Spring Harbor, NY, 1989.

Purified antigens are then evaluated for their ability to elicit an appropriate immune response (*e.g.*, cellular) using, for example, the representative methods described herein. Immunogenic antigens may then be partially sequenced using techniques such as traditional Edman chemistry. *See* Edman and Berg, Eur. J. Biochem. 80:116-132, 1967. Immunogenic antigens may also be produced recombinantly using a DNA sequence that encodes the antigen, which has been inserted into an expression vector and expressed in an appropriate host.

DNA sequences encoding the inventive antigens may also be obtained by screening an appropriate *M. tuberculosis* cDNA or genomic DNA library for DNA sequences that hybridize to degenerate oligonucleotides derived from partial amino acid sequences of isolated antigens. Degenerate oligonucleotide sequences for use in such a screen may be designed and synthesized, and the screen may be performed, as described (for example) in Sambrook et al., Molecular Cloning: A Laboratory Manual, Cold Spring Harbor Laboratories, Cold Spring Harbor, NY, 1989 (and references cited therein). Polymerase chain reaction (PCR) may also be employed, using the above oligonucleotides in methods well known in the art, to isolate a nucleic acid probe from a cDNA or genomic library. The library screen may then be performed using the isolated probe.

Regardless of the method of preparation, the antigens (and immunogenic portions thereof) described herein have the ability to induce an immunogenic response. More specifically, the antigens have the ability to induce proliferation and/or cytokine production (*i.e.*, interferon-γ and/or interleukin-12 production) in T cells, NK cells, B cells and/or macrophages derived from an *M. tuberculosis*-immune individual. The selection of cell type for use in evaluating an immunogenic response to a antigen will, of course, depend on the desired response. For example, interleukin-12 production is most readily evaluated using preparations containing B cells and/or macrophages. An *M. tuberculosis*-immune individual is one who is considered to be resistant to the development of tuberculosis by virtue of having mounted an effective T cell response to *M. tuberculosis* (*i.e.,* substantially free of disease symptoms). Such individuals may be identified based on a strongly positive (*i.e.*, greater than about 10 mm diameter induration) intradermal skin test response to tuberculosis proteins (PPD) and an absence of any signs or symptoms of tuberculosis disease. T cells. NK cells, B cells and macrophages derived from *M. tuberculosis*-immune individuals may be prepared using methods known to those of ordinary skill in the art. For example, a preparation of PBMCs (*i.e.*, peripheral blood mononuclear cells) may be employed without further separation of component cells. PBMCs may generally be prepared, for example, using density centrifugation through Ficoll^{™} (Winthrop Laboratories, NY).

T cells for use in the assays described herein may also be purified directly from PBMCs. Alternatively, an enriched T cell line reactive against mycobacterial proteins, or T cell clones reactive to individual mycobacterial proteins, may be employed. Such T cell clones may be generated by, for example. culturing PBMCs from *M. tuberculosis*-immune individuals with mycobacterial proteins for a period of 2-4 weeks. This allows expansion of only the mycobacterial protein-specific T cells, resulting in a line composed solely of such cells. These cells may then be cloned and tested with individual proteins. using methods known to those of ordinary skill in the art, to more accurately define individual T cell specificity. In general, antigens that test positive in assays for proliferation and/or cytokine production (*i.e.*, interferon-γ and/or interleukin-12 production) performed using T cells, NK cells, B cells and/or macrophages derived from an *M. tuberculosis*-immune individual are considered immunogenic. Such assays may be performed, for example, using the representative procedures described below. Immunogenic portions of such antigens may be identified using similar assays. and may be present within the polypeptides described herein.

The ability of a polypeptide (*e.g.*, an immunogenic antigen, or a portion or other variant thereof) to induce cell proliferation is evaluated by contacting the cells (*e.g.*, T cells and/or NK cells) with the polypeptide and measuring the proliferation of the cells. In general, the amount of polypeptide that is sufficient for evaluation of about 10⁵ cells ranges from about 10 ng/mL to about 100 µg/mL and preferably is about 10 µ g/mL. The incubation of polypeptide with cells is typically performed at 37°C for about six days. Following incubation with polypeptide, the cells are assayed for a proliferative response, which may be evaluated by methods known to those of ordinary skill in the art, such as exposing cells to a pulse of radiolabeled thymidine and measuring the incorporation of label into cellular DNA. In general, a polypeptide that results in at least a three fold increase in proliferation above background (*i.e.*, the proliferation observed for cells cultured without polypeptide) is considered to be able to induce proliferation.

The ability of a polypeptide to stimulate the production of interferon-γ and/or interleukin-12 in cells may be evaluated by contacting the cells with the polypeptide and measuring the level of interferon-γ or interleukin-12 produced by the cells. In general, the amount of polypeptide that is sufficient for the evaluation of about 10⁵ cells ranges from about 10 ng/mL to about 100 µg/mL and preferably is about 10 µ g/mL. The polypeptide may, but need not, be immobilized on a solid support. such as a bead or a biodegradable microsphere, such as those described in U.S. Patent Nos. 4,897,268 and 5,075,109. The incubation of polypeptide with the cells is typically performed at 37°C for about six days. Following incubation with polypeptide, the cells are assayed for interferon-γ and/or interleukin-12 (or one or more subunits thereof), which may be evaluated by methods known to those of ordinary skill in the art. such as an enzyme-linked immunosorbent assay (ELISA) or, in the case of IL-12 P70 heterodimer, a bioassay such as an assay measuring proliferation of T cells. In general, a polypeptide that results in the production of at least 50 pg of interferon-γ per mL of cultured supernatant (containing 10⁴-10⁵ T cells per mL) is considered able to stimulate the production of interferon-γ. A polypeptide that stimulates the production of at least 10 pg/mL of IL-12 P70 subunit, and/or at least 100 pg/mL of IL-12 P40 subunit, per 10⁵ macrophages or B cells (or per 3 x 10⁵ PBMC) is considered able to stimulate the production of IL-12.

In general, immunogenic antigens are those antigens that stimulate proliferation and/or cytokine production (*i.e.*, interferon-γ and/or interleukin-12 production) in T cells, NK cells, B cells and/or macrophages derived from at least about 25% of *M. tuberculosis-*immune individuals. Among these immunogenic antigens, polypeptides having superior therapeutic properties may be distinguished based on the magnitude of the responses in the above assays and based on the percentage of individuals for which a response is observed. In addition, antigens having superior therapeutic properties will not stimulate proliferation and/or cytokine production *in vitro* in cells derived from more than about 25% of individuals that are not *M. tuberculosis-*immune, thereby eliminating responses that are not specifically due to *M. tuberculosis*-responsive cells. Those antigens that induce a response in a high percentage of T cell, NK cell, B cell and/or macrophage preparations from *M. tuberculosis*-immune individuals (with a low incidence of responses in cell preparations from other individuals) have superior therapeutic properties.

Antigens with superior therapeutic properties may also be identified based on their ability to diminish the severity of *M. tuberculosis* infection in experimental animals, when administered as a vaccine. Suitable vaccine preparations for use on experimental animals are described in detail below. Efficacy may be determined based on the ability of the antigen to provide at least about a 50% reduction in bacterial numbers and/or at least about a 40% decrease in mortality following experimental infection. Suitable experimental animals include mice, guinea pigs and primates.

Antigens having superior diagnostic properties may generally be identified based on the ability to elicit a response in an intradermal skin test performed on an individual with active tuberculosis, but not in a test performed on an individual who is not infected with *M. tuberculosis.* Skin tests may generally be performed as described below, with a response of at least 5 mm induration considered positive.

Immunogenic portions of the antigens described herein may be prepared and identified using well known techniques, such as those summarized in Paul, Fundamental Immunology, 3d ed.. Raven Press. 1993. pp. 243-247 and references cited therein. Such techniques include screening polypeptide portions of the native antigen for immunogenic properties. The representative proliferation and cytokine production assays described herein may generally be employed in these screens. An immunogenic portion of a polypeptide is a portion that, within such representative assays, generates an immune response (*e.g.*, proliferation, interferon-γ production and/or interleukin-12 production) that is substantially similar to that generated by the full length antigen. In other words, an immunogenic portion of an antigen may generate at least about 20%, and preferably about 100%, of the proliferation induced by the full length antigen in the model proliferation assay described herein. An immunogenic portion may also, or alternatively, stimulate the production of at least about 20%, and preferably about 100%, of the interferon-γ and/or interleukin-12 induced by the full length antigen in the model assay described herein.

Portions and other variants of *M. tuberculosis* antigens may be generated by synthetic or recombinant means. Synthetic polypeptides having fewer than about 100 amino acids, and generally fewer than about 50 amino acids, may be generated using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Perkin Elmer/Applied BioSystems Division, Foster City, CA, and may be operated according to the manufacturer's instructions. Variants of a native antigen may generally be prepared using standard mutagenesis techniques, such as oligonucleotide-directed site-specific mutagenesis. Sections of the DNA sequence may also be removed using standard techniques to permit preparation of truncated polypeptides.

Recombinant polypeptides containing portions and/or variants of a native antigen may be readily prepared from a DNA sequence encoding the polypeptide using a variety of techniques well known to those of ordinary skill in the art. For example, supernatants from suitable host/vector systems which secrete recombinant protein into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant protein.

Any of a variety of expression vectors known to those of ordinary skill in the art may be employed to express recombinant polypeptides of this invention. Expression may be achieved in any appropriate host cell that has been transformed or transfected with an expression vector containing a DNA molecule that encodes a recombinant polypeptide. Suitable host cells include prokaryotes, yeast and higher eukaryotic cells. Preferably, the host cells employed are *E. coli,* yeast or a mammalian cell line such as COS or CHO. The DNA sequences expressed in this manner may encode naturally occurring antigens, portions of naturally occurring antigens, or other variants thereof.

In general, regardless of the method of preparation, the polypeptides disclosed herein are prepared in substantially pure form. Preferably, the polypeptides are at least about 80% pure, more preferably at least about 90% pure and most preferably at least about 99% pure. In certain preferred embodiments, described in detail below, the substantially pure polypeptides are incorporated into pharmaceutical compositions or vaccines for use in one or more of the methods disclosed herein.

In a related aspect, the present invention provides fusion proteins comprising a first and a second inventive polypeptide or, alternatively, a polypeptide of the present invention and a known *M tuberculosis* antigen, such as the 38 kD antigen described in Andersen and Hansen, Infect. Immun. 57:2481-2488, 1989, (Genbank Accession No. M30046), or ESAT-6 previously identified in *M. bovis* (Accession No. U34848) and in *M tuberculosis* (Sorensen et al., Infec. Immun. 63:1710-1717, 1995). Variants of such fusion proteins are also provided. The fusion proteins of the present invention may include a linker peptide between the first and second polypeptides.

A DNA sequence encoding a fusion protein of the present invention is constructed using known recombinant DNA techniques to assemble separate DNA sequences encoding the first and second polypeptides into an appropriate expression vector. The 3' end of a DNA sequence encoding the first polypeptide is ligated, with or without a peptide linker, to the 5' end of a DNA sequence encoding the second polypeptide so that the reading frames of the sequences are in phase to permit mRNA translation of the two DNA sequences into a single fusion protein that retains the biological activity of both the first and the second polypeptides.

A peptide linker sequence may be employed to separate the first and the second polypeptides by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad Sci. USA 83:8258-8262, 1986; U.S. Patent No. 4,935,233 and U.S. Patent No. 4,751,180. The linker sequence may be from 1 to about 50 amino acids in length. Peptide sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons require to end translation and transcription termination signals are only present 3' to the DNA sequence encoding the second polypeptide.

Methods are disclosed for using one or more of the above polypeptides or fusion proteins (or DNA molecules encoding such polypeptides) to induce protective immunity against tuberculosis in a patient. As used herein, a "patient" refers to any warm-blooded animal, preferably a human. A patient may be afflicted with a disease, or may be free of detectable disease and/or infection. In other words, protective immunity may be induced to prevent or treat tuberculosis.

In such methods, the polypeptide, fusion protein or DNA molecule is generally present within a pharmaceutical composition and/or a vaccine. Pharmaceutical compositions may comprise one or more polypeptides, each of which may contain one or more of the above sequences (or variants thereof), and a physiologically acceptable carrier. Vaccines may comprise one or more of the above polypeptides and a non-specific immune response enhancer, such as an adjuvant or a liposome (into which the polypeptide is incorporated). Such pharmaceutical compositions and vaccines may also contain other *M. tuberculosis* antigens, either incorporated into a combination polypeptide or present within a separate polypeptides.

Alternatively, a vaccine may contain DNA encoding one or more polypeptides as described above, such that the polypeptide is generated *in situ.* In such vaccines, the DNA may be present within any of a variety of delivery systems known to those of ordinary skill in the art, including nucleic acid expression systems. bacterial and viral expression systems. Appropriate nucleic acid expression systems contain the necessary DNA sequences for expression in the patient (such as a suitable promoter and terminating signal). Bacterial delivery systems involve the administration of a bacterium (such as *Bacil*/*us-Calmette-Guerrin*) that expresses an immunogenic portion of the polypeptide on its cell surface. In a preferred embodiment, the DNA may be introduced using a viral expression system (*e.g.*, vaccinia or other pox virus, retrovirus, or adenovirus), which may involve the use of a non-pathogenic (defective), replication competent virus. Techniques for incorporating DNA into such expression systems are well known to those of ordinary skill in the art. The DNA may also be "naked." as described, for example, in Ulmer et al.. Science 259: 1745-1749, 1993 and reviewed by Cohen, Science 259:1691-1692, 1993. The uptake of naked DNA may be increased by coating the DNA onto biodegradable beads, which are efficiently transported into the cells.

In a related aspect, a DNA vaccine as described above may be administered simultaneously with or sequentially to either a polypeptide of the present invention or a known *M. tuberculosis* antigen, such as the 38 kD antigen described above. For example, administration of DNA encoding a polypeptide of the present invention, either "naked" or in a delivery system as described above, may be followed by administration of an antigen in order to enhance the protective immune effect of the vaccine.

Routes and frequency of administration, as well as dosage, will vary from individual to individual and may parallel those currently being employed in immunization using BCG. In general, the pharmaceutical compositions and vaccines may be administered by injection (*e.g.*, intracutaneous, intramuscular, intravenous or subcutaneous), intranasally (*e.g.*, by aspiration) or orally. Between 1 and 3 doses may be administered for a 1-36 week period. Preferably, 3 doses are administered, at intervals of 3-4 months, and booster vaccinations may be given periodically thereafter. Alternate protocols may be appropriate for individual patients. A suitable dose is an amount of polypeptide or DNA that, when administered as described above, is capable of raising an immune response in an immunized patient sufficient to protect the patient from *M. tuberculosis* infection for at least 1-2 years. In general, the amount of polypeptide present in a dose (or produced *in situ* by the DNA in a dose) ranges from about 1 pg to about 100 mg per kg of host, typically from about 10 pg to about 1 mg, and preferably from about 100 pg to about 1 µg. Suitable dose sizes will vary with the size of the patient, but will typically range from about 0.1 mL to about 5 mL.

While any suitable carrier known to those of ordinary skill in the art may be employed in the pharmaceutical compositions of this invention, the type of carrier will vary depending on the mode of administration. For parenteral administration, such as subcutaneous injection, the carrier preferably comprises water, saline, alcohol, lipids, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g.*, polylactic galactide) may also be employed as carriers for the pharmaceutical compositions of this invention. Suitable biodegradable microspheres are disclosed, for example, in U.S. Patent Nos. 4,897,268 and 5,075,109.

Any of a variety of adjuvants may be employed in the vaccines of this invention to nonspecifically enhance the immune response. Most adjuvants contain a substance designed to protect the antigen from rapid catabolism, such as aluminum hydroxide or mineral oil, and a nonspecific stimulator of immune responses, such as lipid A, *Bortadella pertussis* or *Mycobacterium tuberculosis.* Suitable adjuvants are commercially available as, for example, Freund's Incomplete Adjuvant and Freund's Complete Adjuvant (Difco Laboratories) and Merck Adjuvant 65 (Merck and Company, Inc., Rahway, NJ). Other suitable adjuvants include alum, biodegradable microspheres, monophosphoryl lipid A and quil A.

In another aspect, this invention provides diagnostics kits suitable for use in a skin test. As used herein, a "skin test" is any assay performed directly on a patient in which a delayed-type hypersensitivity (DTH) reaction (such as swelling, reddening or dermatitis) is measured following intradermal injection of one or more polypeptides as described above. Such injection may be achieved using any suitable device sufficient to contact the polypeptide or polypeptides with dermal cells of the patient, such as a tuberculin syringe or 1 mL syringe. Preferably, the reaction is measured at least 48 hours after injection, more preferably 48-72 hours.

The DTH reaction is a cell-mediated immune response, which is greater in patients that have been exposed previously to the test antigen (*i.e.*, the immunogenic portion of the polypeptide employed, or a variant thereof). The response may be measured visually, using a ruler. In general, a response that is greater than about 0.5 cm in diameter, preferably greater than about 1.0 cm in diameter, is a positive response, indicative of tuberculosis infection, which may or may not be manifested as an active disease.

The polypeptides of this invention are preferably formulated. for use in a skin test, as pharmaceutical compositions containing a polypeptide and a physiologically acceptable carrier, as described above. Such compositions typically contain one or more of the above polypeptides in an amount ranging from about 1 µg to about 100 µg, preferably from about 10 µg to about 50 µg in a volume of 0.1 mL. Preferably, the carrier employed in such pharmaceutical compositions is a saline solution with appropriate preservatives, such as phenol and/or Tween 80™.

In a preferred embodiment, a polypeptide employed in a skin test is of sufficient size such that it remains at the site of injection for the duration of the reaction period. In general, a polypeptide that is at least 9 amino acids in length is sufficient. The polypeptide is also preferably broken down by macrophages within hours of injection to allow presentation to T-cells. Such polypeptides may contain repeats of one or more of the above sequences and/or other immunogenic or non-immunogenic sequences.

The following Examples are offered by way of illustration and not by way of limitation.

### EXAMPLE 1

### PURIFICATION AND CHARACTERIZATION OF M. TUBERCULOSIS POLYPEPTIDES USING CD4+ T CELL LINES GENERATED FROM HUMAN PBMC

*M. tuberculosis* antigens of the present invention were isolated by expression cloning of cDNA libraries of *M. tuberculosis* strains H37Rv and Erdman essentially as described by Sanderson et al. (J. Exp. Med., 1995, 182:1751-1757) and were shown to induce PBMC proliferation and IFN-γ in an immunoreactive T cell line.

Two CD4+ T cell lines, referred to as DC-4 and DC-5, were generated against dendritic cells infected with *M. tuberculosis.* Specifically, dendritic cells were prepared from adherent PBMC from a single donor and subsequently infected with tuberculosis. Lymphocytes from the same donor were cultured under limiting dilution conditions with the infected dendritic cells to generate the CD4+ T cell lines DC-4 and DC-5. These cell lines were shown to react with crude soluble proteins from *M. tuberculosis* but not with Tb38-1. Limiting dilution conditions were employed to obtain a third CD4+ T cell line, referred to as DC-6. which was shown to react with both crude soluble proteins and Tb38-1.

Genomic DNA was isolated from the *M. tuberculosis* strains H37Rv and Erdman and used to construct expression libraries in the vector pBSK(-)using the Lambda ZAP expression system (Stratagene. La Jolla, CA). These libraries were transformed into *E. coli.* pools of induced *E*. *coli* cultures were incubated with dendritic cells. and the ability of the resulting incubated dendritic cells to stimulate cell proliferation and IFN-γ production in the CD4+ T cell line DC-6 was examined as described below in Example 2. Positive pools were fractionated and re-tested until pure *M. tuberculosis* clones were obtained.Nineteen clones were isolated, of which nine were found to contain the previously identified *M*. *tuberculosis* antigens TbH-9 and Tb38-1, disclosed in U.S. Patent Application No. 08/533,634. The determined cDNA sequences for the remaining ten clones (hereinafter referred to as Tb224, Tb636, Tb424, Tb436, Tb398, Tb508, Tb441, Tb475, Tb488 and Tb465) are provided in SEQ ID No: 1-10, respectively. The corresponding predicted amino acid sequences for Tb224 and Tb636 are provided in SEQ ID NO: 13 and 14, respectively. The open reading frames for these two antigens were found to show some homology to TbH-9, described above. Tb224 and Tb636 were also found to be overlapping clones.

Tb424, Tb436, Tb398, Tb508, Tb441, Tb475, Tb488 and Tb465 were each found to contain two small open reading frames (referred to as ORF-1 and ORF-2) or truncated forms thereof, with minor variations in ORF-1 and ORF-2 being found for each clone. The predicted amino acid sequences of ORF-1 and ORF-2 for Tb424, Tb436, Tb398, Tb508, Tb441, Tb475, Tb488 and Tb465 are provided in SEQ ID NO: 16 and 17, 18 and 19, 20 and 21, 22 and 23, 24 and 25, 26 and 27, 28 and 29, and 30 and 31, respectively. In addition, clones Tb424 and Tb436 were found to contain a third apparent open reading frame, referred to as ORF-U. The predicted amino acid sequences of ORF-U for Tb424 and Tb436 are provided in SEQ ID NO: 32 and 33. respectively. Tb424 and Tb436 were found to be either overlapping clones or recently duplicated/transposed copies. Similarly Tb398, Tb508 and Tb465 were found to be either overlapping clones or recently duplicated/transposed copies, as were Tb475 and Tb488.

These sequences were compared with known sequences in the gene bank using the BLASTN system. No homologies to the antigens Tb224 and Tb431 were found. Tb636 was found to be 100% identical to a cosmid previously identified in *M*. *tuberculosis.* Similarly, Tb508, Tb488, Tb398, Tb424, Tb436, Tb441, Tb465 and Tb475 were found to show homology to known *M. tuberculosis* cosmids. In addition. Tb488 was found to have 100% homology to *M. tuberculosis* topoisomerase I.

Seventeen overlapping peptides to the open reading frame ORF-1 (referred to as 1-1 - 1-17; SEQ ID NO: 34-50, respectively) and thirty overlapping peptides to the open reading frame ORF-2 (referred to as 2-1 - 2-30, SEQ ID NO: 51-80) were synthesized using the procedure described below in Example 3.

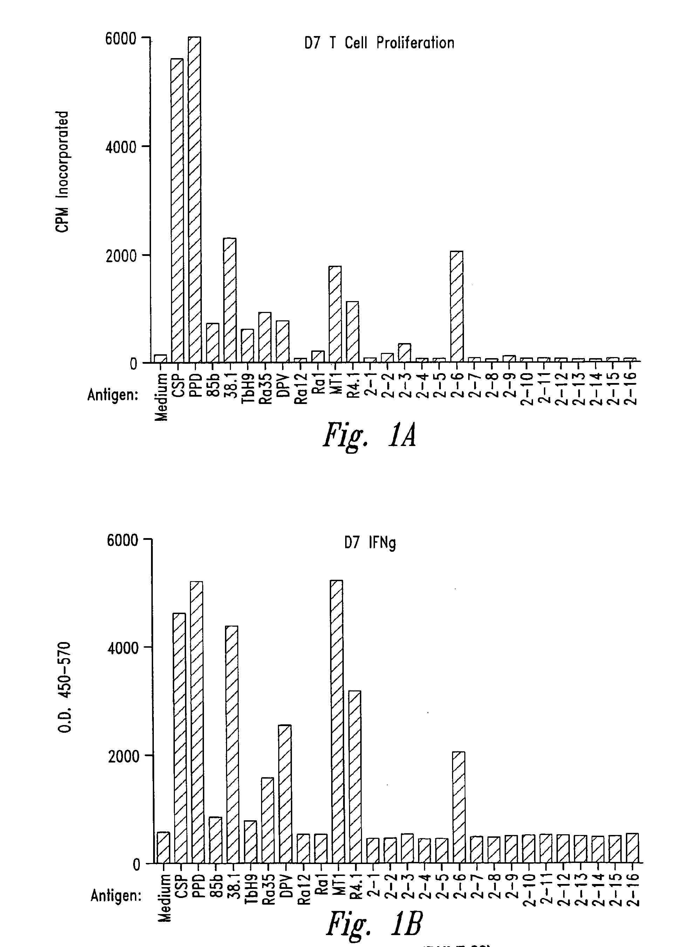

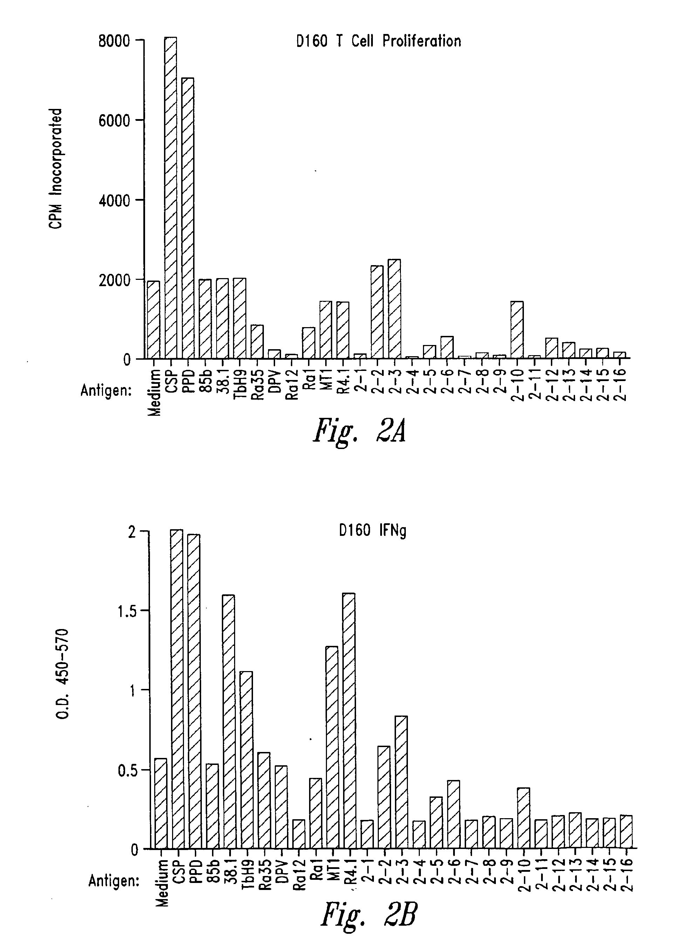

The ability of the synthetic peptides, and of recombinant ORF-1 and ORF-2, to induce T cell proliferation and IFN-γ production in PBMC from PPD-positive donors was assayed as described below in Example 2. Figs. 1A-B and 2A-B illustrate stimulation of T cell proliferation and IFN-γ by recombinant ORF-2 and the synthetic peptides 2-1 - 2-16 for two donors, referred to as D7 and D160, respectively. Recombinant ORF-2 (referred to as MTI) stimulated T cell proliferation and IFN-γ production in PBMC from both donors. The amount of PBMC stimulation seen with the individual synthetic peptides varied with each donor, indicating that each donor recognizes different epitopes on ORF-2. The proteins encoded by ORF-1. ORF-2 and ORF-U were subsequently named MTS, MTI and MSF, respectively.

Eighteen overlapping peptides to the sequence of MSF (referred to as MSF-1 -MSF-18; SEQ ID NO: 84-101, respectively) were synthesized and their ability to stimulate T cell proliferation and IFN-γ production in a CD4+ T cell line generated against *M. tuberculosis* culture filtrate was examined as described below. The peptides referred to as MSF-12 and MSF-13 (SEQ ID NO: 95 and 96, respectively) were found to show the highest levels of reactivity. Two overlapping peptides (SEQ ID NO:81 and 82) to the open reading frame of Tb224 were synthesized and shown to induce T cell proliferation and IFN-γ production in PBMC from PPD-positive donors.

Two CD4+ T cell lines from different donors were generated against *M. tuberculosis* infected dendritic cells using the above methodology. Screening of the *M. tuberculosis* cDNA expression library described above using this cell line. resulted in the isolation of two clones referred to as Tb867 and Tb391. The determined cDNA sequence for Tb867 (SEQ ID NO: 102) was found to be identical to the previously isolated *M. tuberculosis* cosmid SCY22G10. with the candidate reactive open reading frame encoding a 750 amino acid *M. tuberculosis* protein kinase. Comparison of the determined cDNA sequence for Tb391 (SEQ ID NO: 103) with those in the gene bank revealed no significant homologies to known sequences.

In further studies, CD4+ T cell lines were generated against *M. tuberculosis* culture filtrate, essentially as outlined above, and used to screen the *M. tuberculosis* Erdman cDNA expression library described above. Five reactive clones, referred to as Tb431, Tb472, Tb470, Tb838 and Tb962 were isolated. The determined cDNA sequences for Tb431, Tb472, Tb470, and Tb838 are provided in SEQ ID NO: 11, 12, 104 and 105, respectively, with the determined cDNA sequences for Tb962 being provided in SEQ ID NO: 106 and 107. The corresponding predicted amino acid sequence for Tb431 is provided in SEQ ID NO: 15.

Subsequent studies led to the isolation of a full-length cDNA sequence for Tb472 (SEQ ID NO: 108). Overlapping peptides were synthesized and used to identify the reactive open reading frame. The predicted amino acid sequence for the protein encoded by Tb472 (referred to as MSL) is provided in SEQ ID NO: 109. Comparison of the sequences for Tb472 and MSL with those in the gene bank, as described above, revealed no homologies to known sequences. Fifteen overlapping peptides to the sequence of MSL (referred to as MSL-1 - MSL-15; SEQ ID NO: 110-124, respectively) were synthesized and their ability to stimulate T cell proliferation and IFN-γ production in a CD4+ T cell line generated against *M. tuberculosis* culture filtrate was examined as described below. The peptides referred to as MSL-10 (SEQ ID NO: 119) and MSL-11 (SEQ ID NO: 120) were found to show the highest level of reactivity.

Comparison of the determined cDNA sequence for Tb838 with those in the gene bank revealed identity to the previously isolated *M. tuberculosis* cosmid SCY07H7. Comparison of the determined cDNA sequences for the clone Tb962 with those in the gene bank revealed some homology to two previously identified *M. tuberculosis* cosmids, one encoding a portion of bactoferritin. However, recombinant bactoferritin was not found to be reactive with the T cell line used to isolate Tb962.

The clone Tb470, described above, was used to recover a full-length open reading (SEQ ID NO: 125) that showed homology with TbH9 and was found to encode a 40 kDa antigen, referred to as Mtb40. The determined amino acid sequence for Mtb40 is provided in SEQ ID NO: 126. Similarly, SUBSEQUENT STUDIES LED TO THE ISOLATION OF THE FULL-LENGTH cDNA SEQUENCE FOR TB431, PROVIDED IN SEQ ID NO: 83, which was determined to contain an open reading frame encoding Mtb40. Tb470 and Tb431 were also found to contain a potential open reading frame encoding a U-ORF-like antigen.

Screening of an *M. tuberculosis* Erdman cDNA expression library with multiple CD4+ T cell lines generated against *M tuberculosis* culture filtrate, resulted in the isolation of three clones, referred to as Tb366, Tb433 and Tb439. The determined cDNA sequences for Tb366, Tb433 and Tb439 are provided in SEQ ID NO: 127, 128 and 129, respectively. Comparison of these sequences with those in the gene bank revealed no significant homologies to Tb366. Tb433 was found to show some homology to the previously identified *M. tuberculosis* antigen MPT83. Tb439 was found to show 100% identity to the previously isolated *M. tuberculosis* cosmid SCY02B10.

A CD4+ T cell line was generated against *M. tuberculosis* PPD, essentially described above, and used to screen the above *M. tuberculosis* Erdman cDNA expression library. One reactive clone (referred to as Tb372) was isolated, with the determined cDNA sequences being provided in SEQ ID NO: 130 and 131. Comparison of these sequences with those in the gene bank revealed no significant homologies.

In further studies, screening of an *M. tuberculosis* cDNA expression library with a CD4+ T cell line generated against dendritic cells that had been infected with tuberculosis for 8 days, as described above, led to the isolation of two clones referred to as Tb390R5C6 and Tb390R2C11. The determined cDNA sequence for Tb390R5C6 is provided in SEQ ID NO: 132, with the determined cDNA sequences for Tb390R2C11 being provided in SEQ ID NO: 133 and 134. Tb390R5C6 was found to show 100% identity to a previously identified *M. tuberculosis* cosmid.

In subsequent studies; the methodology described above was used to screen an *M. tuberculosis* genomic DNA library prepared as follows. Genomic DNA from *M. tuberculosis* Erdman strain was randomly sheared to an average size of 2 kb, and blunt ended with Klenow polymerase, followed by the addition of EcoRI adaptors. The insert was subsequently ligated into the Screen phage vector (Novagen, Madison, WI) and packaged *in vitro* using the PhageMaker extract (Novagen). The phage library (referred to as the Erd λScreen library) was amplified and a portion was converted into a plasmid expression library by an autosubcloning mechanism using the *E. coli* strain BM25.8 (Novagen). Plasmid DNA was purified from BM25.8 cultures containing the pSCREEN recombinants and used to transform competent cells of the expressing host strain BL21 (DE3)pLysS. Transformed cells were aliquoted into 96 well microtiter plates with each well containing a pool size of approximately 50 colonies. Replica plates of the 96 well plasmid library format were induced with IPTG to allow recombinant protein expression. Following induction, the plates were centrifuged to pellet the *E. coli* which was used directly in T cell expression cloning of a CD4+ T cell line prepared from a PPD-positive donor (donor 160) as described above. Pools containing *E. coli* expressing *M. tuberculosis* T cell antigens were subsequently broken down into individual colonies and reassayed in a similar fashion to identify positive hits.

Screening of the T cell line from donor 160 with one 96 well plate of the Erd λScreen library provided a total of nine positive hits. Previous experiments on the screening of the pBSK library described above with T cells from donor 160 suggested that most or all of the positive clones would be TbH-9, Tb38-1 or MTI or variants thereof. However, Southern analysis revealed that only three wells hybridized with a mixed probe of TbH-9, Tb38-1 and MTI. Of the remaining six positive wells, two were found to be identical. The determined 5' cDNA sequences for two of the isolated clones (referred to as Y1-26C1 and Y1-86C11) are provided in SEQ ID NO: 135 and 136, respectively. The full length cDNA sequence for the isolated clone referred to as hTcc#1 is provided in SEQ ID NO: 137, with the corresponding predicted amino acid sequence being provided in SEQ ID NO: 138. Comparison of the sequences of hTcc#1 to those in the gene bank as described above, revealed some homology to the previously isolated *M. tuberculosis* cosmid MTCY07H7B.06

### EXAMPLE 2

### INDUCTION OF T CELL PROLIFERATION AND INTERFERON-γ PRODUCTION BY M. TUBERCULOSIS ANTIGENS

The ability of recombinant *M. tuberculosis* antigens to induce T cell proliferation and interferon-γ production may be determined as follows.

Proteins may be induced by IPTG and purified by gel elution, as described in Skeiky et al. J. Exp. Med., 1995, 181:1527-1537. The purified polypeptides are then screened for the ability to induce T-cell proliferation in PBMC preparations. The PBMCs from donors known to be PPD skin test positive and whose T-cells are known to proliferate in response to PPD, are cultured in medium comprising RPMI 1640 supplemented with 10% pooled human serum and 50 µg/ml gentamicin. Purified polypeptides are added in duplicate at concentrations of 0.5 to 10 µg/mL. After six days of culture in 96-well round-bottom plates in a volume of 200 µl, 50 µl of medium is removed from each well for determination of IFN-γ levels, as described below. The plates are then pulsed with 1 µCi/well of tritiated thymidine for a further 18 hours, harvested and tritium uptake determined using a gas scintillation counter. Fractions that result in proliferation in both replicates three fold greater than the proliferation observed in cells cultured in medium alone are considered positive.

IFN-γ is measured using an enzyme-linked immunosorbent assay (ELISA). ELISA plates are coated with a mouse monoclonal antibody directed to human IFN-γ (PharMingen, San Diego, CA) in PBS for four hours at room temperature. Wells are then blocked with PBS containing 5% (W/V) non-fat dried milk for 1 hour at room temperature. The plates are washed six times in PBS/0.2% TWEEN-20 and samples diluted 1:2 in culture medium in the ELISA plates are incubated overnight at room temperature. The plates are again washed and a polyclonal rabbit anti-human IFN-γ serum diluted 1:3000 in PBS/10% normal goat serum is added to each well. The plates are then incubated for two hours at room temperature. washed and horseradish peroxidase-coupled anti-rabbit IgG (Sigma Chemical So., St. Louis. MO) is added at a 1:2000 dilution in PBS/5% non-fat dried milk. After a further two hour incubation at room temperature, the plates are washed and TMB substrate added. The reaction is stopped after 20 min with 1 N sulfuric acid. Optical density is determined at 450 nm using 570 nm as a reference wavelength. Fractions that result in both replicates giving an OD two fold greater than the mean OD from cells cultured in medium alone, plus 3 standard deviations, are considered positive.

### EXAMPLE 3

### PURIFICATION AND CHARACTERIZATION OF M. TUBERCULOSIS POLYPEPTIDES USING CD4+ T CELL LINES GENERATED FROM A MOUSE M. TUBERCULOSIS MODEL

Infection of C57BL/6 mice with *M. tuberculosis* results in the development of a progressive disease for approximately 2-3 weeks. The disease progression is then halted as a consequence of the emergence of a strong protective T cell-mediated immune response. This infection model was used to generate T cell lines capable of recognizing protective *M. tuberculosis* antigens.

Specifically, spleen cells were obtained from C57BL/6 mice infected with *M. tuberculosis* for 28 days and used to raise specific anti-*M. tuberculosis* T cell lines as described above. The resulting CD4+ T cell lines, in conjunction with normal antigen presenting (spleen) cells from C57BL/6 mice were used to screen the *M. tuberculosis* Erd λScreen library described above. One of the reactive library pools, which was found to be highly stimulatory of the T cells, was selected and the corresponding active clone (referred to as Y288C10) was isolated.

Sequencing of the clone Y288C10 revealed that it contains two potential genes, in tandem. The determined cDNA sequences for these two genes (referred to as mTCC#1 and mTCC#2) are provided in SEQ ID NO: 139 and 140, respectively, with the corresponding predicted amino acid sequences being provided in SEQ ID NO: 141 and 142, respectively. Comparison of these sequences with those in the gene bank revealed identity to unknown sequences previously found within the *M. tuberculosis* cosmid MTY21C12. The predicted amino acid sequences of mTCC#1 and mTCC#2 were found to show some homology to previously identified members of the TbH9 protein family, discussed above.

### EXAMPLE 4

### SYNTHESIS OF SYNTHETIC POLYPEPTIDES

Polypeptides may be synthesized on a Millipore 9050 peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugation or labeling of the peptide. Cleavage of the peptides from the solid support may be carried out using the following cleavage mixture: trifluoroacetic acid:ethanedithiol:thioanisole:water:phenol (40:1:2:2:3). After cleaving for 2 hours, the peptides may be precipitated in cold methyl-t-butyl-ether. The peptide pellets may then be dissolved in water containing 0.1% trifluoroacetic acid (TFA) and lyophilized prior to purification by C 18 reverse phase HPLC. A gradient of 0%-60% acetonitrile (containing 0.1 % TFA) in water (containing 0.1 % TFA) may be used to elute the peptides. Following lyophilization of the pure fractions, the peptides may be characterized using electrospray mass spectrometry and by amino acid analysis.

## Claims

1. An isolated polypeptide comprising:
(i) an immunogenic portion of the M. *tuberculosis* antigen recited in SEQ ID NO: 109; or
(ii) a variant of the *M*. *tuberculosis* antigen recited in SEQ ID NO: 109 having at least 95% identity to SEQ ID NO: 109;
with the proviso that the isolated polypeptide does not consist of the *M*. *tuberculosis* antigen recited in SEQ ID NO: 109.

2. An isolated polypeptide according to claim 1, which comprises a *M*. *tuberculosis* antigen recited in SEQ ID NO: 109, or a variant of the *M*. *tuberculosis* antigen recited in SEQ ID NO: 109 having at least 95% identity to SEQ ID NO: 109, with the proviso that the isolated polypeptide does not consist of the *M*. *tuberculosis* antigen recited in SEQ ID NO: 109.

3. An isolated polypeptide according to claim 1, which comprises an immunogenic portion of the *M. tuberculosis* antigen recited in SEQ ID NO: 109.

4. An isolated polypeptide according to claim 3, which comprises an amino acid sequence recited in any one of SEQ ID NOS: 110-124.

5. An isolated polypeptide according to claim 4, which comprises an amino acid sequence recited in SEQ ID NO: 119.

6. An isolated polypeptide according to claim 5, which consists of the amino acid sequence recited in SEQ ID NO: 119.

7. An isolated polypeptide according to claim 4, which comprises an amino acid sequence recited in SEQ ID NO: 120.

8. An isolated polypeptide according to claim 7, which consists of the amino acid sequence recited in SEQ ID NO: 120.

9. An isolated polypeptide according to claim 3, which comprises the *M*. *tuberculosis* antigen recited in SEQ ID NO: 109, with the proviso that the isolated polypeptide does not consist of the *M*. *tuberculosis* antigen recited in SEQ ID NO: 109.

10. A fusion protein comprising a polypeptide comprising:
(i) an immunogenic portion of the *M*. *tuberculosis* antigen recited in SEQ ID NO: 109; or
(ii) a variant of the *M*. *tuberculosis* antigen recited in SEQ ID NO: 109 having at least 90% identity to SEQ ID NO: 109.

11. A DNA molecule comprising a nucleotide sequence encoding a polypeptide according to any one of claims 1 to 9, with the proviso that the DNA molecule does not comprise a nucleotide sequence encoding the polypeptide consisting of the *M*. *tuberculosis* antigen recited in SEQ ID NO: 109.

12. A DNA molecule comprising a nucleotide sequence encoding a fusion protein according to claim 10.

13. A DNA molecule consisting of a nucleotide sequence encoding a polypeptide according to any one of claims 1 to 9 or a fusion protein according to claim 10.

14. An expression vector comprising a DNA molecule comprising a nucleotide sequence encoding a polypeptide comprising:
(i) an immunogenic portion of the *M*. *tuberculosis* antigen recited in SEQ ID NO: 109; or
(ii) a variant of the *M*. *tuberculosis* antigen recited in SEQ ID NO: 109 having at least 95% identity to SEQ ID NO: 109; with the proviso that the expression vector does not comprise a nucleotide sequence encoding the polypeptide consisting of the *M. tuberculosis* antigen recited in SEQ ID NO: 109.

15. An isolated host cell transformed with an expression vector according to claim 14.

16. The host cell of claim 15 wherein the host cell is selected from the group consisting of *E*. *coli,* yeast and mammalian cells.

17. A pharmaceutical composition comprising at least one polypeptide comprising:
(i) an immunogenic portion of the *M*. *tuberculosis* antigen recited in SEQ ID NO: 109; or
(ii) a variant of the *M*. *tuberculosis* antigen recited in SEQ ID NO: 109 having at least 90% identity to SEQ ID NO: 109;
and a physiologically acceptable carrier.

18. A pharmaceutical composition according to claim 17, comprising at least one polypeptide according to any one of claims 1 to 9 or a fusion protein according to claim 10 and a physiologically acceptable carrier.

19. A pharmaceutical composition according to claim 17, comprising at least one polypeptide which consists of the *M. tuberculosis* antigen recited in SEQ ID NO: 109 and a physiologically acceptable carrier.

20. A pharmaceutical composition comprising at least one DNA molecule comprising a nucleotide sequence encoding a polypeptide comprising:
(i) an immunogenic portion of the *M*. *tuberculosis* antigen recited in SEQ ID NO: 109; or
(ii) a variant of the *M*. *tuberculosis* antigen recited in SEQ ID NO: 109 having at least 90% identity to SEQ ID NO: 109
and a physiologically acceptable carrier.

21. A pharmaceutical composition according to claim 20, comprising at least one DNA molecule comprising a nucleotide sequence encoding a polypeptide according to any one of claims 1 to 9 or a fusion protein according to claim 10 and a physiologically acceptable carrier.

22. A pharmaceutical composition according to claim 20, comprising at least one DNA molecule comprising a nucleotide sequence encoding a polypeptide which consists of the *M. tuberculosis* antigen recited in SEQ I D NO: 109 and a physiologically acceptable carrier.

23. A vaccine comprising at least one polypeptide comprising:
(i) an immunogenic portion of the *M*. *tuberculosis* antigen recited in SEQ ID NO: 109; or
(ii) a variant of the *M. tuberculosis* antigen recited in SEQ ID NO: 109 having at least 90% identity to SEQ ID NO: 109
and a non-specific immune response enhancer.

24. A vaccine according to claim 23, comprising at least one polypeptide according to any one of claims 1 to 9 or a fusion protein according to claim 10 and a non-specific immune response enhancer.

25. A vaccine according to claim 23, comprising at least one polypeptide which consists of the *M*. *tuberculosis* antigen recited in SEQ ID NO: 109 and a non-specific immune response enhancer.

26. A vaccine comprising at least one DNA molecule comprising a nucleotide sequence encoding a polypeptide comprising:
(i) an immunogenic portion of the *M*. *tuberculosis* antigen recited in SEQ ID NO: 109; or
(ii) a variant of the *M*. *tuberculosis* antigen recited in SEQ ID NO: 109 having at least 90% identity to SEQ ID NO: 109
and a non-specific immune response enhancer.

27. A vaccine according to claim 26, comprising at least one DNA molecule comprising a nucleotide sequence encoding a polypeptide according to any one of claims 1 to 9 or a fusion protein according to claim 10 and a non-specific immune response enhancer.

28. A vaccine according to claim 26, comprising at least one DNA molecule comprising a nucleotide sequence encoding a polypeptide which consists of the *M. tuberculosis* antigen recited in SEQ ID NO: 109 and a non-specific immune response enhancer.

29. A vaccine according to any one of claims 23 to 28 wherein the non-specific immune response enhancer is an adjuvant.

30. A polypeptide comprising:
(i) an immunogenic portion of the *M*. *tuberculosis* antigen recited in SEQ ID NO: 109; or
(ii) a variant of the *M*. *tuberculosis* antigen recited in SEQ ID NO: 109 having at least 90% identity to SEQ ID NO: 109
for use in inducing protective immunity in a patient.

31. A DNA molecule comprising a nucleotide sequence encoding a polypeptide comprising:
(i) an immunogenic portion of the *M*. *tuberculosis* antigen recited in SEQ ID NO: 109; or
(ii) a variant of the *M. tuberculosis* antigen recited in SEQ ID NO: 109 having at least 90% identity to SEQ ID NO: 109
for use in inducing protective immunity in a patient.

32. Use of a polypeptide comprising:
(i) an immunogenic portion of the *M. tuberculosis* antigen recited in SEQ ID NO: 109; or
(ii) a variant of the *M. tuberculosis* antigen recited in SEQ ID NO: 109 having at least 90% identity to SEQ ID NO: 109
or a DNA molecule comprising a nucleotide sequence encoding said polypeptide, in the manufacture of a pharmaceutical composition for inducing protective immunity in a patient.

33. Use of a polypeptide comprising:
(i) an immunogenic portion of the *M. tuberculosis* antigen recited in SEQ ID NO: 109; or
(ii) a variant of the *M. tuberculosis* antigen recited in SEQ ID NO: 109 having at least 90% identity to SEQ ID NO: 109 or a DNA molecule comprising a nucleotide sequence encoding said polypeptide, in the manufacture of a vaccine for inducing protective immunity in a patient.

34. A diagnostic kit for detecting tuberculosis comprising:
(a) a polypeptide comprising:
(i) an immunogenic portion of the *M*. *tuberculosis* antigen recited in SEQ ID NO: 109; or
(ii) a variant of the M. *tuberculosis* antigen recited in SEQ ID NO: 109 having at least 90% identity to SEQ ID NO: 109; and
(b) apparatus sufficient to contact said polypeptide with the dermal cells of a patient.

## Patentansprüche

1. Isoliertes Polypeptid umfassend:
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 90% Identität mit SEQ. ID. NR. 109;
mit der Maßgabe, dass das isolierte Polypeptid nicht aus dem in SEQ. ID. NR. 109 wiedergegebenen M. tuberculosis-Antigen besteht.

2. Isoliertes Polypeptid gemäß Anspruch 1, welches ein M. tuberculosis-Antigen umfasst, das in SEQ. ID. NR. 109 wiedergegeben ist, oder eine Variante des M. tuberculosis-Antigens, das in SEQ. ID. NR. 109 wiedergegeben ist, mit mindestens 95% Identität mit SEQ. ID. NR. 109, mit der Maßgabe, dass das isolierte Polypeptid nicht aus dem in SEQ. ID. NR. 109 wiedergegebenen M. tuberculosis-Antigen besteht.

3. Isoliertes Polypeptid gemäß Anspruch 1, welches einen immunogenen Teil des M. tuberculosis-Antigens umfasst, welches in SEQ. ID. NR. 109 wiedergegeben ist.

4. Isoliertes Polypeptid gemäß Anspruch 3, welches eine Aminosäuresequenz umfasst, die in irgendeiner der SEQ. ID. NR. 110 bis 124 wiedergegeben ist.

5. Isoliertes Polypeptid gemäß Anspruch 4, welches eine Aminosequenz umfasst, die in SEQ. ID. NR. 119 wiedergegeben ist.

6. Isoliertes Polypeptid gemäß Anspruch 5, welches aus der Aminosäuresequenz, die in SEQ. ID. NR. 119 wiedergegeben ist, besteht.

7. Isoliertes Polypeptid gemäß Anspruch 4, welches eine Aminosäuresequenz umfasst, die in SEQ. ID. NR. 120 wiedergegeben ist.

8. Isoliertes Polypeptid gemäß Anspruch 7, welches aus der Aminosäuresequenz besteht, die in SEQ. ID. NR. 120 wiedergegeben ist.

9. Isoliertes Polypeptid gemäß Anspruch 3, welches das M. tuberculosis-Antigen, das in SEQ. ID. NR. 109 wiedergegeben ist, umfasst, mit der Maßgabe, dass das isolierte Polypeptid nicht aus dem M. tuberculosis-Antigen besteht, das in SEQ. ID. NR. 109 wiedergegeben ist.

10. Fusionsprotein umfassend ein Polypeptid umfassend:
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 90% Identität mit SEQ. ID. NR. 109;

11. DNA-Molekül umfassend eine Nukleotidsequenz, die ein Polypeptid gemäß irgendeinem der Ansprüche 1 bis 9 kodiert, mit der Maßgabe, dass das DNA-Molekül keine Nukleotidsequenz umfasst, die das Polypeptid kodiert, das aus dem M. tuberculosis-Antigen besteht, welches in SEQ. ID. NR. 109 wiedergegeben ist.

12. DNA-Molekül umfassend eine Nukleotidsequenz, die ein Fusionsprotein gemäß Anspruch 10 kodiert.

13. DNA-Molekül bestehend aus einer Nukleotidsequenz, die ein Polypeptid gemäß irgendeinem der Ansprüche 1 bis 9 oder ein Fusionsprotein gemäß Anspruch 10 kodiert.

14. Expressionsvektor umfassend ein DNA-Molekül umfassend eine Nukleotidsequenz, die ein Polypeptid kodiert, das umfasst:
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 95% Identität mit SEQ. ID. NR. 109;
mit der Maßgabe, dass der Expressionsvektor nicht eine Nukleotidsequenz umfasst, die das Polypeptid kodiert, das aus dem M. tuberculosis-Antigen besteht, die in SEQ. ID. NR. 109 wiedergegeben ist.

15. Isolierte Wirtszelle, die mit einem Expressionsvektor gemäß Anspruch 14 transformiert ist.

16. Wirtszelle gemäß Anspruch 15, wobei die Wirtszelle aus der Gruppe ausgewählt ist, die aus E. coli, Hefe und Säugerzellen besteht.

17. Pharmazeutische Zusammensetzung umfassend mindestens ein Polypeptid umfassend:
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 90% Identität mit SEQ. ID. NR. 109;
und einen physiologisch annehmbaren Träger.

18. Pharmazeutische Zusammensetzung gemäß Anspruch 17, umfassend mindestens ein Polypeptid gemäß irgendeinem der Ansprüche 1 bis 9 oder ein Fusionsprotein gemäß Anspruch 10 und einen physiologisch annehmbaren Träger.

19. Pharmazeutische Zusammensetzung gemäß Anspruch 17, welche mindestens ein Polypeptid umfasst, welches aus dem M. tuberculosis-Antigen besteht, das in SEQ. ID. NR. 109 wiedergegeben ist, und einen physiologisch annehmbaren Träger.

20. Pharmazeutische Zusammensetzung umfassend mindestens ein DNA-Molekül umfassend eine Nukleotidsequenz, die ein Polypeptid kodiert, das umfasst:
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 90% Identität mit SEQ. ID. NR. 109;
und einen physiologisch annehmbaren Träger.

21. Pharmazeutische Zusammensetzung gemäß Anspruch 20, umfassend mindestens ein DNA-Molekül umfassend eine Nukleotidsequenz, die ein Polypeptid gemäß irgendeinem der Ansprüche 1 bis 9 oder ein Fusionsprotein gemäß Anspruch 10 kodiert, und einen physiologisch annehmbaren Träger.

22. Pharmazeutische Zusammensetzung gemäß Anspruch 20, umfassend mindestens ein DNA-Molekül, das eine Nukleotidsequenz umfasst, die ein Polypeptid kodiert, welches aus dem M. tuberculosis-Antigen besteht, das in SEQ. ID. NR. 109 wiedergegeben ist, und einen physiologisch annehmbaren Träger.

23. Impfstoff umfassend mindestens ein Polypeptid umfassend:
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 90% Identität mit SEQ. ID. NR. 109;
und einen nicht spezifischen Immunantwortverstärker.

24. Impfstoff gemäß Anspruch 23 umfassend mindestens ein Polypeptid gemäß irgendeinem der Ansprüche 1 bis 9 oder ein Fusionsprotein gemäß Anspruch 10 und einen nicht spezifischen Immunantwortverstärker.

25. Impfstoff gemäß Anspruch 23 umfassend mindestens ein Polypeptid, welches aus dem M. tuberculosis-Antigen besteht, das in SEQ. ID. NR. 109 wiedergegeben ist, und einen nicht spezifischen Immunantwortverstärker.

26. Impfstoff umfassend mindestens ein DNA-Molekül umfassend eine Nukleotidsequenz, die ein Polypeptid kodiert, welches umfasst:
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 90% Identität mit SEQ. ID. NR. 109;
und einen nicht spezifischen Immunantwortverstärker.

27. Impfstoff gemäß Anspruch 26, umfassend mindestens ein DNA-Molekül umfassend eine Nukleotidsequenz, die ein Polypeptid gemäß irgendeinem der Ansprüche 1 bis 9 oder ein Fusionsprotein gemäß Anspruch 10 kodiert, und einen nicht spezifischen Immunantwortverstärker.

28. Impfstoff gemäß Anspruch 26 umfassend mindestens ein DNA-Molekül, umfassend eine Nukleotidsequenz, die ein Polypeptid kodiert, welches aus dem M. tuberculosis-Antigen besteht, welches in SEQ. ID. NR. 109 wiedergegeben ist, und einen nicht spezifischen Immunantwortverstärker.

29. Impfstoff gemäß irgendeinem der Ansprüche 23 bis 28, wobei der nicht spezifische Immunantwortverstärker ein Adjuvans ist.

30. Polypeptid umfassend
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 90% Identität mit SEQ. ID. NR. 109;
zur Verwendung bei der Induzierung einer protektiven Immunität in einem Patienten.

31. DNA-Molekül umfassend eine Nukleotidsequenz, welche ein Polypeptid kodiert, das umfasst:
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 90% Identität mit SEQ. ID. NR. 109;
zur Verwendung bei der Induzierung einer protektiven Immunität in einem Patienten.

32. Verwendung eines Polypeptids umfassend:
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 90% Identität mit SEQ. ID. NR. 109;
oder ein DNA-Molekül umfassend eine Nukleotidsequenz, die dieses Polypeptid kodiert, zur Herstellung einer pharmazeutischen Zusammensetzung zur Induzierung einer protektiven Immunität in einem Patienten.

33. Verwendung eines Polypeptids umfassend:
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 90% Identität mit SEQ. ID. NR. 109;
oder ein DNA-Molekül umfassend eine Nukleotidsequenz, die das Polypeptid kodiert, zur Herstellung eines Impfstoffs zur Induzierung einer protektiven Immunität in einem Patienten.

34. Ein diagnostischer Kit zum Nachweis von Tuberkulose umfassend:
(a) ein Polypeptid umfassend:
(i) einen immunogenen Teil des M. tuberculosis-Antigens, das in SEQ. ID. NR. 109 wiedergegeben ist; oder
(ii) eine Variante des M. tuberculosis-Antigens, das in SEQ. ID. NR. 109 wiedergegeben ist, mit mindestens 90% Identität mit SEQ. ID. NR. 109; und
(b) eine Vorrichtung, die ausreicht, um dieses Polypeptid mit den Hautzellen eines Patienten in Kontakt zu bringen.

## Revendications

1. Polypeptide isolé, qui comprend :
(i) une partie immunogène de l'antigène de *M. tuberculosis* représenté dans SEQ ID NO : 109 ; ou
(ii) un variant de l'antigène de *M. tuberculosis* représenté dans SEQ ID NO : 109
présentant une identité d'au moins 95 % avec SEQ ID NO : 109 ;
à condition que le polypeptide isolé ne soit pas constitué par l'antigène de *M. tuberculosis* représenté dans SEQ ID NO : 109.

2. Polypeptide isolé selon la revendication 1, qui comprend un antigène de *M. tuberculosis* représenté dans SEQ ID NO : 109, ou un variant de l'antigène de *M. tuberculosis* représenté dans SEQ ID NO : 109 présentant une identité d'au moins 95 % avec SEQ ID NO : 109, à condition que le polypeptide isolé ne soit pas constitué par l'antigène de *M. tuberculosis* représenté dans SEQ ID NO : 109.

3. Polypeptide isolé selon la revendication 1, qui comprend une partie immunogène de l'antigène de *M. tuberculosis* représenté dans SEQ ID NO : 109.

4. Polypeptide isolé selon la revendication 3, qui comprend une séquence d'acides aminés représentée dans l'une quelconque des SEQ ID NO : 110 à 124.

5. Polypeptide isolé selon la revendication 4, qui comprend une séquence d'acides aminés représentée dans SEQ ID NO : 119.

6. Polypeptide isolé selon la revendication 5, qui est constitué par la séquence d'acides aminés représentée dans SEQ ID NO : 119.

7. Polypeptide isolé selon la revendication 4, qui comprend une séquence d'acides aminés représentée dans SEQ ID NO : 120.

8. Polypeptide isolé selon la revendication 7, qui est constitué par la séquence d'acides aminés représentée dans SEQ ID NO : 120.

9. Polypeptide isolé selon la revendication 3, qui comprend l'antigène de *M. tuberculosis* représenté dans SEQ ID NO : 109, à condition que le polypeptide isolé ne soit pas constitué par l'antigène de *M. tuberculosis* représenté dans SEQ ID NO : 109.

10. Protéine de fusion comprenant un polypeptide, qui comprend :
(i) une partie immunogène de l'antigène de *M. tuberculosis* représenté dans SEQ ID NO : 109 ; ou
(ii) un variant de l'antigène de *M. tuberculosis* représenté dans SEQ ID NO : 109 présentant une identité d'au moins 90 % avec SEQ ID NO : 109.

11. Molécule d'ADN comprenant une séquence nucléotidique codant pour un polypeptide selon l'une quelconque des revendications 1 à 9, à condition que la molécule d'ADN ne comprenne pas de séquence nucléotidique codant pour le polypeptide constitué par l'antigène de *M. tuberculosis* représenté dans SEQ ID NO : 109.

12. Molécule d'ADN comprenant une séquence nucléotidique codant pour une protéine de fusion selon la revendication 10.

13. Molécule d'ADN constituée par une séquence nucléotidique codant pour un polypeptide selon l'une quelconque des revendications 1 à 9 ou une protéine de fusion selon la revendication 10.

14. Vecteur d'expression comprenant une molécule d'ADN comprenant une séquence nucléotidique codant pour un polypeptide, qui comprend :
(i) une partie immunogène de l'antigène de M. *tuberculosis* représenté dans SEQ ID NO : 109 ; ou
(ii) un variant de l'antigène de *M. tuberculosis* représenté dans SEQ ID NO : 109 présentant une identité d'au moins 95 % avec SEQ ID NO _{:} 109 ;
à condition que le vecteur d'expression ne comprenne pas de séquence nucléotidique codant pour le polypeptide constitué par l'antigène de *M. tuberculosis* représenté dans SEQ ID NO : 109.

15. Cellule hôte isolée transformée avec un vecteur d'expression selon la revendication 14.

16. Cellule hôte selon la revendication 15, dans laquelle la cellule hôte est choisie dans le groupe constitué par des cellules d'*E*. *coli,* de levure et de mammifères.

17. Composition pharmaceutique comprenant au moins un polypeptide comprenant :
(i) une partie immunogène de l'antigène de *M. tuberculosis* représenté dans SEQ ID NO : 109, ou
(ii) un variant de l'antigène de *M. tuberculosis* représenté dans SEQ ID NO : 109 présentant une identité d'au moins 90 % avec SEQ ID NO : 109 ;
et un véhicule physiologiquement acceptable.

18. Composition pharmaceutique selon la revendication 17, comprenant au moins un polypeptide selon l'une quelconque des revendications 1 à 9, ou une protéine de fusion selon la revendication 10 et un véhicule physiologiquement acceptable.

19. Composition pharmaceutique selon la revendication 17, comprenant au moins un polypeptide qui est constitué par l'antigène de *M. tuberculosis* représenté dans SEQ ID NO: 109 et un véhicule physiologiquement acceptable.

20. Composition pharmaceutique comprenant au moins une molécule d'ADN comprenant une séquence nucléotidique codant pour un polypeptide comprenant :
(i) une partie immunogène de l'antigène de *M. tuberculosis* représenté dans SEQ ID NO : 109 ; ou
(ii) un variant de l'antigène de *M. tuberculosis* représenté dans SEQ ID NO : 109
présentant une identité d'au moins 90 % avec SEQ ID NO : 109
et un véhicule physiologiquement acceptable.

21. Composition pharmaceutique selon la revendication 20, comprenant au moins une molécule d'ADN comprenant une séquence nucléotidique codant pour un polypeptide selon l'une quelconque des revendications 1 à 9 ou une protéine de fusion selon la revendication 10 et un véhicule physiologiquement acceptable.

22. Composition pharmaceutique selon la revendication 20, comprenant au moins une molécule d'ADN comprenant une séquence nucléotidique codant pour un polypeptide qui est constitué par l'antigène de *M. tuberculosis* représenté dans SEQ ID NO: 109 et un véhicule physiologiquement acceptable.

23. Vaccin comprenant au moins un polypeptide comprenant :
(i) une partie immunogène de l'antigène de *M. tuberculosis* représenté dans SEQ ID NO : 109 ; ou
(ii) un variant de l'antigène de *M. tuberculosis* représenté dans SEQ ID NO : 109 présentant une identité d'au moins 90 % avec SEQ ID NO : 109
et un stimulateur de la réponse immunitaire non spécifique.

24. Vaccin selon la revendication 23, comprenant au moins un polypeptide selon l'une quelconque des revendications 1 à 9 ou une protéine de fusion selon la revendication 10 et un stimulateur de la réponse immunitaire non spécifique.

25. Vaccin selon la revendication 23, comprenant au moins un polypeptide qui est constitué par l'antigène de *M. tuberculosis* représenté dans SEQ ID NO : 109 et un stimulateur de la réponse immunitaire non spécifique.

26. Vaccin comprenant au moins une molécule d'ADN comprenant une séquence nucléotidique codant pour un polypeptide comprenant .
(i) une partie immunogène de l'antigène de *M. tuberculosis* représenté dans SEQ ID NO : 109 ; ou
(ii) un variant de l'antigène de *M. tuberculosis* représenté dans SEQ ID NO : 109 présentant une identité d'au moins 90 % avec SEQ ID NO : 109
et un stimulateur de la réponse immunitaire non spécifique.

27. Vaccin selon la revendication 26, comprenant au moins une molécule d'ADN comprenant une séquence nucléotidique codant pour un polypeptide selon l'une quelconque des revendications 1 à 9 ou une protéine de fusion selon la revendication 10 et un stimulateur de la réponse immunitaire non spécifique.

28. Vaccin selon la revendication 26, comprenant au moins une molécule d'ADN comprenant une séquence nucléotidique codant pour un polypeptide qui est constitué par l'antigène de *M. tuberculosis* représenté dans SEQ ID NO : 109 et un stimulateur de la réponse immunitaire non spécifique.

29. Vaccin selon l'une quelconque des revendications 23 à 28, dans lequel le stimulateur de la réponse immunitaire non spécifique est un adjuvant.

30. Polypeptide comprenant :
(i) une partie immunogène de l'antigène de *M. tuberculosis* représenté dans SEQ ID NO : 109 ; ou
(ii) un variant de l'antigène de M. *tuberculosis* représenté dans SEQ ID NO : 109 présentant une identité d'au moins 90 % avec SEQ ID NO : 109
destiné à être utilisé pour induire une immunité protectrice chez un patient.

31. Molécule d'ADN comprenant une séquence nucléotidique codant pour un polypeptide comprenant :
(i) une partie immunogène de l'antigène de *M. tuberculosis* représenté dans SEQ ID NO : 109 ; ou
(ii) un variant de l'antigène de *M. tuberculosis* représenté dans SEQ ID NO : 109 présentant une identité d'au moins 90 % avec SEQ ID NO : 109
destinée à être utilisée pour induire une immunité protectrice chez un patient.

32. Utilisation d'un polypeptide comprenant :
(i) une partie immunogène de l'antigène de *M. tuberculosis* représenté dans SEQ ID NO : 109 ; ou
(ii) un variant de l'antigène de *M. tuberculosis* représenté dans SEQ ID NO : 109 présentant une identité d'au moins 90 % avec SEQ ID NO : 109
ou une molécule d'ADN comprenant une séquence nucléotidique codant pour ledit polypeptide, dans la fabrication d'une composition pharmaceutique destinée à induire une immunité protectrice chez un patient.

33. Utilisation d'un polypeptide qui comprend :
(i) une partie immunogène de l'antigène de *M. tuberculosis* représenté dans SEQ ID NO : 109 ; ou
(ii) un variant de l'antigène de *M. tuberculosis* représenté dans SEQ ID NO : 109 présentant une identité d'au moins 90 % avec SEQ ID NO : 109
ou une molécule d'ADN comprenant une séquence nucléotidique codant pour ledit polypeptide, dans la fabrication d'un vaccin destiné à induire une immunité protectrice chez un patient.

34. Trousse de diagnostic destiné à détecter la tuberculose, qui comprend :
(a) un polypeptide comprenant :
(i) une partie immunogène de l'antigène de *M. tuberculosis* représenté dans SEQ ID NO : 109 ; ou
(ii) un variant de l'antigène de *M. tuberculosis* représenté dans SEQ ID NO : 109 présentant une identité d'au moins 90 % avec SEQ ID NO : 109 ; et
(b) un appareil suffisant pour mettre en contact ledit polypeptide avec les cellules dermiques d'un patient.


### SEQUENCE LISTING

(1) GENERAL INFORMATION:
   (i) APPLICANTS: Alderson, Mark
      Dillon, Davin C.
      Skeiky, Yasir A.W.
      Campos-Neto, Antonio
   (ii) TITLE OF INVENTION: COMPOUND FOR IMMUNOTHERAPY AND DIAGNOSIS OF TUBERCULOSIS AND METHODS OF THEIR USE
   (iii) NUMBER OF SEQUENCES: 144
   (iv) CORRESPONDENCE ADDRESS:
      (A) ADDRESSEE: SEED and BERRY
      (B) STREET: 6300 Coumbia Center, 701 Fifth Ave.
      (C) CITY: Seattle
      (D) STATE: Washington
      (E) COUNTRY: US
      (F) ZIP: 98104
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE: 05-MAY-1998
      (C) CLASSIFICATION:
   (viii) ATTORNEY/AGENT INFORMATION:
      (A) NAME: Maki, David J.
      (B) REGISTRATION NUMBER: 31,392
      (C) REFERENCE/DOCKET NUMBER: 210121.440C1
   (ix) TELECOMMUNICATION INFORMATION:
      (A) TELEPHONE: 206-622-4900
      (B) TELEFAX: 206-682-6031
(2) INFORMATION FOR SEQ ID NO:1:
   (i) SEQUENCE CHARACTERISTICS:
      (A) LENGTH: 1886 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:1:
   CGCTCTGGTG ACCACCAACT TCTTCGGTGT CAACACCATC CCGATCGCCC TCAACGAGGC     60
   CGACTACCTG CGCATGTGGA TCCAGGCCGC CACCGTCATG AGCCACTATC AAGCCGTCGC    120
   GCACGAAATC TGGTGTCTCC ATGAATANGC CAGTTCGGGA AAGCCGTGGG CCAGTATCAC    180
   CACGGGTGCG CCGGGCTCAC CGGCCTCGAC CACTCGCAGT CGCACGCCGT TGGTATCAAC    240
   TAACCGTNCN GTANGTGCGC CCATCGTCTC ACCAAATCAC ACCGGGCACC GGCCTGAGAA    300
   GGGCTTGGGG AGCANCCAGA GGCGATTGTC GCGGGTGCTG CCGCGCATCA TTGATCGGCC    360
   GGCCGGACCA NTCGGGCCTC CCTTGACGTC CGGATCNCAC TTCCTGTGCA GCTGGCATGG    420
   CTACAGCTCA CAGTGACTGC CCCACGATTG CCGGCCAGGT CCAGTTCAAA TTCCGGTGAA    480
   TTCGCGGACA AAAGCAGCAG GTCAACCAAC CGCAGTCAGT CGAGGGTCCC AAACGTGAGC    540
   CAATCGGTGA AATGGCTTGC TGCAGTGACA CCGGTCACAG GCTTAGCCGA CAGCACCGGA    600
   ATAGCTCAGG CGGGCTATAG AGTCCTATAG AAACATTTGC TGATAGAATT AACCGCTGTC    660
   TTGGCGTGAT CTTGATACGG CTCGCCGTGC GACCGGTTGG CTCAGTAGCT GACCACCATG    720
   TAACCCATCC TCGGCAGGTG TCTACTAAGG CGAGACACCG CATTGGTGGG GCTGCATCGC    780
   AAATCGGTCC GAGCATGTAG CACTGCCGTT ATCCCGGGAT AGCAAACCAC CCGGAACCAG    840
   GGCTATCCCA GTCGCTCTCC GACGGAGGCC GTTTCGCTTT CCGTTGCCCG ATAACTCCCG    900
   AGTGGATATC GGCGTTATCA NATTCAGGCT TTTCTTCGCA AGGTACCGGT GTTCGCTATA    960
   TTCGGATATC TCGGACGGAT AATTACTAAA ACTTCAGTGG TTTAGATAAG GCCGCCGCAA   1020
   TACTTCGCCG ATCTTGCCGA GCGCAACGGA TTTCCATCGT CGGTTTTCGT CGCCTTATCA   1080
   AACATGATCG GAGATAATGA CAGATCGGCC TAGCTAGGTG TTTAGCGGAC GCGATTTAGG   1140
   ACAACCGAGA TTTGCTTTGC CTCGCAACCA TGAGAGCGCC CCGCTTCGAC GCCGAATCGG   1200
   GTGAGTGATG GTGGGTTAGC ACAGCCCTGA TTGCGCCACC GGCGAGGTGA TTGTGCCCGC   1260
   CACGAGGCCG CCGCCGGCTA GCCCCATGAG CACGNTATAT AGACTCTCCT GCAACAGATC   1320
   TCATACCGAT CGAAGGCGAA GCGCAGGCAT CGACGTCGGA GACACTGCCT TGGGATCGCG   1380
   CCGCCTACAC GGCGGTTGGC GCATTGTCGC AGCGCAGTTG CAGGAGGGCA AATGTGCGCA   1440
   GACGATGTAG TCGACAACAA GTGNACATGC CGTCTTCACG AACTCAAAAC TGACGATCTG   1500
   CTTAGCATGA AAAAAACTGT TGACATCGGC CAAGCATGAC AGCCAGACTG TAGGCCTACG   1560
   CGTGCAATGC AGAACCAAGG NTATGCATGG AATCGACGAC CGTTGAGATA GGCGGCAGGC   1620
   ATGAGCAGAG CGTTCATCAT CGATCCAACG ATCAGTGCCA TTGACGGCTT GTACGACCTT   1680
   CTGGGGATTG GAATACCCAA CCAAGGGGGT ATCCTTTACT CCTCACTAGA GTACTTCGAA   1740
   AAAGCCCTGG AGGAGCTGGC AGCAGCGTTT CCGGGTGATG GCTGGTTAGG TTCGGCCGCG   1800
   GACAAATACG CCGGCAAAAA CCGCAACCAC GTGAATTTTT TCCAGGAACT GGCAGACCTC   1860
   GATCGTCAGC TCATCAGCCT GATCCA                                        1886
(2) INFORMATION FOR SEQ ID NO: 2:
   (i) SEQUENCE CHARACTERISTICS:
      (A) LENGTH: 2305 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:2:
   GGCACGCGCT GGCCGCGCAA TACACCGAAA TTGCAACGGA ACTCGCAAGC GTGCTCGCTG     60
   CGGTGCAGGC AAGCTCGTGG CAGGGGCCCA GCGCCGACCG GTTCGTCGTC GCCCATCAAC    120
   CGTTCCGGTA TTGGCTAACC CACGCTGCCA CGGTGGCCAC CGCAGCAGCC GCCGCGCACN    180
   AAACGGCCGC CGCCGGGTAT ACGTCCGCAT TGGGGGGCAT GCCTACGCTA GCCGAGTTGG    240
   CGGCCAACCA TGCCATGCAC GGCGCTCTGG TGACCACCAA CTTCTTCGGT GTCAACACCA    300
   TCCCGATCGC CCTCAACGAG GCCGACTACC TGCGCATGTG GATCCAGGCC GCCACCGTCA    360
   TGAGCCACTA TCAAGCCGTC GCGCACGAAA GCGTGGCGGC GACCCCCAGC ACGCCGCCGG    420
   CGCCGCAGAT AGTGACCAGT GCGGCCAGCT CGGCGGCTAG CAGCAGCTTC CCCGACCCGA    480
   CCAAATTGAT CCTGCAGCTA CTCAAGGATT TCCTGGAGCT GCTGCGCTAT CTGGCTGTTG    540
   AGCTGCTGCC GGGGCCGCTC GGCGACCTCA TCGCCCAGGT GTTGGACTGG TTCATCTCGT    600
   TCGTGTCCGG TCCAGTCTTC ACGTTTCTCG CCTACCTGGT GCTGGACCCA CTGATCTATT    660
   TCGGACCGTT CGCCCCGCTG ACGAGTCCGG TCCTGTTGCC TGCTGTGGAG TTACGCAACC    720
   GCCTCAAAAC CGCCACCGGA CTGACGCTGC CACCTACCGT GATTTTCGAT CATCCCACTC    780
   CCACTGCGGT CGCCGAGTAT GTCGCCCAGC AAATGTCTGG CAGCCGCCCA ACGGAATCCG    840
   GTGATCCGAC GTCGCAGGTT GTCGAACCCG CTCGTGCCGA ATTCGGCACG AGTGCTGTTC    900
   ATCAAATCCC CCCGAGACCT GCGGACACCC GGCGCGCTTG CCGACATCGA GATGATGTCC    960
   CGCGAGATAG CAGAATTGCC CAACATCGTG ATGGTGCGGG GCTTGACCCG ACCGAACGGG   1020
   GAACCTCTGA AGGAGACCAA GGTCTCGTTT CAGGCTGGTG AAGTGGGCGG CAAGCTCGAC   1080
   GAAGCGACCA CCCTGCTCGA AGAGCACGGA GGCGAGCTGG ACCAGCTGAC CGGCGGTGCG   1140
   CACCAGTTGG CCGACGCCCT CGCCCAAATA CGCAACGAAA TCAATGGGGC CGTGGCCAGC   1200
   TCGAGCGGGA TAGTCAACAC CCTGCAGGCC ATGATGGACC TGATGGGCGG TGACAAGACC   1260
   ATCCGACAAC TGGAAAATGC GTCCCAATAT GTCGGGCGCA TGCGGGCTCT GGGGGACAAT   1320
   CTGAGCGGGA CCGTCACCGA TGCCGAACAA ATCGCCACTT GGGCCAGCCC TATGGTCAAC   1380
   GCCCTCAACT CCAGCCCGGT GTGTAACAGC GATCCCGCCT GTCGGACGTC GCGCGCACAG   1440
   TTGGCGGCGA TTGTCCAGGC GCAGGACGAC GGCCTGCTCA GGTCCATCAG AGCGCTAGCC   1500
   GTCACCCTGC AACAGACGCA GGAATACCAG ACACTCGCCC GGACGGTGAG CACACTGGAC   1560
   GGGCAACTGA AGCAAGTCGT CAGCACCCTC AAAGCGGTCG ACGGCCTACC CACCAAATTG   1620
   GCTCAAATGC AGCAAGGAGC CAACGCTCTC GCCGACGGCA GCGCAGCGCT GGCGGCAGGC   1680
   GTGCAGGAAT TGGTCGATCA GGTCAAAAAG ATGGGCTCAG GGCTCAACGA GGCCGCCGAC   1740
   TTCCTGTTGG GGATCAAGCG GGATGCGGAC AAGCCGTCAA TGGCGGGCTT CAACATTCCA   1800
   CCGCAGATTT TTTCGAGGGA CGAGTTCAAG AAGGGCGCCC AGATTTTCCT GTCGGCCGAT   1860
   GGTCATGCGG CGCGGTACTT CGTGCAGAGC GCGCTGAATC CGGCCACCAC CGAGGCGATG   1920
   GATCAGGTCA ACGATATCCT CCGTGTTGCG GATTCCGCGC GACCGAATAC CGAACTCGAG   1980
   GATGCCACGA TAGGTCTGGC GGGGGTTCCG ACTGCGCTGC GGGATATCCG CGACTACTAC   2040
   AACAGCGATA TGAAATTCAT CGTCATTGCG ACGATCGTTA TCGTATTCTT GATTCTCGTC   2100
   ATTCTGNTGC GCGCACTTGT GGNTCCGATA TATCTGATAG GCTCGGTGCT GATTTCTTAC   2160
   TTGTCGGCCC TAGGCATAGG AACTTTCGTT TTCCAATTGA TACTGGGCCA GGAAATGCAT   2220
   TGGAGCCTGC CGGGACTGTC CTTCATATTA TTGGTTGCCA TCGGCGCTGA CTACAACATG   2280
   CTGCTCATTT CACGCATCCG CGACG                                         2305
(2) INFORMATION FOR SEQ ID NO:3:
   (i) SEQUENCE CHARACTERISTICS:
      (A) LENGTH: 1742 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:3:
   CCGCTCTCTT TCAACGTCAT AAGTTCGGTG GGCCAGTCGG CCGCGCGTGC ATATGGCACC      60
   AATAACGCGT GTCCCATGGA TACCCGGACC GCACGACGGT AGAGCGGATC AGCGCAGCCG     120
   GTGCCGAACA CTACCGCGTC CACGCTCAGC CCTGCCGCGT TGCGGAAGAT CGAGCCCAGG     180
   TTCTCATGGT CGTTAACGCC TTCCAACACT GCGACGGTGC GCGCCCCGGC GACCACCTGA     240
   GCAACGCTCG GCTCCGGCAC CCGGCGCGCG GCTGCCAACA CCCCACGATT GAGATGGAAG     300
   CCGATCACCC GTGCCATGAC ATCAGCCGAC GCTCGATAGT ACGGCGCGCC GACACCGGCC     360
   AGATCATCCT TGAGCTCGGC CAGCCGGCGG TCGGTGCCGA ACAGCGCCAG CGGCGTGAAC     420
   CGTGAGGCCA GCATGCGCTG CACCACCAGC ACACCCTCGG CGATCACCAA CGCCTTGCCG     480
   GTCGGCAGAT CGGGACNACN GTCGATGCTG TTCAGGTCAC GGAAATCGTC GAGCCGTGGG     540
   TCGTCGGGAT CGCAGACGTC CTGAACATCG AGGCCGTCGG GGTGCTGGGC ACAACGGCCT     600
   TCGGTCACGG GCTTTCGTCG ACCAGAGCCA GCATCAGATC GGCGGCGCTG CGCAGGATGT     660
   CACGCTCGCT GCGGTTCAGC GTCGCGAGCC GCTCAGCCAG CCACTCTTGC AGAGAGCCGT     720
   TGCTGGGATT AATTGGGAGA GGAAGACAGC ATGTCGTTCG TGACCACACA GCCGGAAGCC     780
   CTGGCAGCTG CGGCGGCGAA CCTACAGGGT ATTGGCACGA CAATGAACGC CCAGAACGCG     840
   GCCGCGGCTG CTCCAACCAC CGGAGTAGTG CCCGCAGCCG CCGATGAAGT ATCAGCGCTG     900
   ACCGCGGCTC AGTTTGCTGC GCACGCGCAG ATGTACCAAA CGGTCAGCGC CCAGGCCGCG     960
   GCCATTCACG AAATGTTCGT GAACACGCTG GTGGCCAGTT CTGGCTCATA CGCGGCCACC    1020
   GAGGCGGCCA ACGCAGCCGC TGCCGGCTGA ACGGGCTCGC ACGAACCTGC TGAAGGAGAG    1080
   GGGGAACATC CGGAGTTCTC GGGTCAGGGG TTGCGCCAGC GCCCAGCCGA TTCAGNTATC    1140
   GGCGTCCATA ACAGCAGACG ATCTAGGCAT TCAGTACTAA GGAGACAGGC AACATGGCCT    1200
   CACGTTTTAT GACGGATCCG CATGCGATGC GGGACATGGC GGGCCGTTTT GAGGTGCACG    1260
   CCCAGACGGT GGAGGACGAG GCTCGCCGGA TGTGGGCGTC CGCGCAAAAC ATTTCCGGTG    1320
   CGGGCTGGAG TGGCATGGCC GAGGCGACCT CGCTAGACAC CATGACCTAG ATGAATCAGG    1380
   CGTTTCGCAA CATCGTGAAC ATGCTGCACG GGGTGCGTGA CGGGCTGGTT CGCGACGCCA    1440
   ACAANTACGA ACAGCAAGAG CAGGCCTCCC AGCAGATCCT GAGCAGNTAG CGCCGAAAGC    1500
   CACAGCTGNG TACGNTTTCT CACATTAGGA GAACACCAAT ATGACGATTA ATTACCAGTT    1560
   CGGGGACGTC GACGCTCATG GCGCCATGAT CCGCGCTCAG GCGGCGTCGC TTGAGGCGGA    1620
   GCATCAGGCC ATCGTTCGTG ATGTGTTGGC CGCGGGTGAC TTTTGGGGCG GCGCCGGTTC    1680
   GGTGGCTTGC CAGGAGTTCA TTACCCAGTT GGGCCGTAAC TTCCAGGTGA TCTACGAGCA    1740
   GG                                                                   1742
(2) INFORMATION FOR SEQ ID NO:4:
   (i) SEQUENCE CHARACTERISTICS:
      (A) LENGTH: 2836 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:4:
   GTTGATTCCG TTCGCGGCGC CGCCGAAGAC CACCAACTCC GCTGGGGTGG TCGCACAGGC     60
   GGTTGCGTCG GTCAGCTGGC CGAATCCCAA TGATTGGTGG CTCNGTGCGG TTGCTGGGCT    120
   CGATTACCCC CACGGAAAGG ACGACGATCG TTCGTTTGCT CGGTCAGTCG TACTTGGCGA    180
   CGGGCATGGC GCGGTTTCTT ACCTCGATCG CACAGCAGCT GACCTTCGGC CCAGGGGGCA    240
   CAACGGCTGG CTCCGGCGGA GCCTGGTACC CAACGCCACA ATTCGCCGGC CTGGGTGCAG    300
   GCCCGGCGGT GTCGGCGAGT TTGGCGCGGG CGGAGCCGGT CGGGAGGTTG TCGGTGCCGC    360
   CAAGTTGGGC CGTCGCGGCT CCGGCCTTCG CGGAGAAGCC TGAGGCGGGC ACGCCGATGT    420
   CCGTCATCGG CGAAGCGTCC AGCTGCGGTC AGGGAGGCCT GCTTCGAGGC ATACCGCTGG    480
   CGAGAGCGGG GCGGCGTACA GGCGCCTTCG CTCACCGATA CGGGTTCCGC CACAGCGTGA    540
   TTACCCGGTC TCCGTCGGCG GGATAGCTTT CGATCCGGTC TGCGCGGCCG CCGGAAATGC    600
   TGCAGATAGC GATCGACCGC GCCGGTCGGT AAACGCCGCA CACGGCACTA TCAATGCGCA    660
   CGGCGGGCGT TGATGCCAAA TTGACCGTCC CGACGGGGCT TTATCTGCGG CAAGATTTCA    720
   TCCCCAGCCC GGTCGGTGGG CCGATAAATA CGCTGGTCAG CGCGACTCTT CCGGCTGAAT    780
   TCGATGCTCT GGGCGCCCGC TCGACGCCGA GTATCTCGAG TGGGCCGCAA ACCCGGTCAA    840
   ACGCTGTTAC TGTGGCGTTA CCACAGGTGA ATTTGCGGTG CCAACTGGTG AACACTTGCG    900
   AACGGGTGGC ATCGAAATCA ACTTGTTGCG TTGCAGTGAT CTACTCTCTT GCAGAGAGCC    960
   GTTGCTGGGA TTAATTGGGA GAGGAAGACA GCATGTCGTT CGTGACCACA CAGCCGGAAG   1020
   CCCTGGCAGC TGCGGCGGCG AACCTACAGG GTATTGGCAC GACAATGAAC GCCCAGAACG   1080
   CGGCCGCGGC TGCTCCAACC ACCGGAGTAG TGCCCGCAGC CGCCGATGAA GTATCAGCGC   1140
   TGACCGCGGC TCAGTTTGCT GCGCACGCGC AGATGTACCA AACGGTCAGC GCCCAGGCCG   1200
   CGGCCATTCA CGAAATGTTC GTGAACACGC TGGTGGCCAG TTCTGGCTCA TACGCGGCCA   1260
   CCGAGGCGGC CAACGCAGCC GCTGCCGGCT GAACGGGCTC GCACGAACCT GCTGAAGGAG   1320
   AGGGGGAACA TCCGGAGTTC TCGGGTCAGG GGTTGCGCCA GCGCCCAGCC GATTCAGCTA   1380
   TCGGCGTCCA TAACAGCAGA CGATCTAGGC ATTCAGTACT AAGGAGACAG GCAACATGGC   1440
   CTCACGTTTT ATGACGGATC CGCATGCGAT GCGGGACATG GCGGGCCGTT TTGAGGTGCA   1500
   CGCCCAGACG GTGGAGGACG AGGCTCGCCG GATGTGGGCG TCCGCGCAAA ACATTTCCGG   1560
   TGCGGGCTGG AGTGGCATGG CCGAGGCGAC CTCGCTAGAC ACCATGACCT AGATGAATCA   1620
   GGCGTTTCGC AACATCGTGA ACATGCTGCA CGGGGTGCGT GACGGGCTGG TTCGCGACGC   1680
   CAACAACTAC GAACAGCAAG AGCAGGCCTC CCAGCAGATC CTGAGCAGCT AGCGCCGAAA   1740
   GCCACAGCTG CGTACGCTTT CTCACATTAG GAGAACACCA ATATGACGAT TAATTACCAG   1800
   TTCGGGGACG TCGACGCTCA TGGCGCCATG ATCCGCGCTC AGGCGGCGTC GCTTGAGGCG   1860
   GAGCATCAGG CCATCGTTCG TGATGTGTTG GCCGCGGGTG ACTTTTGGGG CGGCGCCGGT   1920
   TCGGTGGCTT GCCAGGAGTT CATTACCCAG TTGGGCCGTA ACTTCCAGGT GATCTACGAG   1980
   CAGGCCAACG CCCACGGGCA GAAGGTGCAG GCTGCCGGCA ACAACATGGC GCAAACCGAC   2040
   AGCGCCGTCG GCTCCAGCTG GGCCTAAAAC TGAACTTCAG TCGCGGCAGC ACACCAACCA   2100
   GCCGGTGTGC TGCTGTGTCC TGCAGTTAAC TAGCACTCGA CCGCTGAGGT AGCGATGGAT   2160
   CAACAGAGTA CCCGCACCGA CATCACCGTC AACGTCGACG GCTTCTGGAT GCTTCAGGCG   2220
   CTACTGGATA TCCGCCACGT TGCGCCTGAG TTACGTTGCC GGCCGTACGT CTCCACCGAT   2280
   TCCAATGACT GGCTAAACGA GCACCCGGGG ATGGCGGTCA TGCGCGAGCA GGGCATTGTC   2340
   GTCAACGACG CGGTCAACGA ACAGGTCGCT GCCCGGATGA AGGTGCTTGC CGCACCTGAT   2400
   CTTGAAGTCG TCGCCCTGCT GTCACGCGGC AAGTTGCTGT ACGGGGTCAT AGACGACGAG   2460
   AACCAGCCGC CGGGTTCGCG TGACATCCCT GACAATGAGT TCCGGGTGGT GTTGGCCCGG   2520
   CGAGGCCAGC ACTGGGTGTC GGCGGTACGG GTTGGCAATG ACATCACCGT CGATGACGTG   2580
   ACGGTCTCGG ATAGCGCCTC GATCGCCGCA CTGGTAATGG ACGGTCTGGA GTCGATTCAC   2640
   CACGCCGACC CAGCCGCGAT CAACGCGGTC AACGTGCCAA TGGAGGAGAT CTCGTGCCGA   2700
   ATTCGGCACG AGGCACGAGG CGGTGTCGGT GACGACGGGA TCGATCACGA TCATCGACCG   2760
   GCCGGGATCC TTGGCGATCT CGTTGAGCAC GACCCGGGCC CGCGGGAAGC TCTGCGACAT   2820
   CCATGGGTTC TTCCCG                                                   2836
(2) INFORMATION FOR SEQ ID NO:5:
   (i) SEQUENCE CHARACTERISTICS:
      (A) LENGTH: 900 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:5:
   AACATGCTGC ACGGGGTGCG TGACGGGCTG GTTCGCGACG CCAACAACTA CGAGCAGCAA      60
   GAGCAGGCCT CCCAGCAGAT CCTCAGCAGC TAACGTCAGC CGCTGCAGCA CAATACTTTT     120
   ACAAGCGAAG GAGAACAGGT TCGATGACCA TCAACTATCA GTTCGGTGAT GTCGACGCTC     180
   ACGGCGCCAT GATCCGCGCT CAGGCCGGGT TGCTGGAGGC CGAACATCAG GCCATCATTC     240
   GTGATGTGTT GACCGCGAGT GACTTTTGGG GCGGCGCCGG TTCGGCGGCC TGCCAGGGGT     300
   TCATTACCCA ATTGGGCCGT AACTTCCAGG TGATCTACGA ACAGGCCAAC GCCCACGGGC     360
   AGAAGGTGCA GGCTGCCGGC AACAACATGG CGCAAACCGA CAGCGCCGTC GGCTCCAGCT     420
   GGGCCTGACA CCAGGCCAAG GCCAGGGACG TGGTGTACGA GTGAAGGTTC CTCGCGTGAT     480
   CCTTCGGGTG GCAGTCTAGG TGGTCAGTGC TGGGGTGTTG GTGGTTTGCT GCTTGGCGGG     540
   TTCTTCGGTG CTGGTCAGTG CTGCTCGGGC TCGGGTGAGG ACCTCGAGGC CCAGGTAGCG     600
   CCGTCCTTCG ATCCATTCGT CGTGTTGTTC GGCGAGGACG GCTCCGACGA GGCGGATGAT     660
   CGAGGCGCGG TCGGGGAAGA TGCCCACGAC GTCGGTTCGG CGTCGTACCT CTCGGTTGAG     720
   GCGTTCCTGG GGGTTGTTGG ACCAGATTTG GCGCCAGATC TTCTTGGGGA AGGCGGTGAA     780
   CGCCAGCAGG TCGGTGCGGG CGGTGTCGAN GTGCTCGGCC ACCGCGGGGA GTTTGTCGGT     840
   CAGAGCGTCG AGTACCCGAT CATATTGGGC AACAACTGAT TCGGCGTTGG GCTGGTCGTA     900
(2) INFORMATION FOR SEQ ID NO:6:
   (i) SEQUENCE CHARACTERISTICS:
      (A) LENGTH: 1905 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:6:
   GCTCGCCGGA TGTGGGCGTC CGCGCAAAAC ATTTCCGGTG CGGGCTGGAG TGGCATGGCC    60
   GAGGCGACCT CGCTAGACAC CATGGCCCAG ATGAATCAGG CGTTTCGCAA CATCGTGAAC   120
   ATGCTGCACG GGGTGCGTGA CGGGCTGGTT CGCGACGCCA ACAACTACGA GCAGCAAGAG   180
   CAGGCCTCCC AGCAGATCCT CAGCAGCTAA CGTCAGCCGC TGCAGCACAA TACTTTTACA   240
   AGCGAAGGAG AACAGGTTCG ATGACCATCA ACTATCAGTT CGGTGATGTC GACGCTCACG   300
   GCGCCATGAT CCGCGCTCAG GCCGGGTTGC TGGAGGCCGA GCATCAGGCC ATCATTCGTG   360
   ATGTGTTGAC CGCGAGTGAC TTTTGGGGCG GCGCCGGTTC GGCGGCCTGC CAGGGGTTCA   420
   TTACCCAGTT GGGCCGTAAC TTCCAGGTGA TCTACGAACA AGCCAACACC CACGGGCAGA   480
   AGGTGCAAGC TGCCGGCAAC AACATGGCGC AAACCGACAG CGCCGTCNGC TCCAGCTGGG   540
   CCTGACACCA GGCCAAGGCC AGGGACGTGG TGTACNAGTG AAGGTTCCTC GCGTGATCCT   600
   TCGGGTGGCA GTCTAGGTGG TCAGTGCTGG GGTGTTGGTG GTTTGCTGCT TGGCGGGTTC   660
   TTCGGTGCTG GTCAGTGCTG CTCGGGCTCG GGTGAGGACC TCGAGGCCCA GGTAGCGCCG   720
   TCCTTCGATC CATTCGTCGT GTTGTTCGGC GAGGACNGCT CCGACGANGC GGATGATCGA   780
   GGCGCGGTCG GGGAAGATGC CCACGACGTC GGTTCGGCGT CGTACCTCTC GGTTGAAGCG   840
   TTCCTGGGGG CCACCGCTTG GCGCCNANGC ACTCCACGCC AATTCGTCNC ACCTAACAGC   900
   GGTGGCCAAC GACTATGACT ACGACACCGT TTTTGCCAGG GCCCTCNAAA GGATCTGCGC   960
   GTCCCGGCGA CACGCTTTTT GCGATAAGTA CCTCCGGCAA TTCTATGAGT GTACTGCGGN  1020
   CCGCGAAAAC CGCAAGGGAG TTGGGTGTGA CGGTTNTTGC AAATGACGGG CGAATCCGGC  1080
   GGCCAGCTGG CAGAATTCGC AGATTTCTTG ATCAACGTCC CGTCACGCGA CACCGGGCGA  1140
   ATCCAGGAAT CTCACATCGT TTTTATTCAT GCGATCTCCG AACATGTCGA ACACGCGCTT  1200
   TTCGCGCCTC GCCAATAGGA AAGCCGATCC TTACGCGGCC ATTCGAAAGA TGGTCGCGGA  1260
   ACGTGCGGGA CACCAATGGT GTCTCTTCCT CGATAGAGAC GGGGTCATCA ATCGACAAGT  1320
   GGTCGGCGAC TACGTACGGA ACTGGCGGCA GTTTGAATGG TTGCCCGGGG CGGCGCGGGC  1380
   GTTGAAGAAG CTACGGGCAT GGGCTCCGTA CATCGTTGTC GTGACAAACC AGCAGGGCGT  1440
   GGGTGCCGGA TTGATGAGCG CCGTCGACGT GATGGTGATA CATCGGCACC TCCAAATGCA  1500
   GCTTGCATCC GATGGCGTGC TGATAGATGG ATTTCAGGTT TGCCCGCACC ACCGTTCGCA  1560
   GCGGTGTGGC TGCCGTAAGC CGAGACCGGG TCTGGTCCTC GACTGGCTCG GACGACACCC  1620
   CGACAGTGAG CCATTGCTGA GCATCGTGGT TGGGGACAGC CTCAGCGATC TTGACATTGG  1680
   CACACAACGT CGCCGCTGCT GCCGGTGCAT GTGCCAGTGT CCAGATAGGG GGCGCCAGTT  1740
   CTGGCGGTGT CGCTGACGCG TCATTTGACT CGCTCTGGGA GTTCGCTGTC GCAGTCGGAC  1800
   ATGCGCGGGG GGAGCGGGGC TAATGGCGAT CTTGCGCGGG CGAGCGCCGT NGCGGNTCGG  1860
   ACTNNGCGGT GGCGGGACAG ACGTGGAACC GTACTCGAGC CAGTT                  1905
(2) INFORMATION FOR SEQ ID NO:7:
   (i) SEQUENCE CHARACTERISTICS:
      (A) LENGTH: 2921 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:7:
   CGGGATGCCG TGGTGGTTGG TATTGCCCAA ACCCTGGCGC TGGTCCCCGG GGTATCCAGG     60
   TCCGGGTCGA CCATCAGCGC TGGACTGTTT CTCGGACTCG ACCGTGAACT GGCCGCCCGA    120
   TTCGGATTCC TGCTGGCCAT TCCAGCGGTG TTCGCCTCCG GGTTGTTCTC GTTGCCCGAC    180
   GCATTCCACC CGGTAACCGA GGGCATGAGC GCTACTGGCC CGCAGTTGCT GGTGGCCACC    240
   CTGATCGCGT TCGTCCTCGG TCTGACCGCG GTGGCCTGGC TGCTGCGGTT TCTGGTGCGA    300
   CACAACATGT ACTGGTTCGT CGGCTACCGG GTGCTCGTCG GGACGGGCAT GCTCGTGCTG    360
   CTGGCTACCG GGACGGTAGC CGCGACATGA CCGTCATCTT GCTACGCCAT GCCCGTTCCA    420
   CCTCGAACAC CGCGGGCGTG CTGGCCGGCC GGTCCGGCGT CGACCTCGAC GAGAAGGGGC    480
   GCGAGCAGGC CACCGGGTTG ATCGATCGAA TTGGTGACCT GCCGATCCGG GCGGTCGCGT    540
   CTTCTCCAAT GCTGCGGTGT CAACGCACCG TCGAACCGCT GGCCGAGGCG CTGTGCCTGG    600
   AGCCGCTCAT CGATGACCGG TTCTCCGAAG TCGACTACGG CGAATGGACT GGCAGAAAAA    660
   TCGGTGACCT GGTCGACGAG CCGTTGTGGC GGGTAGTCCA GGCCCACCCC AGCGCGGCGG    720
   TGTTTCCCGG CGGTGAGGGT TTGGCGCAGG TGCAGACGTG GTTGTCCTGA CGGATTTCCA    780
   TGCCGGGGAA CACCAAGACC GGATCGGCAC TGGCGGTCGC CGGCGAAAAC CCGGCCGCCA    840
   ATAGGGCGAC CGTCGCTGCG AATGCGCGTG GTACCAGGCG GACCACCTTG AACTCCCATC    900
   CGTCGGGGCC AAGCGCATCG CCCGCCGCCG GTTACGGCTA AGGCGTACCA AAACCCGACG    960
   GTAATACTTC GGCAATGTCG GGTCNCGACG TTACCGAGAC GTGACCAGNG AGGCNGCGGC   1020
   ATTGGATTTA TCGATGGTGC GCGGTTCCCA NCCCGGCGGT CCGAANACGT AGCCCAGCCG   1080
   ATCCCGCAGA CGTGTTGCCG ACCGCCAGTC ACGCACGATC GCCACGTACT CGCGGGTCTG   1140
   CAGCTTCCAG ATGTTGAACG TGTCGACCCG CTTGGTCAGG CCATAATGCG GTCGGAATAG   1200
   CTCCGGCTGA AAGCTACCGA ACAGGCGGTC CCAGATGATG AGGATGCCGC CATAGTTCTT   1260
   GTCCANATAC ACCGGGTCCA TTCCGTGGTG GACCCGGTGG TGCGACGGGG TATTGAAGAC   1320
   GAATTCGAAC CACCGCGGCA GCCTGTCGAT CCGCTCGGTG TGCACCCAGA ACTGGTAGAT   1380
   CAAGTTCAGC GACCAATTGC AGAACACCAT CCAAGGGGGA AGCCCCATCA GTGGCAGCGG   1440
   AACCCACATG AGAATCTCGC CGCTGTTGTT CCANTTTCTG GCGCAGCGCG GTGGCGAAGT   1500
   TGAAGTATTC GCTGGAGTGA TGCGCCTGGT GGGTAGCCCA GATCAGCCGA ACTCGGTGGG   1560
   CGATGCGGTG ATAGGAGTAG TACAGCAGAT CGACACCAAC GATCGCGATC ACCCAGGTGT   1620
   ACCACCGGTG GGCGGACAGC TGCCAGGGGG CAAGGTAGGC ATAGATTGCG GCATAACCGA   1680
   GCAGGGCAAG GGACTTCCAG CCGGCGGTGG TGGCTATCGA AACCAGCCCC ATCGAGATGC   1740
   TGGCCACCGA GTCGCGGGTG AGGTAAGCGC CCGAGGCGGG CCGTGGCTGC CCGGTAGCAG   1800
   CGGTCTCGAT GCTTTCCAGC TTGCGGGCCG CCGTCCATTC GAGAATCAGC AGCAATAGAA   1860
   AACATGGAAT GGCGAACAGT ACCGGGTCCC GCATTTCCTC GGGCAGCGCT GAGAAGAATC   1920
   CGGCGACGGC ATGGCCGAGG CGACCTCGNT AGACACCATG ACCCAGATGA ATCAGGCGTT   1980
   TCGCAACATC GTGAACATGC TGCACGGGGT GCGTGACGGG CTGGTTCGCG ACGCCAACAA   2040
   NTACGAACAG CAAGAGCAGG CCTCCCAGCA GATCCTCAGC AGCTGACCCG GCCCGACGAC   2100
   TCAGGAGGAC ACATGACCAT CAACTATCAA TTCGGGGACG TCGACGCTCA CGGCGCCATG   2160
   ATCCGCGCTC AGGCCGGGTC GCTGGAGGCC GAGCATCAGG CCATCATTTC TGATGTGTTG   2220
   ACCGCGAGTG ACTTTTGGGG CGGCGCCGGT TCGGCGGCCT GCCAGGGGTT CATTACCCAG   2280
   CTGGGCCGTA ACTTCCAGGT GATNTACGAG CAGGCCAACG CCCACGGGCA GAAGGTGCAG   2340
   GCTGCCGGCA ACAACATGGC ACAAACCGAC AGCGCCGTCG GCTCCAGCTG GGCATAAAGN   2400
   TGGCTTAAGG CCCGCGCCGT CAATTACAAC GTGGCCGCAC ACCGGTTGGT GTGTGGCCAC   2460
   GTTGTTATCT GAACGACTAA CTACTTCGAC CTGCTAAAGT CGGCGCGTTG ATCCCCGGTC   2520
   GGATGGTGCT GAACTGGGAA GATGGCCTCA ATGCCCTTGT TGCGGAAGGG ATTGAGGCCA   2580
   TCGTGTTTCG TACTTTAGGC GATCAGTGCT GGTTGTGGGA GTCGCTGCTG CCCGACGAGG   2640
   TGCGCCGACT GCCCGAGGAA CTGGCCCGGG TGGACGCATT GTTGGACGAT CCGGCGTTCT   2700
   TCGCCCCGTT CGTGCCGTTC TTCGACCCGC GCAGGGGCCG GCCGTCGACG CCGATGGAGG   2760
   TCTATCTGCA GTTGATGTTT GTGAAGTTCC GCTACCGGCT GGGCTATGAG TCGCTGTGCC   2820
   GGGAGGTGGC TGATTCGATC ACCTGACGGC GGTTTTGCCG CATTGCGCTG GACGGGTCGG   2880
   TGCCGCATCC GACCACATTG ATGAAGCTCA CCACGCGTTG C                       2921
(2) INFORMATION FOR SEQ ID NO:8:
   (i) SEQUENCE CHARACTERISTICS:
      (A) LENGTH: 1704 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:8:
   CGCGATCGTC GTCAACGANG TCGACCGTCA CCACGGACTG ATCAACAAGT TCGCAGGCGA     60
   CGCCGCCCTG GCCATCTTCG GAGCCCCGAA CCGCCTCGAC CGTCCCGAAG ACGCCGCGCT    120
   GGCCGCCGCC CGGGCCATAN CCGANCGGCT GGCCNACGAG ATGCCCGAGG TCCAAGCCGG    180
   CATCGGGGTG GCGGCAGGCC ANATCGTCGC CGGCAATGTC GGCGCCAAGC AAAGATTCNA    240
   ATACACAGTG GTCGGCAAGC CGGTCAACCA NGCGGCCCGA TTGTGCGAAC TGGCCAAATC    300
   ACACCCCGCG CGATTGGGTC TCGCCCGCTC GGCTCATGGT CACCCAATTC AAGGACTACT    360
   TTGGCCTGGC GCACGACCTG CCGAAGTGGG CGAGTGAAGG CGCCAAAGCC GCCGGTGAGG    420
   CCGCCAAGGC GTTGCCGGCC GCCGTTCCGG CCATTCCGAG TGCTGGCCTG AGCGGCGTTG    480
   CGGGCGCCGT CGGTCAGGCG GCGTCGGTCG GGGGATTGAA GGTTCCGGCC GTTTGGACCG    540
   CCACGACCCC GGCGGCGAGC CCCGCGGTGC TGGCGGCGTC CAACGGCCTC GGAGCCGCGG    600
   CCGCCGCTGA AGGTTCGACA CACGCGTTTG GCGGGATGCC GCTCATGGGT ANCGGTGCCG    660
   GACGTGCGTT TAACAACTTC GCTGCCCCTC GATACGGATT CAAGCCGACC GTGATCGCCC    720
   AACCGCCGGC TGGCGGATGA CCAACTACGT TCGTTGATCG AGGATCGAAT TCNACGATTC    780
   AAAGGGAGGA ATTCATATGA CCTCNCGTTT TATGACGGAT CCGCACGCNA TNCGGGACAT    840
   GGCGGGCCGT TTTGAGGTGC ACGCCCAGAC GGTGGAGGAC GAGGCTNGCN GGATGTGGGC    900
   GTCCGCGCAA AACATTTCCG GTGCGGGCTG GAGTGGCATG GCCGAGGCGA CCTCGNTAGA    960
   CACCATGGCC CAGATGAATC AGGCGTTTCN CAACATCGTG AACATGCTGC ACGGGGTGNG   1020
   TGACGGGCTG GTTCGCGACG CCAACAACTA CGAACAGCAA GAGCAGGCCT CCCAGCAGAT   1080
   CCTCAGCAGC TGACCCGGCC CGACGACTCA GGAGGACACA TGACCATCAA CTATCAATTC   1140
   GGGGACGTCG ACGCTCATGG CGCCATGATC CGCGCTNTGG CCGGGTTGCT GGAGGCCGAG   1200
   CATCAGGCCA TCATTTCTGA TGTGTTGACC GCGAGTGACT TTTGGGGCGG CGCCGGTTCG   1260
   GCGGCCTGCC AGGGGTTCAT TACCCAGTTG GGCCGTAACT TCCAGGTGAT TTACGAGCAG   1320
   GCCAACGCCC ACGGGCAGAA GGTGCAGGCT GCCGGCAACA ACATGGCACA AACCGACAGC   1380
   GCCGTNGGNT CCAGCTGGGC CTAACCCGGG TCNTAAGTTG GGTCCGCGCA GGGCGGGCCG   1440
   ATCAGCGTNG ACTTTGGCGC CCGATACACG GGCATNTTNT NGTCGGGAAC ACTGCGCCCG   1500
   CGTCAGNTGC CCGCTTCCCC TTGTTNGGCG ACGTGCTCGG TGATGGCTTT GACGACCGCT   1560
   TCGCCGGCGC GGCCAATCAA TTGGTCGCGC TTGCCTNTAG CCCATTCGTG CGACGCCCGC   1620
   GGCGCCGCGA GTTGTCCCTT GAAATAAGGA ATCACAGCAC GGGCGAACAG CTCATAGGAG   1680
   TGAAAGGTTG CCGTGGCGGG GCCC                                          1704
(2) INFORMATION FOR SEQ ID NO:9:
   (i) SEQUENCE CHARACTERISTICS:
      (A) LENGTH: 2286 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:9:
   CCGTCTTGGC GTCTGGGCGC ATTGTGATCT GGGCCANTTG CCCCTCCACC CAGACCGCGC     60
   CCAGCTTGTC GATCCAGCCC GCGACCCGGA TTGCCACCGC GCGAACCGGG AACGGATTCT    120
   CCGCTGAATT CTGGGTCACT TCGCAGTCGC GCGGGTGATC CTGTTGGCGA NCAGCGTCTG    180
   GAACGGGCGT CNAACGCGTG CCGTAAGCCC AGCGTGTACG CCGTCAGCCC GACGCCGATG    240
   CCGAATGCCT TGCCGCCCAA GCTGAGCCGC GCGGGCTCCA CCAAGAGCGT CACGGTGAGC    300
   CAGCCAACCA GATGCAAGGC GACGATCACC GCGAAGTGCC GAATTCGGCA CGAGAGGTGC    360
   TGGAAATCCA GCAATACGCC CGCGAGCCGA TCTCGTTGGA CCAGACCATC GGCGACGANG    420
   GCGACAGNCA GCTTGGCGAT TTCATCGAAA ACAGCGAGGC GGTGGTGGNC GTCGACGCGG    480
   TGTCCTTCAC TTTGCTGCAT GATCAACTGC ANTCGGTGCT GGACACGCTC TCCGAGCGTG    540
   AGGCGGGCGT GGTGCGGCTA CGCTTCGGCC TTACCGACGG CCAGCCGCGC ACCCTTGACG    600
   AGATCGGCCA GGTCTACGGC GTGACCCGGG AACGCATCCG CCAGATCGAA TCCAAGACTA    660
   TGTCGAAGTT GCGCCATCCG AGCCGCTCAC AGGTCCTGCG CGACTATCGT GCCGAATTCG    720
   GCACGAGCCG TTTTGAGGTG CACGCCCAGA CGGTGGAGGA CGAGGCTCGC CGGATGTGGG    780
   CGTCCGCGCA AAACATTTCC GGTGCGGGCT GGAGTGGCAT GGCCGANGCG ACCTCGCTAG    840
   ACACCATGGC CCAGATGAAT CAGGCGTTTC GCAACATCGT GAACATGCTG CACGGGGTGC    900
   GTGACGGGCT GGTTCGCGAC GCCAACAACT ACGAACAGCA AGAGCAGGCC TCCCAGCAGA    960
   TCCTCAGCAG CTGACCCGGC CCGACGACTC AGGAGGACAC ATGACCATCA ACTATCAATT   1020
   CGGGGACGTC GACGCTCATG GCGCCATGAT CCGCGCTCTG GCCGGGTTGC TGGAGGCCGA   1080
   GCATCAGGCC ATCATTTCTG ATGTGTTGAC CGCGAGTGAC TTTTGGGGCG GCGCCGGTTC   1140
   GGCGGCCTGC CAGGGGTTCA TTACCCAGTT GGGCCGTAAC TTCCAGGTGA TCTACGAGCA   1200
   GGCCAACGCC CACGGGCAGA AGGTGCAGGC TGCCGGCAAC AACATGGCAC AAACCGACAG   1260
   CGCCGTCGGC TCCAGCTGGG CCTAACCCGG GTCCTAAGTT GGGTCCGCGC AGGGCGGGCC   1320
   GATCAGCGTC GACTTTGGCG CCCGATACAC GGGCATGTNG TNGTCGGGAA CACTGCGCCC   1380
   GCGTCAGCTG CCCGCTTCCC CTTGTTCGGC GACGTGCTCG GTGATGGCTT TGACGACCGC   1440
   TTCGCCGGCG CGGCCAATCA ATTGGTCGCG CTTGCCTCTA GCCTCGTGCC GAATTCGGCA   1500
   CGAGGGTGCT GGTGCCGCGC TATCGGCAGC ACGTGAGCTC CACGACGAAC TCATCCCAGT   1560
   GCTGGGTTCC GCGGAGTTCG GCATCGGCGT GTCGGCCGGA AGGGCCATCG CCGGCCACAT   1620
   CGGCGCTCAA GCCCGCTTCG AGTACACCGT CATCGGCGAC CCGGTCAACG AGGCCGCCCG   1680
   GCTCACCGAA CTGGCCAAAG TCGAGGATGG CCACGTTCTG GCGTCGGCGA TCGCGGTCAG   1740
   TGGCGCCCTG GACGCCGAAG CATTGTGTTG GGATGTTGGC GAGGTGGTTG AGCTCCGCGG   1800
   ACGTGCTGCA CCCACCCAAC TAGCCAGGCC AATGAATNTG GCNGCACCCG AAGAGGTTTC   1860
   CAGCGAAGTA CGCGGCTAGT CGCGCTTGGC TGCNTTCTTC GCCGGCACCT TCCGGGCAGC   1920
   TTTCCTGGCT GGCCGTTTTG CCGGACCCCG GGCTCGGCGA TCGGCCAACA GCTCGGCGGC   1980
   GCGCTCGTCG GTTATGGAAG CCACGTNGTC GCCCTTACGC AGGCTGGCAT TGGTCTCACC   2040
   GTCGGTGACG TACGGCCCGA ATCGGCCGTC CTTGATGACC ATTGGCTTGC CAGACGCCGG   2100
   ATNTGNTCCC AGCTCGCGCA GCGGCGGAGC CGAAGCGCTT TGCCGGCCAC GACNTTTCGG   2160
   CTCTGNGTAG ATNTTCAGGG CTTCGTCGAG CGNGATGGTG AATATATGGT CTTCGGTGAC   2220
   CAGTGATCGA GAATCGTTGC CGCGCTTTAG ATACGGTCNG TAGCGCCCGT TCTGCGCGGT   2280
   GATNTC                                                              2286
(2) INFORMATION FOR SEQ ID NO:10:
   (i) SEQUENCE CHARACTERISTICS:
      (A) LENGTH: 1136 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:10:
   GGGCATCTTC CCCGACCGCG CCTCGATCAT CCGCCTCGTC GGAGCCGTCC TCGCCGAACA     60
   ACACGACGAA TGGATCGAAG GACGGCGCTA CCTGGGCCTC GAGGTCCTCA CCCGAGCCCG    120
   AGCAGCACTG ACCAGCACCG AAGAACCGCC AAGCAGCAAA CCACCAACAC CCCAGCACTG    180
   ACCACCTAGA CTGCCACCCG AAGGATCACG CGAGGAACCT TCACTCGTAC ACCACGTCCC    240
   TGGCCTTGGC CTGGTGTCAG GCCCAGCTGG AGCCGACGGC GCTGTCGGTT TGCGCCATGT    300
   TGTTGCCGGC AGCCTGCACC TTCTGCCCGT GGGCGTTGGC CTGCTCGTAG ATCACCTGGA    360
   AGTTACGGCC CAACTGGGTA ATGAACCCCT GGCAGGCCGC CGAACCGGCG CCGCCCCAAA    420
   AGTCACTCGC GGTCAACACA TCACGAATGA TGGCCTGATG CTCGGCCTCC AGCAACCCGG    480
   CCTGAGCGCG GATCATGGCG CCGTGAGCGT CGACATCACC GAACTGATAG TTGATGGTCA    540
   TCGAACCTGT TCTCCTTCGC TTGTAAAAGT ATTGTGCTGC AGCGGCTGAC GTTAGCTGCT    600
   GAGGATCTGC TGGGAGGCCT GCTCTTGCCT CGTGCCGAAT TCGGCACGAG AGGCCGCCTT    660
   CGAAGAAATC CTTTGAGAAT TCGCCAAGGC CGTCGACCCA GCATGGGGTC AGCTCGCCAG    720
   CCGCGCCGGC TGGCAACCGT TCCCGCTCGA GAAAGACCTG GAGGAATACC AGTGACAAAC    780
   GACCTCCCAG ACGTCCGAGA GCGTGACGGC GGTCCACGTC CCGCTCCTCC TGCTGGCGGG    840
   CCACGCTTGT CAGACGTGTG GGTTTACAAC GGGCGGGCGT ACGACCTGAG TGAGTGGATT    900
   TCCAAGCATC CCGGCGGCGC CTTNTTCATT GGGCGGACCA AGAACCGCGA CATCACCGCA    960
   ATCGTCAAGT CCTACCATCG TGATCCGGCG ATTGTCGAGC GAATCCTGCA GCGGAGGTAC   1020
   GCGTTGGGCC GCGACGCAAC CCCTAGGGAC ATCCACCCCA AGCACAATGC ACCGGCATTT   1080
   CTGTTCAAAG ACGACTTCAA CAGCTGGCGG GACACCCCGA AGTATCGATT NGACGA       1136
(2) INFORMATION FOR SEQ ID NO:11:
   (i) SEQUENCE CHARACTERISTICS:
      (A) LENGTH: 967 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:11:
   TGAGCGCCAA CCCTACCGTC GGTTCGTCAC ACGGACCGCA TGGCCTGCTC CGCGGACTGC     60
   CGCTAGGGTC GCGGATCACT CGGCGTAGCG GCGCCTTTGC CCACCGATAT GGGTTCCGTC    120
   ACAGTGTGGT TGCCCGCCCG CCATCGGCCG GATAACGCCA TGACCTCAGC TCGGCAGAAA    180
   TGACAATGCT CCCAAAGGCG TGAGCACCCG AAGACAACTA AGCAGGAGAT CGCATGCCGT    240
   TTGTGACTAC CCAACCAGAA GCACTGGCGG CGGCGGCCGG CAGTCTGCAG GGAATCGGCT    300
   CCGCATTGAA CGCCCAGAAT GCGGCTGCGG CGACTCCCAC GACGGGGGTG GTCCGGCGGC    360
   CGCCGATGAA NTGTCGGCGC TGACGGCGGC TCAGTTCGCG GCACACGCCC AGATCTATCA    420
   GGCCGTCAGC GCCCAGGCCG CGGCGATTCA CGAGATGTTC GTCAACACTC TACAGATGAG    480
   CTCAGGGTCG TATGCTGCTA CCGAGGCCGC CAACGCGGCC GCGGCCGGNT AGAGGAGTCA    540
   CTGCGATGGA TTTTGGGGCG TTGCCGCCGG AGGTCAATTC GGTGCGGATG TATGCCGTTC    600
   CTGGCTCGGC ACCAATGGTC GCTGCGGCGT CGGCCTGGAA CGGGTTGGCC GCGGAGCTGA    660
   GTTCGGCGGC CACCGGTTAT GAGACGGTGA TCACTCAGCT CAGCAGTGAG GGGTGGCTAG    720
   GTCCGGCGTC AGCGGCGATG GCCGAGGCAG TTGCGCCGTA TGTGGCGTGG ATGAGTGCCG    780
   CTGCGGCGCA AGCCGAGCAG GCGGCCACAC AGGCCAGGGC CGCCGCGGCC GCTTTTGAGG    840
   CGGCGTTTGC CGCGACGGTG CCTCCGCCGT TGATCGCGGC CAACCGGGCT TCGTTGATGC    900
   AGCTGATCTC GACGAATGTC TTTGGTCAGA ACACCTCGGC GATCGCGGCC GCCGAAGCTC    960
   AGTACGG                                                              967
(2) INFORMATION FOR SEQ ID NO:12:
   (i) SEQUENCE CHARACTERISTICS:
      (A) LENGTH: 585 base pairs
      (B) TYPE: nucleic acid
      (C) STRANDEDNESS: double
      (D) TOPOLOGY: linear
   (ii) MOLECULE TYPE: DNA (genomic)
   (vi) ORIGINAL SOURCE:
      (A) ORGANISM: Mycobacterium tuberculosis
   (xi) SEQUENCE DESCRIPTION: SEQ ID NO:12:
   TGGATTCCGA TAGCGGTTTC GGCCCCTCGA CGGGCGACCA CGGCGCGCAG GCCTCCGAAC    60
   GGGGGGCCGG GACGCTGGGA TTCGCCGGGA CCGCAACCAA AGAACGCCGG GTCCGGGCGG   120
   TCGGGCTGAC CGCACTGGCC GGTGATGAGT TCGGCAACGG CCCCCGGATG CCGATGGTGC   180
   CGGGGACCTG GGAGCAGGGC AGCAACGAGC CCGAGGCGCC CGACGGATCG GGGAGAGGGG   240
   GAGGCGACGG CTTACCGCAC GACAGCAAGT AACCGAATTC CGAATCACGT GGACCCGTAC   300
   GGGTCGAAAG GAGAGATGTT ATGAGCCTTT TGGATGCTCA TATCCCACAG TTGGTGGCCT   360
   CCCAGTCGGC GTTTGCCGCC AAGGCGGGGC TGATGCGGCA CACGATCGGT CAGGCCGAGC   420
   AGGCGGCGAT GTCGGCTCAG GCGTTTCACC AGGGGGAGTC GTCGGCGGCG TTTCAGGCCG   480
   CCCATGCCCG GTTTGTGGCG GCGGCCGCCA AAGTCAACAC CTTGTTGGAT GTCGCGCAGG   540
   CGAATCTGGG TGAGGCCGCC GGTACCTATG TGGCCGCCGA TGCTG                   585
(2) INFORMATION FOR SEQ ID NO:13:
   (i) SEQUENCE CHARACTERISTICS:
      (A) LENGTH: 144 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:13:
   Ala Leu Val Thr Thr Asn Phe Phe Gly Val Asn Thr Ile Pro Ile Ala
   1               5                   10                  15
   Leu Asn Glu Ala Asp Tyr Leu Arg Met Trp Ile Gln Ala Ala Thr Val
               20                  25                  30
   Met Ser His Tyr Gln Ala Val Ala His Glu Ile Trp Cys Leu His Glu
           35                  40                  45
   Xaa Ala Ser Ser Gly Lys Pro Trp Ala Ser Ile Thr Thr Gly Ala Pro
       50                  55                  60
   Gly Ser Pro Ala Ser Thr Thr Arg Ser Arg Thr Pro Leu Val Ser Thr
   65                  70                  75                  80
   Asn Arg Xaa Val Xaa Ala Pro Ile Val Ser Pro Asn His Thr Gly His
                   85                  90                  95
   Arg Pro Glu Lys Gly Leu Gly Ser Xaa Gln Arg Arg Leu Ser Arg Val
               100                 105                 110
   Leu Pro Arg Ile Ile Asp Arg Pro Ala Gly Pro Xaa Gly Pro Pro Leu
           115                 120                 125
   Thr Ser Gly Ser His Phe Leu Cys Ser Trp His Gly Tyr Ser Ser Gln
       130                 135                 140
(2) INFORMATION FOR SEQ ID NO:14:
   (i) SEQUENCE CHARACTERISTICS:
      (A) LENGTH: 352 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO: 14:
   His Ala Leu Ala Ala Gln Tyr Thr Glu Ile Ala Thr Glu Leu Ala Ser
   1               5                   10                  15
   Val Leu Ala Ala Val Gln Ala Ser Ser Trp Gln Gly Pro Ser Ala Asp
               20                  25                  30
   Arg Phe Val Val Ala His Gln Pro Phe Arg Tyr Trp Leu Thr His Ala
           35                  40                  45
   Ala Thr Val Ala Thr Ala Ala Ala Ala Ala His Xaa Thr Ala Ala Ala
       50                  55                  60
   Gly Tyr Thr Ser Ala Leu Gly Gly Met Pro Thr Leu Ala Glu Leu Ala
   65                  70                  75                  80
   Ala Asn His Ala Met His Gly Ala Leu Val Thr Thr Asn Phe Phe Gly
                   85                  90                  95
   Val Asn Thr Ile Pro Ile Ala Leu Asn Glu Ala Asp Tyr Leu Arg Met
               100                 105                 110
   Trp Ile Gln Ala Ala Thr Val Met Ser His Tyr Gln Ala Val Ala His
           115                 120                 125
   Glu Ser Val Ala Ala Thr Pro Ser Thr Pro Pro Ala Pro Gln Ile Val
       130                 135                 140
   Thr Ser Ala Ala Ser Ser Ala Ala Ser Ser Ser Phe Pro Asp Pro Thr
   145                 150                 155                 160
   Lys Leu Ile Leu Gln Leu Leu Lys Asp Phe Leu Glu Leu Leu Arg Tyr
                   165                 170                 175
   Leu Ala Val Glu Leu Leu Pro Gly Pro Leu Gly Asp Leu Ile Ala Gln
               180                 185                 190
   Val Leu Asp Trp Phe Ile Ser Phe Val Ser Gly Pro Val Phe Thr Phe
           195                 200                 205
   Leu Ala Tyr Leu Val Leu Asp Pro Leu Ile Tyr Phe Gly Pro Phe Ala
       210                 215                 220
   Pro Leu Thr Ser Pro Val Leu Leu Pro Ala Val Glu Leu Arg Asn Arg
   225                 230                 235                 240
   Leu Lys Thr Ala Thr Gly Leu Thr Leu Pro Pro Thr Val Ile Phe Asp
                   245                 250                 255
   His Pro Thr Pro Thr Ala Val Ala Glu Tyr Val Ala Gln Gln Met Ser
               260                 265                 270
   Gly Ser Arg Pro Thr Glu Ser Gly Asp Pro Thr Ser Gln Val Val Glu
           275                 280                 285
   Pro Ala Arg Ala Glu Phe Gly Thr Ser Ala Val His Gln Ile Pro Pro
       290                 295                 300
   Arg Pro Ala Asp Thr Arg Arg Ala Cys Arg His Arg Asp Asp Val Pro
   305                 310                 315                 320
   Arg Asp Ser Arg Ile Ala Gln His Arg Asp Gly Ala Gly Leu Asp Pro
                   325                 330                 335
   Thr Glu Arg Gly Thr Ser Glu Gly Asp Gln Gly Leu Val Ser Gly Trp
               340                 345                 350
(2) INFORMATION FOR SEQ ID NO:15:
   (i) SEQUENCE CHARACTERISTICS:
      (A) LENGTH: 141 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:15:
   Met Asp Phe Gly Ala Leu Pro Pro Glu Val Asn Ser Val Arg Met Tyr
   1               5                   10                  15
   Ala Val Pro Gly Ser Ala Pro Met Val Ala Ala Ala Ser Ala Trp Asn
               20                  25                  30
   Gly Leu Ala Ala Glu Leu Ser Ser Ala Ala Thr Gly Tyr Glu Thr Val
           35                  40                  45
   Ile Thr Gln Leu Ser Ser Glu Gly Trp Leu Gly Pro Ala Ser Ala Ala
       50                  55                  60
   Met Ala Glu Ala Val Ala Pro Tyr Val Ala Trp Met Ser Ala Ala Ala
   65                  70                  75                  80
   Ala Gln Ala Glu Gln Ala Ala Thr Gln Ala Arg Ala Ala Ala Ala Ala
                   85                  90                  95
   Phe Glu Ala Ala Phe Ala Ala Thr Val Pro Pro Pro Leu Ile Ala Ala
               100                 105                 110
   Asn Arg Ala Ser Leu Met Gln Leu Ile Ser Thr Asn Val Phe Gly Gln
           115                 120                 125
   Asn Thr Ser Ala Ile Ala Ala Ala Glu Ala Gln Tyr Gly
       130                 135                 140
(2) INFORMATION FOR SEQ ID NO:16:
   (i) SEQUENCE CHARACTERISTICS:
      (A) LENGTH: 58 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:16:
   Met Ala Ser Arg Phe Met Thr Asp Pro His Ala Met Arg Asp Met Ala
   1               5                   10                  15
   Gly Arg Phe Glu Val His Ala Gln Thr Val Glu Asp Glu Ala Arg Arg
               20                  25                  30
   Met Trp Ala Ser Ala Gln Asn Ile Ser Gly Ala Gly Trp Ser Gly Met
           35                  40                  45
   Ala Glu Ala Thr Ser Leu Asp Thr Met Thr
       50                  55
(2) INFORMATION FOR SEQ ID NO:17:
   (i) SEQUENCE CHARACTERISTICS:
      (A) LENGTH: 67 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:17:
   Met Thr Ile Asn Tyr Gln Phe Gly Asp Val Asp Ala His Gly Ala Met
   1               5                   10                  15
   Ile Arg Ala Gln Ala Ala Ser Leu Glu Ala Glu His Gln Ala Ile Val
               20                  25                  30
   Arg Asp Val Leu Ala Ala Gly Asp Phe Trp Gly Gly Ala Gly Ser Val
           35                  40                  45
   Ala Cys Gln Glu Phe Ile Thr Gln Leu Gly Arg Asn Phe Gln Val Ile
       50                  55                  60
   Tyr Glu Gln
   65
(2) INFORMATION FOR SEQ ID NO:18:
   (i) SEQUENCE CHARACTERISTICS:
      (A) LENGTH: 58 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:18:
   Met Ala Ser Arg Phe Met Thr Asp Pro His Ala Met Arg Asp Met Ala
   1               5                   10                  15
   Gly Arg Phe Glu Val His Ala Gln Thr Val Glu Asp Glu Ala Arg Arg
               20                  25                  30
   Met Trp Ala Ser Ala Gln Asn Ile Ser Gly Ala Gly Trp Ser Gly Met
           35                  40                  45
   Ala Glu Ala Thr Ser Leu Asp Thr Met Thr
       50                  55
(2) INFORMATION FOR SEQ ID NO:19:
   (i) SEQUENCE CHARACTERISTICS:
      (A) LENGTH: 94 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:19:
   Met Thr Ile Asn Tyr Gln Phe Gly Asp Val Asp Ala His Gly Ala Met
   1               5                   10                  15
   Ile Arg Ala Gln Ala Ala Ser Leu Glu Ala Glu His Gln Ala Ile Val
               20                  25                  30
   Arg Asp Val Leu Ala Ala Gly Asp Phe Trp Gly Gly Ala Gly Ser Val
           35                  40                  45
   Ala Cys Gln Glu Phe Ile Thr Gln Leu Gly Arg Asn Phe Gln Val Ile
       50                  55                  60
   Tyr Glu Gln Ala Asn Ala His Gly Gln Lys Val Gln Ala Ala Gly Asn
   65                  70                  75                  80
   Asn Met Ala Gln Thr Asp Ser Ala Val Gly Ser Ser Trp Ala
                   85                  90
(2) INFORMATION FOR SEQ ID NO:20:
   (i) SEQUENCE CHARACTERISTICS:
      (A) LENGTH: 30 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:20:
   Asn Met Leu His Gly Val Arg Asp Gly Leu Val Arg Asp Ala Asn Asn
   1               5                   10                  15
   Tyr Glu Gln Gln Glu Gln Ala Ser Gln Gln Ile Leu Ser Ser
               20                  25                  30
(2) INFORMATION FOR SEQ ID NO:21:
   (i) SEQUENCE CHARACTERISTICS:
      (A) LENGTH: 94 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:21:
   Met Thr Ile Asn Tyr Gln Phe Gly Asp Val Asp Ala His Gly Ala Met
   1               5                   10                  15
   Ile Arg Ala Gln Ala Gly Leu Leu Glu Ala Glu His Gln Ala Ile Ile
               20                  25                  30
   Arg Asp Val Leu Thr Ala Ser Asp Phe Trp Gly Gly Ala Gly Ser Ala
           35                  40                  45
   Ala Cys Gln Gly Phe Ile Thr Gln Leu Gly Arg Asn Phe Gln Val Ile
       50                  55                  60
   Tyr Glu Gln Ala Asn Ala His Gly Gln Lys Val Gln Ala Ala Gly Asn
   65                  70                  75                  80
   Asn Met Ala Gln Thr Asp Ser Ala Val Gly Ser Ser Trp Ala
                   85                  90
(2) INFORMATION FOR SEQ ID NO:22:
   (i) SEQUENCE CHARACTERISTICS:
      (A) LENGTH: 69 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:22:
   Ala Arg Arg Met Trp Ala Ser Ala Gln Asn Ile Ser Gly Ala Gly Trp
   1               5                   10                  15
   Ser Gly Met Ala Glu Ala Thr Ser Leu Asp Thr Met Ala Gln Met Asn
               20                  25                  30
   Gln Ala Phe Arg Asn Ile Val Asn Met Leu His Gly Val Arg Asp Gly
           35                  40                  45
   Leu Val Arg Asp Ala Asn Asn Tyr Glu Gln Gln Glu Gln Ala Ser Gln
       50                  55                  60
   Gln Ile Leu Ser Ser
   65
(2) INFORMATION FOR SEQ ID NO:23:
   (i) SEQUENCE CHARACTERISTICS:
      (A) LENGTH: 94 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:23:
   Met Thr Ile Asn Tyr Gln Phe Gly Asp Val Asp Ala His Gly Ala Met
   1               5                   10                  15
   Ile Arg Ala Gln Ala Gly Leu Leu Glu Ala Glu His Gln Ala Ile Ile
               20                  25                  30
   Arg Asp Val Leu Thr Ala Ser Asp Phe Trp Gly Gly Ala Gly Ser Ala
           35                  40                  45
   Ala Cys Gln Gly Phe Ile Thr Gln Leu Gly Arg Asn Phe Gln Val Ile
       50                  55                  60
   Tyr Glu Gln Ala Asn Thr His Gly Gln Lys Val Gln Ala Ala Gly Asn
   65                  70                  75                  80
   Asn Met Ala Gln Thr Asp Ser Ala Val Xaa Ser Ser Trp Ala
                   85                  90
(2) INFORMATION FOR SEQ ID NO:24:
   (i) SEQUENCE CHARACTERISTICS:
      (A) LENGTH: 52 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:24:
   Gly Met Ala Glu Ala Thr Ser Xaa Asp Thr Met Thr Gln Met Asn Gln
   1               5                   10                  15
   Ala Phe Arg Asn Ile Val Asn Met Leu His Gly Val Arg Asp Gly Leu
               20                  25                  30
   Val Arg Asp Ala Asn Xaa Tyr Glu Gln Gln Glu Gln Ala Ser Gln Gln
           35                  40                  45
   Ile Leu Ser Ser
       50
(2) INFORMATION FOR SEQ ID NO:25:
   (i) SEQUENCE CHARACTERISTICS:
      (A) LENGTH: 94 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:25:
   Met Thr Ile Asn Tyr Gln Phe Gly Asp Val Asp Ala His Gly Ala Met
   1               5                   10                  15
   Ile Arg Ala Gln Ala Gly Ser Leu Glu Ala Glu His Gln Ala Ile Ile
               20                  25                  30
   Ser Asp Val Leu Thr Ala Ser Asp Phe Trp Gly Gly Ala Gly Ser Ala
           35                  40                  45
   Ala Cys Gln Gly Phe Ile Thr Gln Leu Gly Arg Asn Phe Gln Val Xaa
       50                  55                  60
   Tyr Glu Gln Ala Asn Ala His Gly Gln Lys Val Gln Ala Ala Gly Asn
   65                  70                  75                  80
   Asn Met Ala Gln Thr Asp Ser Ala Val Gly Ser Ser Trp Ala
                   85                  90
(2) INFORMATION FOR SEQ ID NO:26:
   (i) SEQUENCE CHARACTERISTICS:
      (A) LENGTH: 98 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:26:
   Met Thr Ser Arg Phe Met Thr Asp Pro His Ala Met Arg Asp Met Ala
   1               5                   10                  15
   Gly Arg Phe Glu Val His Ala Gln Thr Val Glu Asp Glu Ala Arg Arg
               20                  25                  30
   Met Trp Ala Ser Ala Gln Asn Ile Ser Gly Ala Gly Trp Ser Gly Met
           35                  40                  45
   Ala Glu Ala Thr Ser Leu Asp Thr Met Ala Gln Met Asn Gln Ala Phe
       50                  55                  60
   Arg Asn Ile Val Asn Met Leu His Gly Val Arg Asp Gly Leu Val Arg
   65                  70                  75                  80
   Asp Ala Asn Asn Tyr Glu Gln Gln Glu Gln Ala Ser Gln Gln Ile Leu
                   85                  90                  95
   Ser Ser
(2) INFORMATION FOR SEQ ID NO:27:
   (i) SEQUENCE CHARACTERISTICS:
      (A) LENGTH: 94 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:27:
   Met Thr Ile Asn Tyr Gln Phe Gly Asp Val Asp Ala His Gly Ala Met
   1               5                   10                  15
   Ile Arg Ala Xaa Ala Gly Leu Leu Glu Ala Glu His Gln Ala Ile Ile
               20                  25                  30
   Ser Asp Val Leu Thr Ala Ser Asp Phe Trp Gly Gly Ala Gly Ser Ala
           35                  40                  45
   Ala Cys Gln Gly Phe Ile Thr Gln Leu Gly Arg Asn Phe Gln Val Ile
       50                  55                  60
   Tyr Glu Gln Ala Asn Ala His Gly Gln Lys Val Gln Ala Ala Gly Asn
   65                  70                  75                  80
   Asn Met Ala Gln Thr Asp Ser Ala Val Gly Ser Ser Trp Ala
                   85                  90
(2) INFORMATION FOR SEQ ID NO:28:
   (i) SEQUENCE CHARACTERISTICS:
      (A) LENGTH: 81 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:28:
   Arg Phe Glu Val His Ala Gln Thr Val Glu Asp Glu Ala Arg Arg Met
   1               5                   10                  15
   Trp Ala Ser Ala Gln Asn Ile Ser Gly Ala Gly Trp Ser Gly Met Ala
               20                  25                  30
   Xaa Ala Thr Ser Leu Asp Thr Met Ala Gln Met Asn Gln Ala Phe Arg
           35                  40                  45
   Asn Ile Val Asn Met Leu His Gly Val Arg Asp Gly Leu Val Arg Asp
       50                  55                  60
   Ala Asn Asn Tyr Glu Gln Gln Glu Gln Ala Ser Gln Gln Ile Leu Ser
   65                  70                  75                  80
   Ser
(2) INFORMATION FOR SEQ ID NO:29:
   (i) SEQUENCE CHARACTERISTICS:
      (A) LENGTH: 94 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:29:
   Met Thr Ile Asn Tyr Gln Phe Gly Asp Val Asp Ala His Gly Ala Met
   1               5                   10                  15
   Ile Arg Ala Leu Ala Gly Leu Leu Glu Ala Glu His Gln Ala Ile Ile
               20                  25                  30
   Ser Asp Val Leu Thr Ala Ser Asp Phe Trp Gly Gly Ala Gly Ser Ala
           35                  40                  45
   Ala Cys Gln Gly Phe Ile Thr Gln Leu Gly Arg Asn Phe Gln Val Ile
       50                  55                  60
   Tyr Glu Gln Ala Asn Ala His Gly Gln Lys Val Gln Ala Ala Gly Asn
   65                  70                  75                  80
   Asn Met Ala Gln Thr Asp Ser Ala Val Gly Ser Ser Trp Ala
                   85                  90
(2) INFORMATION FOR SEQ ID NO:30:
   (i) SEQUENCE CHARACTERISTICS:
      (A) LENGTH: 11 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:30:
   Gln Glu Gln Ala Ser Gln Gln Ile Leu Ser Ser
   1               5                   10
(2) INFORMATION FOR SEQ ID NO:31:
   (i) SEQUENCE CHARACTERISTICS:
      (A) LENGTH: 94 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:31:
   Met Thr Ile Asn Tyr Gln Phe Gly Asp Val Asp Ala His Gly Ala Met
   1               5                   10                  15
   Ile Arg Ala Gln Ala Gly Leu Leu Glu Ala Glu His Gln Ala Ile Ile
               20                  25                  30
   Arg Asp Val Leu Thr Ala Ser Asp Phe Trp Gly Gly Ala Gly Ser Ala
           35                  40                  45
   Ala Cys Gln Gly Phe Ile Thr Gln Leu Gly Arg Asn Phe Gln Val Ile
       50                  55                  60
   Tyr Glu Gln Ala Asn Ala His Gly Gln Lys Val Gln Ala Ala Gly Asn
   65                  70                  75                  80
   Asn Met Ala Gln Thr Asp Ser Ala Val Gly Ser Ser Trp Ala
                   85                  90
(2) INFORMATION FOR SEQ ID NO: 32:
   (i) SEQUENCE CHARACTERISTICS:
      (A) LENGTH: 99 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:32:
   Met Ser Phe Val Thr Thr Gln Pro Glu Ala Leu Ala Ala Ala Ala Ala
   1               5                   10                  15
   Asn Leu Gln Gly Ile Gly Thr Thr Met Asn Ala Gln Asn Ala Ala Ala
               20                       25                       30
   Ala Ala Pro Thr Thr Gly Val Val Pro Ala Ala Ala Asp Glu Val Ser
           35                      40                      45
   Ala Leu Thr Ala Ala Gln Phe Ala Ala His Ala Gln Met Tyr Gln Thr
       50                      55                      60
   Val Ser Ala Gln Ala Ala Ala Ile His Glu Met Phe Val Asn Thr Leu
   65                      70                      75                      80
   Val Ala Ser Ser Gly Ser Tyr Ala Ala Thr Glu Ala Ala Asn Ala Ala
                   85                      90                      95
   Ala Ala Gly
(2) INFORMATION FOR SEQ ID NO:33:
   (i) SEQUENCE CHARACTERISTICS:
      (A) LENGTH: 99 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:33:
   Met Ser Phe Val Thr Thr Gln Pro Glu Ala Leu Ala Ala Ala Ala Ala
   1               5                   10                  15
   Asn Leu Gln Gly Ile Gly Thr Thr Met Asn Ala Gln Asn Ala Ala Ala
               20                  25                  30
   Ala Ala Pro Thr Thr Gly Val Val Pro Ala Ala Ala Asp Glu Val Ser
           35                  40                  45
   Ala Leu Thr Ala Ala Gln Phe Ala Ala His Ala Gln Met Tyr Gln Thr
       50                  55                  60
   Val Ser Ala Gln Ala Ala Ala Ile His Glu Met Phe Val Asn Thr Leu
   65                  70                  75                  80
   Val Ala Ser Ser Gly Ser Tyr Ala Ala Thr Glu Ala Ala Asn Ala Ala
                   85                  90                  95
   Ala Ala Gly
(2) INFORMATION FOR SEQ ID NO:34:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:34:
   Asp Pro His Ala Met Arg Asp Met Ala Gly Arg Phe Glu Val His
   1 5 10 15
(2) INFORMATION FOR SEQ ID NO:35:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:35:
   Arg Asp Met Ala Gly Arg Phe Glu Val His Ala Gln Thr Val Glu
   1 5 10 15
(2) INFORMATION FOR SEQ ID NO:36:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:36:
   Arg Phe Glu Val His Ala Gln Thr Val Glu Asp Glu Ala Arg Arg
   1 5 10 15
(2) INFORMATION FOR SEQ ID NO:37:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:37:
   Ala Gln Thr Val Glu Asp Glu Ala Arg Arg Met Trp Ala Ser Ala
       1                 5                     10                    15
(2) INFORMATION FOR SEQ ID NO:38:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:38:
   Asp Glu Ala Arg Arg Met Trp Ala Ser Ala Gln Asn Ile Ser Gly
   1               5                   10                  15
(2) INFORMATION FOR SEQ ID NO:39:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:39:
   Met Trp Ala Ser Ala Gln Asn Ile Ser Gly Ala Gly Trp Ser Gly
   1               5                   10                  15
(2) INFORMATION FOR SEQ ID NO:40:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:40:
   Gln Asn Ile Ser Gly Ala Gly Trp Ser Gly Met Ala Glu Ala Thr
   1               5                   10                  15
(2) INFORMATION FOR SEQ ID NO:41:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:41:
   Ala Gly Trp Ser Gly Met Ala Glu Ala Thr Ser Leu Asp Thr Met Thr
   1               5                   10                  15
(2) INFORMATION FOR SEQ ID NO:42:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:42:
   Met Ala Glu Ala Thr Ser Leu Asp Thr Met Ala Gln Met Asn Gln
   1 5 10 15
(2) INFORMATION FOR SEQ ID NO:43:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:43:
   Ser Leu Asp Thr Met Ala Gln Met Asn Gln Ala Phe Arg Asn Ile
   1 5 10 15
(2) INFORMATION FOR SEQ ID NO:44:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:44:
   Ala Gln Met Asn Gln Ala Phe Arg Asn Ile Val Asn Met Leu His
   1 5 10 15
(2) INFORMATION FOR SEQ ID NO:45:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:45:
   Ala Phe Arg Asn Ile Val Asn Met Leu His Gly Val Arg Asp Gly
   1               5                   10                  15
(2) INFORMATION FOR SEQ ID NO:46:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:46:
   Val Asn Met Leu His Gly Val Arg Asp Gly Leu Val Arg Asp Ala
   1               5                   10                  15
(2) INFORMATION FOR SEQ ID NO:47:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:47:
   Gly Val Arg Asp Gly Leu Val Arg Asp Ala Asn Asn Tyr Glu Gln
   1               5                   10                  15
(2) INFORMATION FOR SEQ ID NO:48:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:48:
   Leu Val Arg Asp Ala Asn Asn Tyr Glu Gln Gln Glu Gln Ala Ser
   1               5                   10                  15
(2) INFORMATION FOR SEQ ID NO:49:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:49:
   Asn Asn Tyr Glu Gln Gln Glu Gln Ala Ser Gln Gln Ile Leu Ser Ser
   1               5                   10                  15
(2) INFORMATION FOR SEQ ID NO:50:
   (i) SEQUENCE CHARACTERISTICS:
      (A) LENGTH: 17 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:50:
   Met Ala Ser Arg Phe Met Thr Asp Pro His Ala Met Arg Asp Met Ala
   1               5                   10                  15
   Gly
(2) INFORMATION FOR SEQ ID NO: 51:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:51:
   Met Thr Ile Asn Tyr Gln Phe Gly Asp Val Asp Ala His Gly Ala
   1               5                   10                  15
(2) INFORMATION FOR SEQ ID NO:52:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:52:
   Gln Phe Gly Asp Val Asp Ala His Gly Ala Met Ile Arg Ala Gln
   1               5                   10                  15
(2) INFORMATION FOR SEQ ID NO:53:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:53:
   Asp Ala His Gly Ala Met Ile Arg Ala Gln Ala Ala Ser Leu Glu
   1               5                   10                  15
(2) INFORMATION FOR SEQ ID NO:54:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:54:
   Met Ile Arg Ala Gln Ala Ala Ser Leu Glu Ala Glu His Gln Ala
   1               5                   10                  15
(2) INFORMATION FOR SEQ ID NO:55:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:55:
   Ala Ala Ser Leu Glu Ala Glu His Gln Ala Ile Val Arg Asp Val
   1               5                   10                  15
(2) INFORMATION FOR SEQ ID NO:56:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:56:
   Ala Glu His Gln Ala Ile Val Arg Asp Val Leu Ala Ala Gly Asp
   1               5                   10                  15
(2) INFORMATION FOR SEQ ID NO:57:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:57:
   Ile Val Arg Asp Val Leu Ala Ala Gly Asp Phe Trp Gly Gly Ala
   1               5                   10                  15
(2) INFORMATION FOR SEQ ID NO:58:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:58:
   Leu Ala Ala Gly Asp Phe Trp Gly Gly Ala Gly Ser Val Ala Cys Gln
   1               5                   10                  15
(2) INFORMATION FOR SEQ ID NO:59:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:59:
   Phe Trp Gly Gly Ala Gly Ser Val Ala Cys Gln Glu Phe Ile Thr
   1               5                   10                  15
(2) INFORMATION FOR SEQ ID NO:60:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:60:
   Gly Ser Val Ala Cys Gln Glu Phe Ile Thr Gln Leu Gly Arg Asn
   1               5                   10                  15
(2) INFORMATION FOR SEQ ID NO:61:
   (i) SEQUENCE CHARACTERISTICS:
      (A) LENGTH: 18 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:61:
   Gln Glu Phe Ile Thr Gln Leu Gly Arg Asn Phe Gln Val Ile Tyr Glu
   1               5                   10                  15
   Gln Ala
(2) INFORMATION FOR SEQ ID NO:62:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:62:
   Arg Asn Phe Gln Val Ile Tyr Glu Gln Ala Asn Ala His Gly Gln
   1               5                   10                  15
(2) INFORMATION FOR SEQ ID NO:63:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:63:
   Ile Tyr Glu Gln Ala Asn Ala His Gly Gln Lys Val Gln Ala Ala
   1               5                   10                  15
(2) INFORMATION FOR SEQ ID NO:64:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:64:
   Asn Ala His Gly Gln Lys Val Gln Ala Ala Gly Asn Asn Met Ala
   1 5 10 15
(2) INFORMATION FOR SEQ ID NO:65:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:65:
   Lys Val Gln Ala Ala Gly Asn Asn Met Ala Gln Thr Asp Ser Ala
   1 5 10 15
(2) INFORMATION FOR SEQ ID NO:66:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:66:
   Gly Asn Asn Met Ala Gln Thr Asp Ser Ala Val Gly Ser Ser Trp Ala
   1 5 10 15
(2) INFORMATION FOR SEQ ID NO:67:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:67:
   Asp Ala His Gly Ala Met Ile Arg Ala Leu Ala Gly Leu Leu Glu
   1               5                   10                  15
(2) INFORMATION FOR SEQ ID NO:68:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:68:
   Asp Ala His Gly Ala Met Ile Arg Ala Gln Ala Gly Leu Leu Glu
   1               5                   10                  15
(2) INFORMATION FOR SEQ ID NO:69:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:69:
   Met Ile Arg Ala Leu Ala Gly Leu Leu Glu Ala Glu His Gln Ala
   1               5                   10                  15
(2) INFORMATION FOR SEQ ID NO:70:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:70:
   Met Ile Arg Ala Gln Ala Gly Leu Leu Glu Ala Glu His Gln Ala
   1               5                   10                  15
(2) INFORMATION FOR SEQ ID NO:71:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:71:
   Ala Gly Leu Leu Glu Ala Glu His Gln Ala Ile Ile Ser Asp Val
   1               5                   10                  15
(2) INFORMATION FOR SEQ ID NO:72:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:72:
   Ala Gly Leu Leu Glu Ala Glu His Gln Ala Ile Ile Arg Asp Val
   1               5                   10                  15
(2) INFORMATION FOR SEQ ID NO:73:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:73:
   Ala Glu His Gln Ala Ile Ile Ser Asp Val Leu Thr Ala Ser Asp
   1               5                   10                  15
(2) INFORMATION FOR SEQ ID NO:74:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:74:
   Ala Glu His Gln Ala Ile Ile Arg Asp Val Leu Thr Ala Ser Asp
   1               5                   10                  15
(2) INFORMATION FOR SEQ ID NO:75:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:75:
   Ile Ile Ser Asp Val Leu Thr Ala Ser Asp Phe Trp Gly Gly Ala
   1 5 10 15
(2) INFORMATION FOR SEQ ID NO:76:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:76:
   Ile Ile Arg Asp Val Leu Thr Ala Ser Asp Phe Trp Gly Gly Ala
   1 5 10 15
(2) INFORMATION FOR SEQ ID NO:77:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:77:
   Leu Thr Ala Ser Asp Phe Trp Gly Gly Ala Gly Ser Ala Ala Cys Gln
   1 5 10 15
(2) INFORMATION FOR SEQ ID NO:78:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:78:
   Phe Trp Gly Gly Ala Gly Ser Ala Ala Cys Gln Gly Phe Ile Thr
   1               5                   10                  15
(2) INFORMATION FOR SEQ ID NO:79:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:79:
   Gly Ser Ala Ala Cys Gln Gly Phe Ile Thr Gln Leu Gly Arg Asn
   1               5                   10                  15
(2) INFORMATION FOR SEQ ID NO:80:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:80:
   Gln Gly Phe Ile Thr Gln Leu Gly Arg Asn Phe Gln Val Ile Tyr
   1               5                   10                  15
(2) INFORMATION FOR SEQ ID NO:81:
   (i) SEQUENCE CHARACTERISTICS:
      (A) LENGTH: 25 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:81:
   Val Thr Thr Asn Phe Phe Gly Val Asn Thr Ile Pro Ile Ala Leu Asn
   1               5                   10                  15
   Glu Ala Asp Tyr Leu Arg Met Trp Ile
               20                  25
(2) INFORMATION FOR SEQ ID NO:82:
   (i) SEQUENCE CHARACTERISTICS:
      (A) LENGTH: 25 amino acids
      (B) TYPE: amino acid
      (C) STRANDEDNESS: single
      (D) TOPOLOGY: linear
   (ii) MOLECULE TYPE: peptide
   (vi) ORIGINAL SOURCE:
      (A) ORGANISM: Mycobacterium tuberculosis
   (xi) SEQUENCE DESCRIPTION: SEQ ID NO:82:
   Asn Glu Ala Asp Tyr Leu Arg Met Trp Ile Gln Ala Ala Thr Val Met
   1               5                   10                  15
   Ser His Tyr Gln Ala Val Ala His Glu
               20                  25
(2) INFORMATION FOR SEQ ID NO:83:
   (i) SEQUENCE CHARACTERISTICS:
      (A) LENGTH: 967 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:83:
   TGAGCGCCAA CCCTACCGTC GGTTCGTCAC ACGGACCGCA TGGCCTGCTC CGCGGACTGC      60
   CGCTAGGGTC GCGGATCACT CGGCGTAGCG GCGCCTTTGC CCACCGATAT GGGTTCCGTC     120
   ACAGTGTGGT TGCCCGCCCG CCATCGGCCG GATAACGCCA TGACCTCAGC TCGGCAGAAA     180
   TGACAATGCT CCCAAAGGCG TGAGCACCCG AAGACAACTA AGCAGGAGAT CGCATGCCGT     240
   TTGTGACTAC CCAACCAGAA GCACTGGCGG CGGCGGCCGG CAGTCTGCAG GGAATCGGCT     300
   CCGCATTGAA CGCCCAGAAT GCGGCTGCGG CGACTCCCAC GACGGGGGTG GTCCGGCGGC     360
   CGCCGATGAA NTGTCGGCGC TGACGGCGGC TCAGTTCGCG GCACACGCCC AGATCTATCA     420
   GGCCGTCAGC GCCCAGGCCG CGGCGATTCA CGAGATGTTC GTCAACACTC TACAGATGAG     480
   CTCAGGGTCG TATGCTGCTA CCGAGGCCGC CAACGCGGCC GCGGCCGGNT AGAGGAGTCA     540
   CTGCGATGGA TTTTGGGGCG TTGCCGCCGG AGGTCAATTC GGTGCGGATG TATGCCGTTC     600
   CTGGCTCGGC ACCAATGGTC GCTGCGGCGT CGGCCTGGAA CGGGTTGGCC GCGGAGCTGA     660
   GTTCGGCGGC CACCGGTTAT GAGACGGTGA TCACTCAGCT CAGCAGTGAG GGGTGGCTAG     720
   GTCCGGCGTC AGCGGCGATG GCCGAGGCAG TTGCGCCGTA TGTGGCGTGG ATGAGTGCCG     780
   CTGCGGCGCA AGCCGAGCAG GCGGCCACAC AGGCCAGGGC CGCCGCGGCC GCTTTTGAGG     840
   CGGCGTTTGC CGCGACGGTG CCTCCGCCGT TGATCGCGGC CAACCGGGCT TCGTTGATGC     900
   AGCTGATCTC GACGAATGTC TTTGGTCAGA ACACCTCGGC GATCGCGGCC GCCGAAGCTC     960
   AGTACGG                                                               967
(2) INFORMATION FOR SEQ ID NO:84:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:84:
   Met Ser Phe Val Thr Thr Gln Pro Glu Ala Leu Ala Ala Ala Ala
    1               5                  10                  15
(2) INFORMATION FOR SEQ ID NO:85:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:85:
   Thr Gln Pro Glu Ala Leu Ala Ala Ala Ala Ala Asn Leu Gln Gly
    1               5                  10                  15
(2) INFORMATION FOR SEQ ID NO:86:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:86:
   Leu Ala Ala Ala Ala Ala Asn Leu Gln Gly Ile Gly Thr Thr Met
    1               5                  10                  15
(2) INFORMATION FOR SEQ ID NO:87:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:87:
   Ala Asn Leu Gln Gly Ile Gly Thr Thr Met Asn Ala Gln Asn Ala
    1               5                  10                  15
(2) INFORMATION FOR SEQ ID NO:88:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:88:
   Ile Gly Thr Thr Met Asn Ala Gln Asn Ala Ala Ala Ala Ala Pro
    1               5                  10                  15
(2) INFORMATION FOR SEQ ID NO:89:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:89:
   Asn Ala Gln Asn Ala Ala Ala Ala Ala Pro Thr Thr Gly Val Val
   1               5                   10                  15
(2) INFORMATION FOR SEQ ID NO:90:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:90:
   Ala Ala Ala Ala Pro Thr Thr Gly Val Val Pro Ala Ala Ala Asp
   1               5                   10                  15
(2) INFORMATION FOR SEQ ID NO:91:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:91:
   Thr Thr Gly Val Val Pro Ala Ala Ala Asp Glu Val Ser Ala Leu
   1               5                   10                  15
(2) INFORMATION FOR SEQ ID NO:92:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:92:
   Pro Ala Ala Ala Asp Glu Val Ser Ala Leu Thr Ala Ala Gln Phe
   1               5                   10                  15
(2) INFORMATION FOR SEQ ID NO:93:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:93:
   Glu Val Ser Ala Leu Thr Ala Ala Gln Phe Ala Ala His Ala Gln
    1               5                  10                  15
(2) INFORMATION FOR SEQ ID NO:94:
   (i) SEQUENCE CHARACTERISTICS:
      (A) LENGTH: 15 amino acids
      (B) TYPE: amino acid
      (C) STRANDEDNESS: single
      (D) TOPOLOGY: linear.
   (ii) MOLECULE TYPE: peptide
   (xi) SEQUENCE DESCRIPTION: SEQ ID NO:94:
   Thr Ala Ala Gln Phe Ala Ala His Ala Gln Met Tyr Gln Thr Val
    1               5                  10                  15
(2) INFORMATION FOR SEQ ID NO:95:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:95:
   Ala Ala His Ala Gln Met Tyr Gln Thr Val Ser Ala Gln Ala Ala
    1               5                  10                  15
(2) INFORMATION FOR SEQ ID NO:96:
   (i) SEQUENCE CHARACTERISTICS:
      (A) LENGTH: 16 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:96:
   Met Tyr Gln Thr Val Ser Ala Gln Ala Ala Ala Ile His Glu Met Phe
    1               5                   10                  15
(2) INFORMATION FOR SEQ ID NO:97:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:97:
   Ser Ala Gln Ala Ala Ala Ile His Glu Met Phe Val Asn Thr Leu
    1               5                   10                  15
(2) INFORMATION FOR SEQ ID NO:98:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:98:
   Ala Ile His Glu Met Phe Val Asn Thr Leu Val Ala Ser Ser Gly
    1               5                   10                  15
(2) INFORMATION FOR SEQ ID NO:99:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:99:
   Phe Val Asn Thr Leu Val Ala Ser Ser Gly Ser Tyr Ala Ala Thr
    1               5                   10                  15
(2) INFORMATION FOR SEQ ID NO:100:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:100:
   Val Ala Ser Ser Gly Ser Tyr Ala Ala Thr Glu Ala Ala Asn Ala
    1               5                  10                  15
(2) INFORMATION FOR SEQ ID NO:101:
   (i) SEQUENCE CHARACTERISTICS:
      (A) LENGTH: 14 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:101:
   Ser Tyr Ala Ala Thr Glu Ala Ala Asn Ala Ala Ala Ala Gly
    1               5                  10
(2) INFORMATION FOR SEQ ID NO:102:
   (i) SEQUENCE CHARACTERISTICS:
      (A) LENGTH: 1784 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:102:
   ATTCGTTCCT GCCGCAGCTA AATCCCGGGG ACATCGTCGC CGGCCAGTAC GAGGTCAAAG     60
   GCTGCATCGC GCACGGCGGA CTGGGCTGGA TCTACCTCGC TCTCGACCGC AATGTCAACG    120
   GCCGTCCGGT GGTGCTCAAG GGCCTGGTGC ATTCCGGTGA TGCCGAAGCG CAGGCAATGG    180
   CGATGGCCGA ACGCCAGTTC CTGGCCGAGG TGGTGCACCC GTCGATCGTG CAGATCTTCA    240
   ACTTTGTCGA GCACACCGAC AGGCACGGGG ATCCGGTCGG CTACATCGTG ATGGAATACG    300
   TCGGCGGGCA ATCGCTCAAA CGCAGCAAGG GTCANAAACT GCCCGTCGCG GAGGCCATCG    360
   CCTACCTGCT GGAGATCCTG CCGGCGCTGA GCTACCTGCA TTCCATCGGC TTGGTCTACA    420
   ACGACCTGAA GCCGGAAAAC ATCATGCTGA CCGAGGAACA GCTCAAGCTG ATCGACCTGG    480
   GCGCGGTATC GCGGATCAAC TCGTTCGGCT ACCTCTACGG GACCCCAGGC TTCCAGGCGC    540
   CCGAGATCGT GCGGACCGGT CCGACGGTGG CCACCGACAT CTACACCGTG GGACGCACGC    600
   TCGCGGCGCT CACGCTGGAC CTGCCCACCC GCAATGGCCG TTATGTGGAT GGGCTACCCG    660
   AAGACGACCC GGTGCTGAAA ACCTACGACT CTTACGGCCG GTTGCTGCGC AGGGCCATCG    720
   ACCCCGATCC GCGGCAACGG TTCACCACCG CCGAAGAGAT GTCCGCGCAA TTGACGGGCG    780
   TGTTGCGGGA GGTGGTCGCC CAGACACCGG GGTGCCGCGG CCAGGCTATC AACGATCTTC    840
   AGTCCCAGTC GGTCGACATT TGGAGTGGAC TGCTGGTGGC GCACACCGAC GTGTATCTGG    900
   ACGGGCAGGT GCACGCGGAG AAGCTGACCG CCAACGAGAT CGTGACCGCG CTGTCGGTGC    960
   CGCTGGTCGA TCCGACCGAC GTCGCAGCTT CGGTCCTGCA GGCCACGGTG CTCTCCCAGC   1020
   CGGTGCAGAC CCTAGACTCG NTGCGCGCGG CCCGCCACGG TGCGCTGGAC GCCGACGGCG   1080
   TCGATTNTCC GAGTCAGTGG AGCTGCCGCT AATGGAAGTC CGCGCGCTGC TGGATCTCGG   1140
   CGATGTGGCC AAGGCCACCC GAAAACTCGA CGATCTGGCC GAACGCGTTG GCTGGCGATG   1200
   GCGATTGGTC TGGTACCGGG CCGTCGCCGA GCTGCTCACC GGCGACTATG ACTCGGCCAC   1260
   CAAACATTTC ACCGAGGTGC TGGATACCTT TCCCGGCGAG CTGGCGCCCA AGCTCGCCCT   1320
   GGCCGCCACC GCCGAACTAG CCGGCAACAC CGACGAACAC AAGTTCTATC AGACGGTGTG   1380
   GAGCACCAAC GACGGCGTGA TCTCGGCGGC TTTCGGACTG GCCAGAGCCC GGTCGGCCGA   1440
   AGGTGATCGG GTCGGCGCCG TGCGCACGCT CGACGAGGTA CCGCCCACTT CTCGGCATTT   1500
   CACCACGGCA CGGCTGACCA GCGCGGTGAC TCTGTTGTCC GGCCGGTCAA CGAGTGAAGT   1560
   CACCGAGGAA CAGATCCGCG ACGCCGCCCG AAGAGTGGAG GCGCTGCCCC CGACCGAACC   1620
   ACGCGTGCTG CAGATCCGCG CCCTGGTGCT GGGTGGCGCG CTGGACTGGC TGAAGGACAA   1680
   CAAGGCCAGC ACCAACCACA TCCTCGGTTT CCCGTTCACC AGTCACGGGC TGCGGCTGGG   1740
   TGTCGAGGCG TCACTGCGCA GCCTGGCCCG GGTAGCTCCC ACTC                    1784
(2) INFORMATION FOR SEQ ID NO:103:
   (i) SEQUENCE CHARACTERISTICS:
      (A) LENGTH: 766 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:103:
   ACAARACACT CGGYGGCKGC CGMTCCGGCC TGATCGTCGG TGATCAGCYT CGTGCCAAAY     60
   TCGGCACAAG GTGCGCGCTR CCCAANGAGT TCTTCGCCGC RGTGCGMGCM KAACTGGCCT    120
   ATCNTGGTTG GGTGCCGTCC CGCANAACCC GCGAACTTAA ACCCATTTTA ACCGGGCAGG    180
   AAGTTTCCTA CATYTACCCN RGSMANCCAA CCGGGCCGCC NANAAMTCCG TCCTGGANTC    240
   CGANCGGTTC CCGGTGTTCG CCGCACTGCT GACCGGCACG GARTATCCGC AGGCGGCGTT    300
   GGCCAACGCG TGGGTGCAAC TGGCCTACGG TGCGCACCAS GACGCCATCA CCGGCTCGGA    360
   GTCCGACCAG GTACTCAATG CTGGCGACCA CACCAGCCAG CAGACCAAAC TGGTGCACGC    420
   CGATCTCCAG GCGCGCCGGC CCGGTGGCAT ACGGATTGGT CGAAACCAAT CCGAAGGAAT    480
   TCATCACGGA CGGTCACGGA AAACGATCGC CCCAATGGGN GGACNACCCN AGCCAGGCGN    540
   ATTNACCGTT NAACAAGTTG GNGTAGGTTC TTTGATATCG AKCAACCGAT ACGGAKCGGM    600
   CCGCGGAATG GTAGACCACC ACCAGTGCCC NCAMGTMGTG CACCAGTTTG GTCATCGCCC    660
   GCAGATCGGT GACCCCGCCA AGCGTTCCGG ATGCGGAGAT GASGGTGACC AGCCYGGTTG    720
   ACCTGTTGAT CAGGTTNTCC CAGTGCCACG TCGGCAGCTG GCCGGT                   766
(2) INFORMATION FOR SEQ ID NO:104:
   (i) SEQUENCE CHARACTERISTICS:
      (A) LENGTH: 1231 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:104:
   CGGCACGAGA ATGTCGCCTG TGCCTCGATA GCCACTTGCG TGTGGTCGCG CTGCCAGCGG     60
   GTCAGCCAGG TCGCCTGGTC CAGGCCATCG GGCCGGCGCA GGAGCGCGAT GTTGGCCAGA    120
   CCCGGTGTAC GAGAACCGGA CTCGACNAAG TGTCGGCGCT GACGGCGGCT CAGTTCGCGG    180
   CACACGCCCA GATCTATCAG GCCGTCAGCG CCCAGGCCGC GGCGATTCAC GAGATGTTCG    240
   TCAACACTCT ACAGATNANC TCAGGGTCGT ATGCTGCTAC CGAGGCCGCC AACGCGGCCG    300
   CGGCCGGCTA GAGGAGTCAC TGCGATGGAT TTTGGGGCGT TGCCGCCGGA GGTCAATTCG    360
   GTGCGGATGT ATGCCGGTCC TGGCTCGGCA CCAATGGTCG CTGCGGCGTC GGCCTGGAAC    420
   GGGTTGGCCG CGGAGCTGAG TTCGGCGGCC ACCGGTTATG AGACGGTGAT CACTCAGCTC    480
   AGCAGTGAGG GGTGGCTAGG TCCGGCGTCA GCGGCGATGG CCGAGGCAGT TGCGCCGTAT    540
   GTGGCGTGGA TGAGTGCCGC TGCGGCGCAA GCCGAGCAGG CGGCCACACA GGCCAGGGCC    600
   GCCGCGGCCG CTTTTGAGGC GGCGTTTGCC GCGACGGTGC CTCCGCCGTT GATCGCGGCC    660
   AACCGGGCTT CGTTGATGCA GCTGATCTCG ACGAATGTCT TTGGTCAGAA CACCTCGGCG    720
   ATCGCGGCCG CCGAAGCTCA GTACGGCGAG ATGTGGGCCC AAGACTCCGC GGCGATGTAT    780
   GCCTACGCGG GCAGTTCGGC GAGCGCCTCG GCGGTCACGC CGTTTAGCAC GCCGCCGCAG    840
   ATTGCCAACC CGACCGCTCA GGGTACGCAG GCCGCGGCCG TGGCCACCGC CGCCGGTACC    900
   GCCCAGTCGA CGCTGACGGA GATGATCACC GGGCTACCCA ACGCGCTGCA AAGCCTCACC    960
   TCACNTCTGT TGCAGTCGTC TAACGGTCCG CTGTCGTGGC TGTGGCAGAT CTTGTTCGGC   1020
   ACGCCCAATT TCCCCACCTC AATTTCGGCA CTGCTGACCG ACCTGCAGCC CTACGCGAGC   1080
   TTNTTNTATA ACACCGAGGG CCTGCCGTAC TTCAGCATCG GCATGGGCAA CAACTTCATT   1140
   CAGTCGGCCA AGACCCTGGG ATTGATCGGC TAGGCGGCAC CGGCTGCGGT CGCGGNTGCT   1200
   GGGGATNCCG CCAAGGGCTT GCCTCGTGCC G                                  1231
(2) INFORMATION FOR SEQ ID NO: 105:
   (i) SEQUENCE CHARACTERISTICS:
      (A) LENGTH: 2041 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:105:
   CGGCACGAGC TCGTGCCGAT CAGTGCCATT GACGGCTTGT ACGACCTTCT GGGGATTGGA      60
   ATACCCAACC AAGGGGGTAT CCTTTACTCC TCACTAGAGT ACTTCGAAAA AGCCCTGGAG     120
   GAGCTGGCAG CAGCGTTTCC GGGTGATGGC TGGTTAGGTT CGGCCGCGGA CAAATACGCC     180
   GGCAAAAACC GCAACCACGT GAATTTTTTC CAGGAACTGG CAGACCTCGA TCGTCAGCTC     240
   ATCAGCCTGA TCCACGACCA GGCCAACGCG GTCCAGACGA CCCGCGACAT CCTGGAGGGC     300
   GCCAAGAAAG GTCTCGAGTT CGTGCGCCCG GTGGCTGTGG ACCTGACCTA CATCCCGGTC     360
   GTCGGGCACG CCCTATCGGC CGCCTTCCAN GCGCCGTTTT GCGCGGGCGC GATGGCCGTA     420
   GTGGGCGGCG CGCTTGCCTA CTTGGTCGTG AAAACGCTGA TCAACGCGAC TCAACTCCTC     480
   AAATTGCTTG CCAAATTGGC GGAGTTGGTC GCGGCCGCCA TTGCGGACAT CATTTCGGAT     540
   GTGGCGGACA TCATCAAGGG CATCCTCGGA GAAGTGTGGG AGTTCATCAC AAACGCGCTC     600
   AACGGCCTGA AAGAGCTTTG GGACAAGCTC ACGGGGTGGG TGACCGGACT GTTCTCTCGA     660
   GGGTGGTCGA ACCTGGAGTC CTTCTTTGCG GGCGTCCCCG GCTTGACCGG CGCGACCAGC     720
   GGCTTGTCGC AAGTGACTGG CTTGTTCGGT GCGGCCGGTC TGTCCGCATC GTCGGGCTTG     780
   GCTCACGCGG ATAGCCTGGC GAGCTCAGCC AGCTTGCCCG CCCTGGCCGG CATTGGGGGC     840
   GGGTCCGGTT TTGGGGGCTT GCCGAGCCTG GCTCAGGTCC ATGCCGCCTC AACTCGGCAG     900
   GCGCTACGGC CCCGAGCTGA TGGCCCGGTC GGCGCCGCTG CCGAGCAGGT CGGCGGGCAG     960
   TCGCAGCTGG TCTCCGCGCA GGGTTCCCAA GGTATGGGCG GACCCGTAGG CATGGGCGGC    1020
   ATGCACCCCT CTTCGGGGGC GTCGAAAGGG ACGACGACGA AGAAGTACTC GGAAGGCGCG    1080
   GCGGCGGGCA CTGAAGACGC CGAGCGCGCG CCAGTCGAAG CTGACGCGGG CGGTGGGCAA    1140
   AAGGTGCTGG TACGAAACGT CGTCTAACGG CATGGCGAGC CAAATCCATT GCTAGCCAGC    1200
   GCCTAACAAC GCGCAATGCT AAACGGAAGG GACACGATCA ATGACGGAAA ACTTGACCGT    1260
   CCAGCCCGAG CGTCTCGGTG TACTGGCGTC GCACCATGAC AACGCGGCGG TCGATGCNTC    1320
   CTCGGGCGTC GAAGCTGCCG CTGGCCTAGG CGAATCTGTG GCGATCACTC ACGGTCCGTA    1380
   CTGCTCACAG TTCAACGACA CGTTAAATGT GTACTTGACT GCCCACAATG CCCTGGGCTC    1440
   GTCCTTGCAT ACGGCCGGTG TCGATCTCGC CAAAAGTCTT CGAATTGCGG CGAAGATATA    1500
   TAGCGAGGCC GACGAAGCGT GGCGCAAGGC TATCGACGGG TTGTTTACCT GACCACGTTT    1560
   GCTGCCCGCA GTGCAGGCCA CGACGTAGCG CAGGTCGTGT CCCTCGTAGG CGTGGATGCG    1620
   ACCGGCCAGC ACCAGCACCC GGTGCGCACC GATGGGCACG GACAGTAGCT CGCCCGCATG    1680
   CCCGGCTGCG GTTGGCGGCA CAAACCCGGG CAGTTCGGCC TGCGGCAGCA CGGTGGTNGG    1740
   GGAGCCCAAC GCCGCAACGG CCGGTAACCA TCCCGACCCG AGCACGACCG AGACGTCATG    1800
   TTCGCCGATC CCGGTGCGGT CAGCGATGAC CTGCGCCGCC CGCCGGGCCA GTTTGTCGGG    1860
   ATCGGGGCGC GGGTCAGCCA CACTGGGCGA GCTTAACTGA GCCGCTCGCC GGGGAGCGGG    1920
   TGCTNGTCGA TGAGATACTG CGAGCATGCC AGCAGCCAGC GCATCCGACC GCGTCGAGGA    1980
   ATTGGTGCGG CGCCGTGGTG GCGAGCTGGT CGAGCTGTCC CATGCCATCC ACCTCGTGCC    2040
   G                                                                    2041
(2) INFORMATION FOR SEQ ID NO:106:
   (i) SEQUENCE CHARACTERISTICS:
      (A) LENGTH: 1202 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:106:
   GAGCTCACCG CTATCAACCA ATACTTTCTG CACTCCAAGA TGCAGGACAA CTGGGGTTTT   60
   ACCGAGCTGG CGGCCCACAC CCGCGCGGAG TCGTTCGACG AAATGCGGCA CGCCGAGGAA  120
   ATCACCGATC GCATCTTGTT GCTGGATGGT TTGCCGAACT ACCAGCGCAT CGGTTCGTTG  180
   CGTATCGGCC AGACGCTCCG CGAGCAATTT GAGGCCGATC TGGCGATCGA ATACGACGTG  240
   TTGAATCGTC TCAAGCCAGG AATCGTCATG TGCCGGGAGA AACAGGACAC CACCAGCGCC  300
   GTACTGCTGG AGAAAATCGT TGCCGACGAG GAAGAACACA TCGACTACTT GGAAACGCAG  360
   CTGGAGCTGA TGGACAAGCT AGGAGAGGAG CTTTACTCGG CGCAGTGCGT CTCTCGCCCA  420
   CCGACCTGAT GCCCGCTTGA GGATTCTCCG ATACCACTCC GGGCGCCGCT GACAAGCTCT  480
   AGCATCGACT CGAACAGCGA TGGGAGGGCG GATATGGCGG GCCCCACAGC ACCGACCACT  540
   GCCCCCACCG CAATCCGAGC CGGTGGCCCG CTGCTCAGTC CGGTGCGACG CAACATTATT  600
   TTCACCGCAC TTGTGTTCGG GGTGCTGGTC GCTGCGACCG GCCAAACCAT CGTTGTGCCC  660
   GCATTGCCGA CGATCGTCGC CGAGCTGGGC AGCACCGTTG ACCAGTCGTG GGCGGTCACC  720
   AGCTATCTGC TGGGGGGAAC ACTSKYGKKK KTGKKGKSKS KSRMRMKCTC GGTGATCTGC  780
   TCGGCCGCAA CAGGGTGCTG CTAGGCTCCG TCGTGGTCTT CGTCGTTGGC TCTGTGCTGT  840
   GCGGGTTATC GCAGACGATG ACCATGCTGG CGATCTCTCG CGCACTGCAG GGCGTCGGTG  900
   CCGGTGCGAT TTCCGTCACC GCCTACGCGC TGGCCGCTGA GGTGGTCCCA CTGCGGGACC  960
   GTGGCCGCTA CCAGGGCGTC TTANGTGCGG TGTTCGGTGT CAACACGGTC ACCGGTCCGC 1020
   TGCTGGGGGG CTGGCTCACC GACTATCTGA GCTGGCGGTG GGCGTTCCGA CCACCAGCCC 1080
   CATCACCGAC CCGATCGCGG TCATCGCGGC GAACACCGCC CTCGCGGCGT TGCGGGCAGG 1140
   TCCCTTGGGG AACGTGGTCC CACAGCGCCA GAACGGTCGG AAATGCGATG GCCGACCCAC 1200
   AC                                                                1202
(2) INFORMATION FOR SEQ ID NO: 107:
   (i) SEQUENCE CHARACTERISTICS:
      (A) LENGTH: 496 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:107:
   GGCGGCGGCA GTTGGCCAGC AGTTNGGGCG GGGGAGCCGG TTCGGNGACC AAGAAATCGG   60
   CCTGGGCAAG CAGCCGGGAC CGCGNACCGT GATCAGTTNG GATCGCCGGG ACCGCCGCCG  120
   ACCAANGCCA TTCCGCCGNT GAGGAAGTCG GAANTNTGCG CAGTGATGAC GCCCTGCTGC  180
   AACGCNTCCC GGATTGCCGA GCGGATCGCC GCCGAACGGC GGTGCTCACC ACCGGCGAGC  240
   ACCCCTACNG ACAGGCCCGC ATAGCTGAAT GACGCCGGGT NACCGCCGTC CCNTCCACCG  300
   NGANATCGGC CCGGANGCAA AAGATCCGTC GGCGCTCCGC CTCGGCGACG ACAGCCACGT  360
   TCACCCGCGC GTTATCGGTG GCCGCGATCG CATACCAGGC GCCGTCAAGG TNGCCGTYGC  420
   GGTAGTCACG CACCGACAAG GTGATYTGGT CCATCGCCTN GACGGCGGGG GTGACGCTGG  480
   GGGCGATCAM GTGCAC                                                 496
(2) INFORMATION FOR SEQ ID NO:108:
   (i) SEQUENCE CHARACTERISTICS:
      (A) LENGTH: 849 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:108:
   TGGATTCCGA TAGCGGTTTC GGCCCCTCGA CGGGCGACCA CGGCGCGCAG GCCTCCGAAC      60
   GGGGGGCCGG GACGCTGGGA TTCGCCGGGA CCGCAACCAA AGAACGCCGG GTCCGGGCGG     120
   TCGGGCTGAC CGCACTGGCC GGTGATGAGT TCGGCAACGG CCCCCGGATG CCGATGGTGC     180
   CGGGGACCTG GGAGCAGGGC AGCAACGAGC CCGAGGCGCC CGACGGATCG GGGAGAGGGG     240
   GAGGCGACGG CTTACCGCAC GACAGCAAGT AACCGAATTC CGAATCACGT GGACCCGTAC     300
   GGGTCGAAAG GAGAGATGTT ATGAGCCTTT TGGATGCTCA TATCCCACAG TTGGTGGCCT     360
   CCCAGTCGGC GTTTGCCGCC AAGGCGGGGC TGATGCGGCA CACGATCGGT CAGGCCGAGC     420
   AGGCGGCGAT GTCGGCTCAG GCGTTTCACC AGGGGGAGTC GTCGGCGGCG TTTCAGGCCG     480
   CCCATGCCCG GTTTGTGGCG GCGGCCGCCA AAGTCAACAC CTTGTTGGAT GTCGCGCAGG     540
   CGAATCTGGG TGAGGCCGCC GGTACCTATG TGGCCGCCGA TGCTGCGGCC GCGTCGACCT     600
   ATACCGGGTT CTGATCGAAC CCTGCTGACC GAGAGGACTT GTGATGTCGC AAATCATGTA     660
   CAACTACCCC GCGATGTTGG GTCACGCCGG GGATATGGCC GGATATGCCG GCACGCTGCA     720
   GAGCTTGGGT GCCGAGATCG CCGTGGAGCA GGCCGCGTTG CAGAGTGCGT GGCAGGGCGA     780
   TACCGGGATC ACGTATCAGG CGTGGCAGGC ACANTGGTAA CCANGCCANG GAAGATTTGG     840
   TGCGGGCCT                                                             849
(2) INFORMATION FOR SEQ ID NO:109:
   (i) SEQUENCE CHARACTERISTICS:
      (A) LENGTH: 97 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:109:
   Met Ser Leu Leu Asp Ala His Ile Pro Gln Leu Val Ala Ser Gln Ser
    1               5                  10                  15
   Ala Phe Ala Ala Lys Ala Gly Leu Met Arg His Thr Ile Gly Gln Ala
               20                  25                  30
   Glu Gln Ala Ala Met Ser Ala Gln Ala Phe His Gln Gly Glu Ser Ser
           35                  40                  45
   Ala Ala Phe Gln Ala Ala His Ala Arg Phe Val Ala Ala Ala Ala Lys
       50                  55                  60
   Val Asn Thr Leu Leu Asp Val Ala Gln Ala Asn Leu Gly Glu Ala Ala
   65                  70                  75                  80
   Gly Thr Tyr Val Ala Ala Asp Ala Ala Ala Ala Ser Thr Tyr Thr Gly
                   85                  90                  95
   Phe
(2) INFORMATION FOR SEQ ID NO:110:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:110:
   Met Ser Leu Leu Asp Ala His Ile Pro Gln Leu Val Ala Ser Gln
    1               5                  10                  15
(2) INFORMATION FOR SEQ ID NO:111:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:111:
   Ala His Ile Pro Gln Leu Val Ala Ser Gln Ser Ala Phe Ala Ala
    1               5                  10                  15
(2) INFORMATION FOR SEQ ID NO:112:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:112:
   Leu Val Ala Ser Gln Ser Ala Phe Ala Ala Lys Ala Gly Leu Met
    1               5                  10                  15
(2) INFORMATION FOR SEQ ID NO:113:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:113:
   Ser Ala Phe Ala Ala Lys Ala Gly Leu Met Arg His Thr Ile Gly
   1 5 10 15
(2) INFORMATION FOR SEQ ID NO:114:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:114:
   Lys Ala Gly Leu Met Arg His Thr Ile Gly Gln Ala Glu Gln Ala
   1 5 10 15
(2) INFORMATION FOR SEQ ID NO:115:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:115:
   Arg His Thr Ile Gly Gln Ala Glu Gln Ala Ala Met Ser Ala Gln
   1 5 10 15
(2) INFORMATION FOR SEQ ID NO:116:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:116:
   Gln Ala Glu Gln Ala Ala Met Ser Ala Gln Ala Phe His Gln Gly
   1 5 10 15
(2) INFORMATION FOR SEQ ID NO:117:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:117:
   Ala Met Ser Ala Gln Ala Phe His Gln Gly Glu Ser Ser Ala Ala
    1               5                  10                  15
(2) INFORMATION FOR SEQ ID NO:118:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:118:
   Ala Phe His Gln Gly Glu Ser Ser Ala Ala Phe Gln Ala Ala His
    1               5                  10                  15
(2) INFORMATION FOR SEQ ID NO:119:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:119:
   Glu Ser Ser Ala Ala Phe Gln Ala Ala His Ala Arg Phe Val Ala
    1               5                  10                  15
(2) INFORMATION FOR SEQ ID NO:120:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:120:
   Phe Gln Ala Ala His Ala Arg Phe Val Ala Ala Ala Ala Lys Val
   1 5 10 15
(2) INFORMATION FOR SEQ ID NO:121:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:121:
   Ala Arg Phe Val Ala Ala Ala Ala Lys Val Asn Thr Leu Leu Asp
   1 5 10 15
(2) INFORMATION FOR SEQ ID NO:122:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:122:
   Ala Ala Ala Lys Val Asn Thr Leu Leu Asp Val Ala Gln Ala Asn
   1 5 10 15
(2) INFORMATION FOR SEQ ID NO:123:
   (i) SEQUENCE CHARACTERISTICS:
      (A) LENGTH: 15 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:123:
   Asn Thr Leu Leu Asp Val Ala Gln Ala Asn Leu Gly Glu Ala Ala
   1 5 10 15
(2) INFORMATION FOR SEQ ID NO:124:
   (i) SEQUENCE CHARACTERISTICS:
      (A) LENGTH: 18 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:124:
   Val Ala Gln Ala Asn Leu Gly Glu Ala Ala Gly Thr Tyr Val Ala Ala
    1               5                   10                  15
   Asp Ala
(2) INFORMATION FOR SEQ ID NO:125:
   (i) SEQUENCE CHARACTERISTICS:
      (A) LENGTH: 1752 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:125:
   CGGCACGAGA ATGTCGCCTG TGCCTCGATA GCCACTTGCG TGTGGTCGCG CTGCCAGCGG   60
   GTCAGCCAGG TCGCCTGGTC CAGGCCATCG GGCCGGCGCA GGAGCGCGAT GTTGGCCAGA  120
   CCCGGTGTAC GAGAACCGGA CTCGACNAAG TGTCGGCGCT GACGGCGGCT CAGTTCGCGG  180
   CACACGCCCA GATCTATCAG GCCGTCAGCG CCCAGGCCGC GGCGATTCAC GAGATGTTCG  240
   TCAACACTCT ACAGATNANC TCAGGGTCGT ATGCTGCTAC CGAGGCCGCC AACGCGGCCG  300
   CGGCCGGCTA GAGGAGTCAC TGCGATGGAT TTTGGGGCGT TGCCGCCGGA GGTCAATTCG  360
   GTGCGGATGT ATGCCGGTCC TGGCTCGGCA CCAATGGTCG CTGCGGCGTC GGCCTGGAAC  420
   GGGTTGGCCG CGGAGCTGAG TTCGGCGGCC ACCGGTTATG AGACGGTGAT CACTCAGCTC  480
   AGCAGTGAGG GGTGGCTAGG TCCGGCGTCA GCGGCGATGG CCGAGGCAGT TGCGCCGTAT  540
   GTGGCGTGGA TGAGTGCCGC TGCGGCGCAA GCCGAGCAGG CGGCCACACA GGCCAGGGCC  600
   GCCGCGGCCG CTTTTGAGGC GGCGTTTGCC GCGACGGTGC CTCCGCCGTT GATCGCGGCC  660
   AACCGGGCTT CGTTGATGCA GCTGATCTCG ACGAATGTCT TTGGTCAGAA CACCTCGGCG  720
   ATCGCGGCCG CCGAAGCTCA GTACGGCGAG ATGTGGGCCC AAGACTCCGC GGCGATGTAT  780
   GCCTACGCGG GCAGTTCGGC GAGCGCCTCG GCGGTCACGC CGTTTAGCAC GCCGCCGCAG  840
   ATTGCCAACC CGACCGCTCA GGGTACGCAG GCCGCGGCCG TGGCCACCGC CGCCGGTACC  900
   GCCCAGTCGA CGCTGACGGA GATGATCACC GGGCTACCCA ACGCGCTGCA AAGCCTCACC  960
   TCACNTCTGT TGCAGTCGTC TAACGGTCCG CTGTCGTGGC TGTGGCAGAT CTTGTTCGGC 1020
   ACGCCCAATT TCCCCACCTC AATTTCGGCA CTGCTGACCG ACCTGCAGCC CTACGCGAGC 1080
   TTNTTNTATA ACACCGAGGG CCTGCCGTAC TTCAGCATCG GCATGGGCAA CAACTTCATT 1140
   CAGTCGGCCA AGACCCTGGG ATTGATCGGC TAGGCGGCAC CGGCTGCGGT CGCGGCTGCT 1200
   GGGGATGCCG CCAAGGGCTT GCCTGGACTG GGCGGGATGC TCGGTGGCGG GCCGGTGGCG 1260
   GCGGGTCTGG GCAATGCGGC TTCGGTTGGC AAGCTGTCGG TGCCGCCGGT GTGGANTGGA 1320
   CCGTTGCCCG GGTCGGTGAC TCCGGGGGCT GCTCCGCTAC CGGTGAGTAC GGTCAGTGCC 1380
   GCCCCGGAGG CGGCGCCCGG AAGCCTGTTG GGCGGCCTGC CGCTANCTGG TGCGGGCGGG 1440
   GCCGGCGCGG GTCCACGCTA CGGATTCCRT CCCACCGTCA TGGCTCGCCC ACCCTTCGMC 1500
   GGGATAGTCG CTGCCGCAAC GTATTAACGC GCCGGCCTCG GCTGGTGTGG TCCGCTGCGG 1560
   GTGGCAATTG GTCNGCGCCG AAATCTCSGT GGGTTATTTR CGGTGGGATT TTTTCCCGAA 1620
   GCCGGGTTCA RCACCGGATT TCCTAACGGT CCCGCKACTC TCGTGCCGAA TTCSGCACTA 1680
   AGTGACGTCC GGCGGAAACC CGTTGGGTNT GAAAGCTTCA GAAAGGCCCG CTCCCAGGGG 1740
   TTCGGCAAAC GG                                                    1752
(2) INFORMATION FOR SEQ ID NO:126:
   (i) SEQUENCE CHARACTERISTICS:
      (A) LENGTH: 400 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:126:
   Met Asp Phe Gly Ala Leu Pro Pro Glu Val Asn Ser Val Arg Met Tyr
    1               5                  10                  15
   Ala Gly Pro Gly Ser Ala Pro Met Val Ala Ala Ala Ser Ala Trp Asn
               20                  25                  30
   Gly Leu Ala Ala Glu Leu Ser Ser Ala Ala Thr Gly Tyr Glu Thr Val
           35                  40                  45
   Ile Thr Gln Leu Ser Ser Glu Gly Trp Leu Gly Pro Ala Ser Ala Ala
       50                  55                  60
   Met Ala Glu Ala Val Ala Pro Tyr Val Ala Trp Met Ser Ala Ala Ala
   65                  70                  75                  80
   Ala Gln Ala Glu Gln Ala Ala Thr Gln Ala Arg Ala Ala Ala Ala Ala
                   85                  90                  95
   Phe Glu Ala Ala Phe Ala Ala Thr Val Pro Pro Pro Leu Ile Ala Ala
               100                 105                 110
   Asn Arg Ala Ser Leu Met Gln Leu Ile Ser Thr Asn Val Phe Gly Gln
           115                 120                 125
   Asn Thr Ser Ala Ile Ala Ala Ala Glu Ala Gln Tyr Gly Glu Met Trp
       130                 135                 140
   Ala Gln Asp Ser Ala Ala Met Tyr Ala Tyr Ala Gly Ser Ser Ala Ser
   145                 150                 155                 160
   Ala Ser Ala Val Thr Pro Phe Ser Thr Pro Pro Gln Ile Ala Asn Pro
                   165                 170                 175
   Thr Ala Gln Gly Thr Gln Ala Ala Ala Val Ala Thr Ala Ala Gly Thr
               180                 185                 190
   Ala Gln Ser Thr Leu Thr Glu Met Ile Thr Gly Leu Pro Asn Ala Leu
           195                 200                 205
   Gln Ser Leu Thr Ser Xaa Leu Leu Gln Ser Ser Asn Gly Pro Leu Ser
       210                 215                 220
   Trp Leu Trp Gln Ile Leu Phe Gly Thr Pro Asn Phe Pro Thr Ser Ile
   225                 230                 235                 240
   Ser Ala Leu Leu Thr Asp Leu Gln Pro Tyr Ala Ser Xaa Xaa Tyr Asn
                   245                 250                 255
   Thr Glu Gly Leu Pro Tyr Phe Ser Ile Gly Met Gly Asn Asn Phe Ile
               260                 265                 270
   Gln Ser Ala Lys Thr Leu Gly Leu Ile Gly Ser Ala Ala Pro Ala Ala
           275                 280                 285
   Val Ala Ala Ala Gly Asp Ala Ala Lys Gly Leu Pro Gly Leu Gly Gly
       290                 295                 300
   Met Leu Gly Gly Gly Pro Val Ala Ala Gly Leu Gly Asn Ala Ala Ser
   305                 310                 315                 320
   Val Gly Lys Leu Ser Val Pro Pro Val Trp Xaa Gly Pro Leu Pro Gly
                   325                 330                 335
   Ser Val Thr Pro Gly Ala Ala Pro Leu Pro Val Ser Thr Val Ser Ala
               340                 345                 350
   Ala Pro Glu Ala Ala Pro Gly Ser Leu Leu Gly Gly Leu Pro Leu Xaa
           355                 360                 365
   Gly Ala Gly Gly Ala Gly Ala Gly Pro Arg Tyr Gly Phe Xaa Pro Thr
       370                 375                 380
   Val Met Ala Arg Pro Pro Phe Xaa Gly Ile Val Ala Ala Ala Thr Tyr
   385                 390                 395                 400
(2) INFORMATION FOR SEQ ID NO:127:
   (i) SEQUENCE CHARACTERISTICS:
      (A) LENGTH: 474 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:127:
   GGCACGAGCA CCAGTTGACC CGCGAAGAAC CTGACCGCGC CACCCAGCGC CGCCCGCATC    60
   ACCGGCCCCG TCCCACGAAC CTTTTCGGTA AACGAGCCAC TCCAGCGGAG ATCGGTACCG   120
   CCCGACGCAT TTGGTGTAAG GACCACCTCG CCGAAGTAGT CCTGGACGGG TGTCCTCGCG   180
   CCAACCAGCT TGTAGACGTG GCGACGGTCC TGCTCATACT CGACGGTCTC TTCCTGCACG   240
   AACACCGGCC ACATGCCTAG TTTGCGGATG GCCCCGATGC CGCCGGGCGC GGGATCACCG   300
   CGTCGCGCCC AACTCGATTG AGCAACGATG GGCTTGGCCC AGGTCGCCCA GTTGCCACCG   360
   TCTGTCACGA GCCGAAACAA GGTTGCAGCC GGCGCGCTGC TGGTCTTGGT GACCTCGAAC   420
   GAAAATTTCC GACCCGACAT GCGCGACTCC CGAAACGACA ACTGAAGCTC GTGC         474
(2) INFORMATION FOR SEQ ID NO:128:
   (i) SEQUENCE CHARACTERISTICS:
      (A) LENGTH: 1431 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:128:
   CTGCGCGCCG GAAAAAANTA TTACTGGCAG GACCGGCAGA ATGCATGGTG ATATTCCGGT    60
   GATGAGGCCG CCGAGGAACC GACTAGTGCG AGGGTCAACA CATCGGTTAT TCGTTGCCGT   120
   TTAGGTCTTG GATCTGCCGG GACGGCAACG AGTTGGCAGG ACCGCTCACG CGAGCGCTGT   180
   TGACAGAGTC GGTTCACGTC GAACTCGCCA CCCGTCAGAT GCGAATGATA GCCACATCGG   240
   CCACACCATC GACGGCGTCG AAGTCGCCGT CGTGGGTCAC GACCGGCACC CCTTGCGACG   300
   TGGCAACGGC AGCGGCCCTC ACCGGACGGG ACCGAGATCG TCGGTGGTGT CGCCAGTGAG   360
   CGTTGCGAGG TCGCGGGTGC AATCCCGCAT CTGCTTGCGT ATGCCGAAGC CGCCGCAGCA   420
   GCTCGTCTCG ACTCAACCAT CGGCGCCGTG CGGGCTGCCT GCGGTCAGCA GCGCAACGGG   480
   TTTGCCGTTG GCAGTGATGG TGATGTCTTC GCCGGCCTGC ACGCGCCGTA GCAGCCCGGC   540
   GGTGTTGTTG CGCAGTTCGC GAGACGCGAC TTCAGCAGGC ATGCTGCGGG GATCGGCTTG   600
   CGCTGGGCGC GGTGTCACCG TCATGCGCTT GGGATATCAC GTGATCTATC GGCACGAAGC   660
   CGCCGGATGA GCGAGGCAAA CCGCCTACAC GGGCTGCCTC GCCTTGACCG CGCCGAACGT   720
   TACTGTGCCG GGGGCATCAG CACCGTATCG ATCATGTACA CCGTCGCGTG GGCGGTGTGA   780
   CTCCGCCACA TACCAAACGG GCGTTGTTGA CCATGAGTCG TCGCGGGCGC CTATCACCGT   840
   CAGGTCGGCA CCTTGCAGGT CTGATGGGTG CCGTCGATCC TGCTCGGACT CGCCTGGCCG    900
   GCTATCACGT GGTAGGTCAG GATGCTGCTG AGCAGCTTGG CGTCAGTCTT GAGTTGATCG    960
   ATAGTGGCCG CCGGCAGCTT GTCGAATGCG GCGTTGGTGG GGGCGAAAAC GGTGTACTCG   1020
   CCGCCGTTGA GGGTGTCGAC CAGATTCACA TCCGGGTTCA GCTTGCCCGA CAGAGCCGAG   1080
   GTCAGGGTAC TGAGCATCGG GTTGTTGGAA GCCGCGGTAG CGACCGGGTC TTGCGCCATT   1140
   CCGGCCACCG ATCCGGGACC GGTGGGATTT TGCGCCGCGT ATTGCGCGCA CCCACGACCA   1200
   ATCAGGTCCG CTGCGGTCAG CCATTGCCGC CGTGGTAACG GGCGCCGCCG GGCTGGTCGC   1260
   CGGTTTCGGG CTGGTGTCTT GCGACACGGG TTTGGTGCTC GAACAACCCG CTAAGAACGC   1320
   AATCGCGATG GCTGCGAGGC TCGCTGCTGC GGCCGGTTTG GCCTGAACGT TGATCATCGC   1380
   TTCGATTCCT TTGCTTCTGC GGCGGCGTTG AACGCCGTCC TCCTGGGTGG A            1431
(2) INFORMATION FOR SEQ ID NO:129:
   (i) SEQUENCE CHARACTERISTICS:
      (A) LENGTH: 279 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:129:
   GCACGAGAGT CGTATCTTTG CACCCAGCGC CCGTAGGAAA CCGCTGGCCT GGCTAACTCA     60
   GATGCGGGCG GCCGTCGATT CGAGAGGTAA CCGATCGCCC GCCGACAATG GGTTACCCAC    120
   CGAGACTGAT TGCCGCGCAG CCGCCTTCGA CGTGTAAGCG CCGGTTCGTG CATGCCCGGA    180
   ACGGCTGCAC TCACGGACCT TCTACGTAGT ACGTGACGGA CTTTTACGCA TTATCGCTGA    240
   CGATCTTTGC CTCCCAGGAC TCCAGAATCT ACTCGTGCC                           279
(2) INFORMATION FOR SEQ ID NO:130:
   (i) SEQUENCE CHARACTERISTICS:
      (A) LENGTH: 1470 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:130:
   ACCGCCACCC GCAGCCCGGA ATCACCGTCG GTAACCTGCG AATACAATTT CTTCATCGAC     60
   GACTTCGCGA ACAGCGAACC CGAGCCCACC GCCTGATAGC CTTCTTCCTC GATGTTCCAA    120
   CCGCCGGCGG CGTCGAACGA AACGATACGA CCCGCGCTCT GCGGGTCAGA CGCATGAATG    180
   TCGTAGCCCG CCAGCAACGG CAACGCCAGC AGACCCTGCA TCGCGGCCGC CAGATTGCCA    240
   CGCACCATAA TCGCCAGCCG GTTGATTTTG CCGGCAAACG TCAGCGGCAC ACCCTCGAGC    300
   TTCTCGTAGT GCTCAAGTTC CACGGCATAC AGCCGGGCAA ACTCAACCGC GACCGCAGCC    360
   GTGCCAGCGA TGCCGGTAGC GGTGTAGTCA TCGGTGATAT ACACCTTGCG CACATCACGC    420
   CCAGAAATCA TGTTGCCCTG CGTCGAACGC CGGTCACCCG CCATGACAAC ACCGCCGGGG    480
   TATTTCAGCG CGACAATGGT GGTGCCGTGC GGCAGTTGCG CATCGCCGCC TGCGAGTGGC    540
   GCACCGCCGC TGATGCTTGC CGGCAGCAAC TCCGGCGCCT GGCGGCGCAG GAAGTCAAGT    600
   GAAAGAAGAT AGGTCTACAG CGGGTGTTCC AGAGAGTGAA TTAATGGACA GGCGATCGGG    660
   CAACGGCCAG GTCACTGTCC GCCCTTTTGG ACGTATGCGC GGACGAAGTC CTCGGCGTTC    720
   TCCTCGAGGA CGTCGTCGAT TTCGTCGAGC AGATCGTCGG TCTCCTCGGT CAGCTTTTCG    780
   CGACGCTCCT GGCCCGCGGC GGTGCTGCCG GCGATGTCGT CATCATCGCC GCCGCCACCG    840
   CCACGCTTGG TCTGCTCTTG CGCCATCGCC GCCTCCTGCT TCCTCATGGC CTTTCAAAAG    900
   GCCGCGGGTG CGCGTCACAC GCCCGCTGTC TTTCTCTCAC CTACCGGTCA ACACCAACGT    960
   TTCCCGGCCT AACCAGGCTT AGCGAGGCTC AGCGGTCAGT TGCTCTACCA GCTCCACGGC   1020
   ACTGTCCACC GAATCCAGCA ACGCACCAAC ATGCGCCTTA CTACCCCGCA ACGGCTCCAG   1080
   CGTCGGGATG CGAACCAGCG AGTCGCCGCC AGGTCGAAGA TCACCGAGTC CCAGCTAGCC   1140
   GCGGCGATAT CAGCCCCGAA CCGGCGCAGG CATTTCGCCG CGGAAATACG CGCGGGTGTC   1200
   GGTCGGCGGT TCTCCACCGC ACTCAGCACC TGGTGTTTCG GTGACTAAAC GCTTTATCGA   1260
   GCCGCGCGCG ACCAGCCGGT TGTACAGGCC CTTGTCCAGC CGGACATCGG AGTACTGCAG   1320
   GTTGACGAGG TGCAGCCGGG GCGCCGACCA GCTCAGGTTC TCCCGCTGCC GGAAACCGTC   1380
   GAGCAGCCGC AGTTTGGCCG GCCAGTCCAG CAGCTCCGCG CAATCCATCG GGTCACGCTC   1440
   GAGCTGATCC AGCACGTGTG CCCAGGTTTC                                    1470
(2) INFORMATION FOR SEQ ID NO:131:
   (i) SEQUENCE CHARACTERISTICS:
      (A) LENGTH: 1059 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:131:
   ATTCCCATCG CTCCGGCACC TATCACCAGG TAGTCGGTTT CGATGGTTTT CGCCGGCCCT     60
   TGCGTTGGCC TGGGCCACGG GTCGTTCATG GGCCCTCCTG TGCGGATTGG AATTTGTGAC    120
   AACGAAATCG GGCGATCGGT GAGCAATCGT CGCCGATGCA AGACACGCTT TCGCTGCCGC    180
   GGCGTCAGGT GGAGTTTAGG CCAGCGTAAC AACGTAGACC GGCCACTGAC CAAACCCCAA    240
   ACCCACAAAC CCTGGACGCA TGCGGGTCTC GGGCGTCAAA TTCCGGGTAG ATATCGTATA    300
   CCGATATCGG ATGCCGTAGC CTTATCGAGG CATGAGACGC CCGCTAGACC CACGCGATAT    360
   TCCAGATGAG CTGCGGCGAC GGCTGGGGCT CTTGGATGCG GTGGTGATCG GGCTTGGGTC    420
   CATGATCGGT GCCGGAATCT TTGCTCGTGC CGAATTCGGC ACGAGCTCGT GCCGAATTCG    480
   GCACGAGATT CCAATCCCCA GAAGGTCGTA CAAGCCGTCA ATGGCACTTG ATCGTTGGAT    540
   CGATGATGAA CGCTCTGCTC ATGCCTGCCG CCTATCTCAA CGGTCGTCGA TTCCATGCAT    600
   TAGCCTTGGT TCTGCATTGC ACGCGTAGGG CCTACAGTCT GGCTGTCATG CTTGGCCGAT    660
   GTCAACAGTT TTTTTCATGC TAAGCAGATC GTCAGTTTTG AGTTCGTGAA GACGGCATGT    720
   TCACTTGTTG TCGACTACAT CGTCTGCGCA CATTTGCCCT CCTGCAACTG CGCTGCGACA    780
   ATGCGCCAAC CGCCGTGTAG CTCGTGCCGA ATTCGGCACG AGGATCCACC GGAGATGGCC    840
   GACGACTACG ACGAGGCCTG GATGCTCAAC ACCGTGTTCG ACTATCACAA CGAGAACGCA    900
   AAAGAAGAGG TCATCCATCT CGTGCCCGAC GTGAACAAGG AGAGGGGGCC CATCGAACTC    960
   GTAACCAAGG TAGACAAAGA GGGACATCAG ACTCGTCTAC GATGGGGAGC CACGTTTTCA   1020
   TACAAGGAAC ATCCTAAGTT TTGATTCGGG AACATCCTA                          1059
(2) INFORMATION FOR SEQ ID NO:132:
   (i) SEQUENCE CHARACTERISTICS:
      (A) LENGTH: 153 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID.NO:132:
   GCACGAGGCA TTGGCGGGCA TCTGCATAAA CGGTGACGTA TCAGCACAAA ACAGCGGAGA     60
   GAACAACATG CGATCAGAAC GTCTCCGGTG GCTGGTAGCC GCAGAAGGTC CGTTCGCCTC    120
   GGTGTATTTC GACGACTCGC ACGACTCGTG CCG                                 153
(2) INFORMATION FOR SEQ ID NO:133:
   (i) SEQUENCE CHARACTERISTICS:
      (A) LENGTH: 387 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:133:
   CCGCGCGGTC GATCAGCGAG CCAGGCAAAA ACTCCGTCGA GCCCGAGTCG ATGATGGTCA    60
   CCCGGCGCAG CATCTGGCGA ACGATCACCT CGATGTGCTT GTCGTGGATC GACACACCTT   120
   GGGCGCGGTA GACCTCCTGG ACCTCGCGAA CCAGGTGTAT CTGCACCTCG CGGGGGCCCT   180
   GCACCCGCAG CACCTCATGC GGGTCGGCCG AGCCTTCCAT CAGCTGCTGG CCCACCTCGA   240
   CGTGGTCGCC ATCGGAGAGC ACCCGTTCGG AACCGTCTTC GTGCTTGAAC ACCCGCAGCC   300
   GCTGCCGCTT GGAGATCTTG TCGTAGACCA CTTCCTCACC GCCGTCGTCA GGAACGATGG   360
   TGATCTTGTA GAACCGCTCG CCGTCCT                                       387
(2) INFORMATION FOR SEQ ID NO:134:
   (i) SEQUENCE CHARACTERISTICS:
      (A) LENGTH: 389 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:134:
   GTTCAGCACG GCTATCCGAT TGTGCCGTTC GCTTCGGTGG GTGCTGAACA CGGCATCGAC    60
   ATCGTGCTCG ACAACGAATC CCCACTGCTG GCACCGGTCC AGTTCCTCGC CGAGAAGCTG   120
   CTCGGCACCA AAGACGGTCC GGCGCTGGTC CGTGGTGTCG GACTGACACC GGTACCGCGC   180
   CCCGAACGGC AGTATTACTG GTTCGGCGAG CCAACCGACA CCACAGAGTT TATGGGGCAG   240
   CAAGCCGACG ATAACGCCGC ACGCAGGGTG CGCGAGCGTG CCGCCGCCGC TATCGAACAC   300
   GGCATCGAGC TGATGCTGGC CGAGCGCGCA GCCGATCCAA ATCGATCCCT GGTCGGACGG   360
   CTCTTGCGCT CGGACGCCTA AGGCGCCCC                                     389
(2) INFORMATION FOR SEQ ID NO:135:
   (i) SEQUENCE CHARACTERISTICS:
      (A) LENGTH: 480 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:135:
   CCCGCGGTCG GAATGATCCC CGTCTCGTCG CGCGCCCATT TGATGCTGTT GATGAGCTGT    60
   TTGGAGAAGC CCGGTTGGCG TACCGGTGAG CCGGAATATC TGTTGGAAGC GTCACCGGAT   120
   GTNCACATGA ANTNCNTTGN CCCNGTNGCG GTNTTGGNTG NGGNAAACAC GTGTTGTNTA   180
   AGCCTTGNTG GNCTCGNAAG NGCCGTNGAC GCCTGTGTCG CCGAAGATAA TGAGCACCTG   240
   ACGGTTGGCG GGATCGCCGT TATCCCAAGG AATTCCGAGG TCGGTCCCGG AGATGCCGAA   300
   GCGTTCCAGG GTCTTGTTGG GGCTGTCCGG TCCGGTCACC CACTCGGCGA GGGATGTGGN   360
   AGCCCCGGCG AGCGTGGCAC CAGGATCCGG CGCCGCCGCC GGAGCAGGGT CGGNNGCTGN   420
   NCTGNNTTCC TNNNGCCNAA TTNNACTCCN NCNACAANCT TGNNNCCGAC TCNNACCCGN   480
(2) INFORMATION FOR SEQ ID NO:136:
   (i) SEQUENCE CHARACTERISTICS:
      (A) LENGTH: 587 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:136:
   GCACGAGGCT ACCGGCGCGT CGCCCGCCAT GCCCTGGATG CACGCGTAGC CACCCGTNCA    60
   TNCAGCGGGT CAGCCGCCGC GTCCGGGCTT AACGCTATAG CAGCTGCAAA CAACCCAGCG   120
   CCGGCAATTA CTTTGATGTT GAACCGATGA CCATNGCCTN CGNGTNCAAT CTCNTCTCTT   180
   NGCGCGCCNC TATTTNNGCC ATANATTTGG TTNNANNCGN AACGCTAGAC GTATCGAGTT   240
   CCTTTTCGAC CACCGGCTCA ATTGTCAGCA TCCTATGGGG AACATGAGCC CCGCCGCACC   300
   GGGCCGTTTC CAAATGGTGA CGTCACAACG GTGTCACAAG CCAGCGCAAT GTCCGCGGTA   360
   GGGACGCGGC GGCTGGGATC GGTGGGGTGA GCGCCCGGCT TCTCAAAGCG AGGGGAGCCC   420
   CGGGACTCTT ACCGGCCGAA GGCGGCGGGT GTCACTGATC TAGGCTGACG GCCAGTGGTT   480
   GNTNAGCCAA CAAGGATGAC NACAAATAAN CCGAGGANAG ACANGNGACG GNCCGANANG   540
   CTNANCCGGN NTTGNNCNAA NNNNACNCAC TTNTACCGNN CTTATGN                 587
(2) INFORMATION FOR SEQ ID NO:137:
   (i) SEQUENCE CHARACTERISTICS:
      (A) LENGTH: 1200 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:137:
   CAGGCATGAG CAGAGCGTTC ATCATCGATC CAACGATCAG TGCCATTGAC GGCTTGTACG    60
   ACCTTCTGGG GATTGGAATA CCCAACCAAG GGGGTATCCT TTACTCCTCA CTAGAGTACT   120
   TCGAAAAAGC CCTGGAGGAG CTGGCAGCAG CGTTTCCGGG TGATGGCTGG TTAGGTTCGG   180
   CCGCGGACAA ATACGCCGGC AAAAACCGCA ACCACGTGAA TTTTTTCCAG GAACTGGCAG   240
   ACCTCGATCG TCAGCTCATC AGCCTGATCC ACGACCAGGC CAACGCGGTC CAGACGACCC   300
   GCGACATCCT GGAGGGCGCC AAGAAAGGTC TCGAGTTCGT GCGCCCGGTG GCTGTGGACC   360
   TGACCTACAT CCCGGTCGTC GGGCACGCCC TATCGGCCGC CTTCCAGGCG CCGTTTTGCG   420
   CGGGCGCGAT GGCCGTAGTG GGCGGCGCGC TTGCCTACTT GGTCGTGAAA ACGCTGATCA   480
   ACGCGACTCA ACTCCTCAAA TTGCTTGCCA AATTGGCGGA GTTGGTCGCG GCCGCCATTG   540
   CGGACATCAT TTCGGATGTG GCGGACATCA TCAAGGGCAC CCTCGGAGAA GTGTGGGAGT   600
   TCATCACAAA CGCGCTCAAC GGCCTGAAAG AGCTTTGGGA CAAGCTCACG GGGTGGGTGA    660
   CCGGACTGTT CTCTCGAGGG TGGTCGAACC TGGAGTCCTT CTTTGCGGGC GTCCCCGGCT    720
   TGACCGGCGC GACCAGCGGC TTGTCGCAAG TGACTGGCTT GTTCGGTGCG GCCGGTCTGT    780
   CCGCATCGTC GGGCTTGGCT CACGCGGATA GCCTGGCGAG CTCAGCCAGC TTGCCCGCCC    840
   TGGCCGGCAT TGGGGGCGGG TCCGGTTTTG GGGGCTTGCC GAGCCTGGCT CAGGTCCATG    900
   CCGCCTCAAC TCGGCAGGCG CTACGGCCCC GAGCTGATGG CCCGGTCGGC GCCGCTGCCG    960
   AGCAGGTCGG CGGGCAGTCG CAGCTGGTCT CCGCGCAGGG TTCCCAAGGT ATGGGCGGAC   1020
   CCGTAGGCAT GGGCGGCATG CACCCCTCTT CGGGGGCGTC GAAAGGGACG ACGACGAAGA   1080
   AGTACTCGGA AGGCGCGGCG GCGGGCACTG AAGACGCCGA GCGCGCGCCA GTCGAAGCTG   1140
   ACGCGGGCGG TGGGCAAAAG GTGCTGGTAC GAAACGTCGT CTAACGGCAT GGCGAGCCAA   1200
(2) INFORMATION FOR SEQ ID NO:138:
   (i) SEQUENCE CHARACTERISTICS:
      (A) LENGTH: 392 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:138:
   Met Ser Arg Ala Phe Ile Ile Asp Pro Thr Ile Ser Ala Ile Asp Gly
    1               5                  10                  15
   Leu Tyr Asp Leu Leu Gly Ile Gly Ile Pro Asn Gln Gly Gly Ile Leu
               20                  25                  30
   Tyr Ser Ser Leu Glu Tyr Phe Glu Lys Ala Leu Glu Glu Leu Ala Ala
           35                  40                  45
   Ala Phe Pro Gly Asp Gly Trp Leu Gly Ser Ala Ala Asp Lys Tyr Ala
       50                  55                  60
   Gly Lys Asn Arg Asn His Val Asn Phe Phe Gln Glu Leu Ala Asp Leu
   65                  70                  75                  80
   Asp Arg Gln Leu Ile Ser Leu Ile His Asp Gln Ala Asn Ala Val Gln
                   85                  90                  95
   Thr Thr Arg Asp Ile Leu Glu Gly Ala Lys Lys Gly Leu Glu Phe Val
               100                 105                 110
   Arg Pro Val Ala Val Asp Leu Thr Tyr Ile Pro Val Val Gly His Ala
           115                 120                 125
   Leu Ser Ala Ala Phe Gln Ala Pro Phe Cys Ala Gly Ala Met Ala Val
       130                 135                 140
   Val Gly Gly Ala Leu Ala Tyr Leu Val Val Lys Thr Leu Ile Asn Ala
   145                 150                 155                 160
   Thr Gln Leu Leu Lys Leu Leu Ala Lys Leu Ala Glu Leu Val Ala Ala
                   165                 170                 175
   Ala Ile Ala Asp Ile Ile Ser Asp Val Ala Asp Ile Ile Lys Gly Thr
               180                 185                 190
   Leu Gly Glu Val Trp Glu Phe Ile Thr Asn Ala Leu Asn Gly Leu Lys
           195                 200                 205
   Glu Leu Trp Asp Lys Leu Thr Gly Trp Val Thr Gly Leu Phe Ser Arg
       210                 215                 220
   Gly Trp Ser Asn Leu Glu Ser Phe Phe Ala Gly Val Pro Gly Leu Thr
   225                 230                 235                 240
   Gly Ala Thr Ser Gly Leu Ser Gln Val Thr Gly Leu Phe Gly Ala Ala
                   245                 250                 255
   Gly Leu Ser Ala Ser Ser Gly Leu Ala His Ala Asp Ser Leu Ala Ser
               260                 265                 270
   Ser Ala Ser Leu Pro Ala Leu Ala Gly Ile Gly Gly Gly Ser Gly Phe
           275                 280                 285
   Gly Gly Leu Pro Ser Leu Ala Gln Val His Ala Ala Ser Thr Arg Gln
       290                 295                 300
   Ala Leu Arg Pro Arg Ala Asp Gly Pro Val Gly Ala Ala Ala Glu Gln
   305                 310                 315                 320
   Val Gly Gly Gln Ser Gln Leu Val Ser Ala Gln Gly Ser Gln Gly Met
                   325                 330                 335
   Gly Gly Pro Val Gly Met Gly Gly Met His Pro Ser Ser Gly Ala Ser
               340                 345                 350
   Lys Gly Thr Thr Thr Lys Lys Tyr Ser Glu Gly Ala Ala Ala Gly Thr
           355                 360                 365
   Glu Asp Ala Glu Arg Ala Pro Val Glu Ala Asp Ala Gly Gly Gly Gln
       370                 375                 380
   Lys Val Leu Val Arg Asn Val Val
   385                 390
(2) INFORMATION FOR SEQ ID NO:139:
   (i) SEQUENCE CHARACTERISTICS:
      (A) LENGTH: 439 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:139:
   ACGTTTACCC ATGCCGTCGG TGCAGAGCAA CGCCAGACAA CACAAAGTAG TCTAATTCCG     60
   TTATAAAGCA GACATTTCCG TGGTTATGTA GAAGATGTCG ACCGATCAGA TGAAGCGATC    120
   CGCGTCAGGT GGTATCCGAT GTCTTTTGTG ACCATCCAGC CGGTGGTCTT GGCAGCCGCG    180
   ACGGGGGACT TGCCGACGAT CGGTACCGCC GTGAGTGCTC GGAACACAGC CGTCTGTGCC    240
   CCGACGACGG GGGTGTTACC CCCTGCTGCC AATGACGTGT CGGTCCTGAC GGCGGCCCGG    300
   TTCACCGCGC ACACCAAGCA CTACCGAGTG GTGAGTAAGC CGGCCGCGCT GGTCCATGGC    360
   ATGTTCGTGG CCCTCCCGGC GGCCACCGCC GATGCGTATG CGACCACCGA GGCCGTCAAT    420
   GTGGTCGCGA CCGGTTAAG                                                 439
(2) INFORMATION FOR SEQ ID NO:140:
   (i) SEQUENCE CHARACTERISTICS:
      (A) LENGTH: 1441 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:140:
   GAGGTTGCTG GCAATGGATT TCGGGCTTTT ACCTCCGGAA GTGAATTCAA GCCGAATGTA     60
   TTCCGGTCCG GGGCCGGAGT CGATGCTAGC CGCCGCGGCC GCCTGGGACG GTGTGGCCGC    120
   GGAGTTGACT TCCGCCGCGG TCTCGTATGG ATCGGTGGTG TCGACGCTGA TCGTTGAGCC    180
   GTGGATGGGG CCGGCGGCGG CCGCGATGGC GGCCGCGGCA ACGCCGTATG TGGGGTGGCT    240
   GGCCGCCACG GCGGCGCTGG CGAAGGAGAC GGCCACACAG GCGAGGGCAG CGGCGGAAGC    300
   GTTTGGGACG GCGTTCGCGA TGACGGTGCC ACCATCCCTC GTCGCGGCCA ACCGCAGCCG    360
   GTTGATGTCG CTGGTCGCGG CGAACATTCT GGGGCAAAAC AGTGCGGCGA TCGCGGCTAC    420
   CCAGGCCGAG TATGCCGAAA TGTGGGCCCA AGACGCTGCC GTGATGTACA GCTATGAGGG    480
   GGCATCTGCG GCCGCGTCGG CGTTGCCGCC GTTCACTCCA CCCGTGCAAG GCACCGGCCC    540
   GGCCGGGCCC GCGGCCGCAG CCGCGGCGAC CCAAGCCGCC GGTGCGGGCG CCGTTGCGGA    600
   TGCACAGGCG ACACTGGCCC AGCTGCCCCC GGGGATCCTG AGCGACATTC TGTCCGCATT    660
   GGCCGCCAAC GCTGATCCGC TGACATCGGG ACTGTTGGGG ATCGCGTCGA CCCTCAACCC    720
   GCAAGTCGGA TCCGCTCAGC CGATAGTGAT CCCCACCCCG ATAGGGGAAT TGGACGTGAT    780
   CGCGCTCTAC ATTGCATCCA TCGCGACCGG CAGCATTGCG CTCGCGATCA CGAACACGGC    840
   CAGACCCTGG CACATCGGCC TATACGGGAA CGCCGGCGGG CTGGGACCGA CGCAGGGCCA    900
   TCCACTGAGT TCGGCGACCG ACGAGCCGGA GCCGCACTGG GGCCCCTTCG GGGGCGCGGC    960
   GCCGGTGTCC GCGGGCGTCG GCCACGCAGC ATTAGTCGGA GCGTTGTCGG TGCCGCACAG   1020
   CTGGACCACG GCCGCCCCGG AGATCCAGCT CGCCGTTCAG GCAACACCCA CCTTCAGCTC   1080
   CAGCGCCGGC GCCGACCCGA CGGCCCTAAA CGGGATGCCG GCAGGCCTGC TCAGCGGGAT   1140
   GGCTTTGGCG AGCCTGGCCG CACGCGGCAC GACGGGCGGT GGCGGCACCC GTAGCGGCAC   1200
   CAGCACTGAC GGCCAAGAGG ACGGCCGCAA ACCCCCGGTA GTTGTGATTA GAGAGCAGCC   1260
   GCCGCCCGGA AACCCCCCGC GGTAAAAGTC CGGCAACCGT TCGTCGCCGC GCGGAAAATG   1320
   CCTGGTGAGC GTGGCTATCC GACGGGCCGT TCACACCGCT TGTAGTAGCG TACGGCTATG   1380
   GACGACGGTG TCTGGATTCT CGGCGGCTAT CAGAGCGATT TTGCTCGCAA CCTCAGCAAA   1440
   G                                                                   1441
(2) INFORMATION FOR SEQ ID NO:141:
   (i) SEQUENCE CHARACTERISTICS:
      (A) LENGTH: 99 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:141:
   Met Ser Phe Val Thr Ile Gln Pro Val Val Leu Ala Ala Ala Thr Gly
    1               5                  10                  15
   Asp Leu Pro Thr Ile Gly Thr Ala Val Ser Ala Arg Asn Thr Ala Val
               20                  25                  30
   Cys Ala Pro Thr Thr Gly Val Leu Pro Pro Ala Ala Asn Asp Val Ser
           35                  40                  45
   Val Leu Thr Ala Ala Arg Phe Thr Ala His Thr Lys His Tyr Arg Val
       50                  55                  60
   Val Ser Lys Pro Ala Ala Leu Val His Gly Met Phe Val Ala Leu Pro
   65                  70                  75                  80
   Ala Ala Thr Ala Asp Ala Tyr Ala Thr Thr Glu Ala Val Asn Val Val
                   85                  90                  95
   Ala Thr Gly
(2) INFORMATION FOR SEQ ID NO:142:
   (i) SEQUENCE CHARACTERISTICS:
      (A) LENGTH: 423 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:142:
   Met Asp Phe Gly Leu Leu Pro Pro Glu Val Asn Ser Ser Arg Met Tyr
    1               5                  10                  15
   Ser Gly Pro Gly Pro Glu Ser Met Leu Ala Ala Ala Ala Ala Trp Asp
               20                  25                  30
   Gly Val Ala Ala Glu Leu Thr Ser Ala Ala Val Ser Tyr Gly Ser Val
           35                  40                  45
   Val Ser Thr Leu Ile Val Glu Pro Trp Met Gly Pro Ala Ala Ala Ala
       50                  55                  60
   Met Ala Ala Ala Ala Thr Pro Tyr Val Gly Trp Leu Ala Ala Thr Ala
   65                  70                  75                  80
   Ala Leu Ala Lys Glu Thr Ala Thr Gln Ala Arg Ala Ala Ala Glu Ala
                   85                  90                  95
   Phe Gly Thr Ala Phe Ala Met Thr Val Pro Pro Ser Leu Val Ala Ala
               100                 105                 110
   Asn Arg Ser Arg Leu Met Ser Leu Val Ala Ala Asn Ile Leu Gly Gln
           115                 120                 125
   Asn Ser Ala Ala Ile Ala Ala Thr Gln Ala Glu Tyr Ala Glu Met Trp
       130                 135                 140
   Ala Gln Asp Ala Ala Val Met Tyr Ser Tyr Glu Gly Ala Ser Ala Ala
   145                 150                 155                 160
   Ala Ser Ala Leu Pro Pro Phe Thr Pro Pro Val Gln Gly Thr Gly Pro
                   165                 170                 175
   Ala Gly Pro Ala Ala Ala Ala Ala Ala Thr Gln Ala Ala Gly Ala Gly
               180                 185                 190
   Ala Val Ala Asp Ala Gln Ala Thr Leu Ala Gln Leu Pro Pro Gly Ile
           195                 200                 205
   Leu Ser Asp Ile Leu Ser Ala Leu Ala Ala Asn Ala Asp Pro Leu Thr
       210                 215                 220
   Ser Gly Leu Leu Gly Ile Ala Ser Thr Leu Asn Pro Gln Val Gly Ser
   225                 230                 235                 240
   Ala Gln Pro Ile Val Ile Pro Thr Pro Ile Gly Glu Leu Asp Val Ile
                   245                 250                 255
   Ala Leu Tyr Ile Ala Ser Ile Ala Thr Gly Ser Ile Ala Leu Ala Ile
               260                 265                 270
   Thr Asn Thr Ala Arg Pro Trp His Ile Gly Leu Tyr Gly Asn Ala Gly
           275                 280                 285
   Gly Leu Gly Pro Thr Gln Gly His Pro Leu Ser Ser Ala Thr Asp Glu
       290                 295                 300
   Pro Glu Pro His Trp Gly Pro Phe Gly Gly Ala Ala Pro Val Ser Ala
   305                 310                 315                 320
   Gly Val Gly His Ala Ala Leu Val Gly Ala Leu Ser Val Pro His Ser
                   325                 330                 335
   Trp Thr Thr Ala Ala Pro Glu Ile Gln Leu Ala Val Gln Ala Thr Pro
               340                 345                 350
   Thr Phe Ser Ser Ser Ala Gly Ala Asp Pro Thr Ala Leu Asn Gly Met
           355                 360                 365
   Pro Ala Gly Leu Leu Ser Gly Met Ala Leu Ala Ser Leu Ala Ala Arg
       370                 375                 380
   Gly Thr Thr Gly Gly Gly Gly Thr Arg Ser Gly Thr Ser Thr Asp Gly
   385                 390                 395                 400
   Gln Glu Asp Gly Arg Lys Pro Pro Val Val Val Ile Arg Glu Gln Pro
                   405                 410                 415
   Pro Pro Gly Asn Pro Pro Arg
               420
(2) INFORMATION FOR SEQ ID NO:143:
   (i) SEQUENCE CHARACTERISTICS:
      (A) LENGTH: 97 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:143:
   Met Ser Leu Leu Asp Ala His Ile Pro Gln Leu Val Ala Ser Gln Ser
    1               5                  10                  15
   Ala Phe Ala Ala Lys Ala Gly Leu Met Arg His Thr Ile Gly Gln Ala
               20                  25                  30
   Glu Gln Ala Ala Met Ser Ala Gln Ala Phe His Gln Gly Glu Ser Ser
           35                  40                  45
   Ala Ala Phe Gln Ala Ala His Ala Arg Phe Val Ala Ala Ala Ala Lys
       50                  55                  60
   Val Asn Thr Leu Leu Asp Val Ala Gln Ala Asn Leu Gly Glu Ala Ala
   65                  70                  75                  80
   Gly Thr Tyr Val Ala Ala Asp Ala Ala Ala Ala Ser Thr Tyr Thr Gly
                   85                  90                  95
   Phe
(2) INFORMATION FOR SEQ ID NO:144:
   (i) SEQUENCE CHARACTERISTICS:
      (A) LENGTH: 99 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:144:
   Cys Arg Leu Cys Leu Asp Ser His Leu Arg Val Val Ala Leu Pro Ala
    1               5                  10                  15
   Gly Gln Pro Gly Arg Leu Val Gln Ala Ile Gly Pro Ala Gln Glu Arg
               20                  25                  30
   Asp Val Gly Gln Thr Arg Cys Thr Arg Thr Gly Leu Asp Xaa Val Ser
           35                  40                  45
   Ala Leu Thr Ala Ala Gln Phe Ala Ala His Ala Gln Ile Tyr Gln Ala
       50                  55                  60
   Val Ser Ala Gln Ala Ala Ala Ile His Glu Met Phe Val Asn Thr Leu
   65                  70                  75                  80
   Gln Xaa Xaa Ser Gly Ser Tyr Ala Ala Thr Glu Ala Ala Asn Ala Ala
                   85                  90                  95
   Ala Ala Gly